# EUROPEAN PATENT APPLICATION

(11) **EP 2 666 856 A1**
(43) Date of publication of application: **27.11.2013**
(21) Application number: 11856020.0
(22) Date of filing: 02.02.2011
(51) Int. Cl.: C12N 15/09, A61K 9/127, A61K 31/713, A61P 29/00, A61P 35/00, C12N 15/113

(54) **COMPOSITION FOR INHIBITING TARGET GENE EXPRESSION**

(30) Priority: 19.01.2011 JP 2011009032
(71) Applicant: Kyowa Hakko Kirin Co., Ltd., Tokyo 100-8185 (JP)
(72) Inventor: SHINOHARA, Fumikazu, Tokyo 100-8185 (JP); YOSHIDA, Tetsuo, Tokyo 100-8185 (JP); SUGISHITA, Hiroko, Tokyo 100-8185 (JP); NAOI, Tomoyuki, Tokyo 100-8185 (JP); ISHII, Toshihiko, Tokyo 100-8185 (JP); ENOKIZONO, Junichi, Tokyo 100-8185 (JP)
(74) Representative: Vossius & Partner
(86) International application number: PCT/JP2011/052092
(87) International publication number: WO 2012/098692

(57) **Abstract**

The present invention provides a composition that comprises a lipidparticle encapsulating a double-stranded nucleic acid molecule,
wherein the antisense strand is a polynucleotide of 17 to 30 bases in which a sequence is complementary to the sequence of the 17 contiguous bases of a target gene's RNA,
the sense strand is a polynucleotide of 17 to 30 bases that contains a base sequence complementary to the base sequence of bases 1 to 17 in the 5'-end to 3'-end direction of the antisense strand, and
a particular amount of the sugars binding to certain bases of the antisense strand and the sense strand are deoxyribose, or ribose whose hydroxyl group at the 2' position is substituted by a modifying group, and
wherein the lipidparticle contains a lipid bilayer membrane whose constituent component is a lipid conjugate, a fatty acid conjugate or an aliphatic hydrocarbon conjugate of a water-soluble substance.

### [SEQUENCE LISTING]

SEQUENCE LISTING

<110>  Kyowa Hakko Kirin Co., Ltd.

<120>  Preparation which inhibit gene expression

<130>  1000P12146

<150>  JP2011-009032
<151>  2011-01-19

<160>  41

<170>  PatentIn version 3.5

<210>  1
<211>  21
<212>  DNA
<213>  Artificial Sequence

<220>
<223>  siRNA sense of Com.1

<220>
<221>  misc_feature
<222>  (1)..(19)
<223>  RNA

<220>
<221>  misc_feature
<222>  (2)..(2)
<223>  n = uracil

<220>
<221>  misc_feature
<222>  (7)..(7)
<223>  n = uracil

<220>
<221>  misc_feature
<222>  (13)..(13)
<223>  n = uracil

<220>
<221>  misc_feature
<222>  (17)..(17)
<223>  n = uracil

<400>  1
gngaagncaa cangccngct t                                                21


<210>  2
<211>  21
<212>  DNA
<213>  Artificial Sequence

<220>
<223>  siRNA antisense of Com.1

<220>
<221>  misc_feature
<222>  (1)..(19)

<223>  RNA

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  misc_feature
<222>  (15)..(15)
<223>  n = uracil

<220>
<221>  misc_feature
<222>  (16)..(16)
<223>  n = uracil

<400>  2
gcaggcangn ngacnncact t                                                21


<210>  3
<211>  21
<212>  DNA
<213>  Artificial Sequence

<220>
<223>  siRNA sense of Exp.1

<220>
<221>  misc_feature
<222>  (1)..(19)
<223>  RNA

<220>
<221>  misc_feature
<222>  (2)..(2)
<223>  n = uracil

<220>
<221>  modified_base
<222>  (2)..(2)
<223>  um

<220>
<221>  misc_feature
<222>  (7)..(7)
<223>  n = uracil

<220>
<221>  modified_base
<222>  (7)..(7)
<223>  um

<220>
<221>  misc_feature
<222>  (13)..(13)
<223>  n = uracil

<220>
<221>  modified_base
<222>  (13)..(13)
<223>  um

<220>
<221>  misc_feature
<222>  (17)..(17)
<223>  n = uracil

<220>
<221>  modified_base
<222>  (17)..(17)
<223>  um

<400>  3
gngaagncaa cangccngct t                                                21


<210>  4
<211>  21
<212>  DNA
<213>  Artificial Sequence

<220>
<223>  siRNA antisense of Exp.1

<220>
<221>  misc_feature
<222>  (1)..(19)
<223>  RNA

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  misc_feature
<222>  (15)..(15)
<223>  n = uracil

<220>
<221>  misc_feature
<222>  (16)..(16)
<223>  n = uracil

<220>
<221>  modified_base
<222>  (17)..(17)
<223>  cm

<220>
<221>  modified_base
<222>  (18)..(18)
<223>  am

<220>
<221>  modified_base
<222>  (19)..(19)
<223>  cm

<400>  4
gcaggcangn ngacnncact t                                                21


<210>  5
<211>  21
<212>  DNA
<213>  Artificial Sequence

<220>
<223>  siRNA sense of Com.2

<220>
<221>  misc_feature
<222>  (1)..(19)
<223>  RNA

<220>
<221>  misc_feature
<222>  (2)..(2)
<223>  n = uracil

<220>
<221>  modified_base
<222>  (2)..(2)
<223>  um

<220>
<221>  misc_feature
<222>  (7)..(7)
<223>  n = uracil

<220>
<221>  modified_base
<222>  (7)..(7)
<223>  um

<220>
<221>  misc_feature
<222>  (13)..(13)
<223>  n = uracil

<220>
<221>  modified_base
<222>  (13)..(13)
<223>  um

<220>

<221>  misc_feature
<222>  (17)..(17)
<223>  n = uracil

<220>
<221>  modified_base
<222>  (17)..(17)
<223>  um

<400>  5
gngaagncaa cangccngct t                                                21


<210>  6
<211>  21
<212>  DNA
<213>  Artificial Sequence

<220>
<223>  siRNA antisense of Com.2

<220>
<221>  misc_feature
<222>  (1)..(19)
<223>  RNA

<220>
<221>  modified_base
<222>  (5)..(5)
<223>  gm

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  misc_feature
<222>  (15)..(15)
<223>  n = uracil

<220>
<221>  modified_base
<222>  (15)..(15)
<223>  um

<220>
<221>  misc_feature
<222>  (16)..(16)
<223>  n = uracil

<400>  6
gcaggcangn ngacnncact t                                                21

<210>  7
<211>  21
<212>  DNA
<213>  Artificial Sequence

<220>
<223>  siRNA sense of Com.3

<220>
<221>  misc_feature
<222>  (1)..(19)
<223>  RNA

<220>
<221>  misc_feature
<222>  (2)..(2)
<223>  n = uracil

<220>
<221>  modified_base
<222>  (2)..(2)
<223>  um

<220>
<221>  misc_feature
<222>  (7)..(7)
<223>  n = uracil

<220>
<221>  modified_base
<222>  (7)..(7)
<223>  um

<220>
<221>  misc_feature
<222>  (13)..(13)
<223>  n = uracil

<220>
<221>  modified_base
<222>  (13)..(13)
<223>  um

<220>
<221>  misc_feature
<222>  (17)..(17)
<223>  n = uracil

<220>
<221>  modified_base
<222>  (17)..(17)
<223>  um

<400>  7
gngaagncaa cangccngct t                                                21


<210>  8
<211>  21
<212>  DNA
<213>  Artificial Sequence

<220>
<223>  siRNA antisense of Com.3

<220>
<221>  misc_feature
<222>  (1)..(19)
<223>  RNA

<220>
<221>  modified_base
<222>  (5)..(5)
<223>  gm

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  modified_base
<222>  (12)..(12)
<223>  gm

<220>
<221>  misc_feature
<222>  (15)..(15)
<223>  n = uracil

<220>
<221>  modified_base
<222>  (15)..(15)
<223>  um

<220>
<221>  misc_feature
<222>  (16)..(16)
<223>  n = uracil

<400>  8
gcaggcangn ngacnncact t                                                21


<210>  9
<211>  21
<212>  DNA
<213>  Artificial Sequence

<220>
<223>  siRNA sense of Com.4

<220>
<221>  misc_feature

<222>  (1)..(19)
<223>  RNA

<220>
<221>  misc_feature
<222>  (2)..(2)
<223>  n = uracil

<220>
<221>  modified_base
<222>  (2)..(2)
<223>  um

<220>
<221>  misc_feature
<222>  (7)..(7)
<223>  n = uracil

<220>
<221>  modified_base
<222>  (7)..(7)
<223>  um

<220>
<221>  misc_feature
<222>  (13)..(13)
<223>  n = uracil

<220>
<221>  modified_base
<222>  (13)..(13)
<223>  um

<220>
<221>  misc_feature
<222>  (17)..(17)
<223>  n = uracil

<220>
<221>  modified_base
<222>  (17)..(17)
<223>  um

<400>  9
gngaagncaa cangccngct t                                                21


<210>  10
<211>  21
<212>  DNA
<213>  Artificial Sequence

<220>
<223>  siRNA antisense of Com.4

<220>
<221>  misc_feature
<222>  (1)..(19)
<223>  RNA

<220>
<221>  modified_base
<222>  (1)..(1)

<223>  gm

<220>
<221>  modified_base
<222>  (3)..(3)
<223>  am

<220>
<221>  modified_base
<222>  (5)..(5)
<223>  gm

<220>
<221>  modified_base
<222>  (7)..(7)
<223>  am

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  modified_base
<222>  (13)..(13)
<223>  am

<220>
<221>  misc_feature
<222>  (15)..(15)
<223>  n = uracil

<220>
<221>  modified_base
<222>  (15)..(15)
<223>  um

<220>
<221>  misc_feature
<222>  (16)..(16)
<223>  n = uracil

<220>
<221>  modified_base
<222>  (17)..(17)
<223>  cm

<220>
<221>  modified_base
<222>  (19)..(19)
<223>  cm

<400>  10

gcaggcangn ngacnncact t                                                21


<210>  11
<211>  21
<212>  DNA
<213>  Artificial Sequence

<220>
<223>  siRNA sense of Com.5

<220>
<221>  misc_feature
<222>  (1)..(19)
<223>  RNA

<220>
<221>  misc_feature
<222>  (2)..(2)
<223>  n = uracil

<220>
<221>  modified_base
<222>  (2)..(2)
<223>  um

<220>
<221>  misc_feature
<222>  (7)..(7)
<223>  n = uracil

<220>
<221>  modified_base
<222>  (7)..(7)
<223>  um

<220>
<221>  misc_feature
<222>  (13)..(13)
<223>  n = uracil

<220>
<221>  modified_base
<222>  (13)..(13)
<223>  um

<220>
<221>  misc_feature
<222>  (17)..(17)
<223>  n = uracil

<220>
<221>  modified_base
<222>  (17)..(17)
<223>  um

<400>  11
gngaagncaa cangccngct t                                                21


<210>  12
<211>  21
<212>  DNA

<213>  Artificial Sequence

<220>
<223>  siRNA antisense of Com.5

<220>
<221>  misc_feature
<222>  (1)..(19)
<223>  RNA

<220>
<221>  modified_base
<222>  (1)..(1)
<223>  gm

<220>
<221>  modified_base
<222>  (2)..(2)
<223>  cm

<220>
<221>  modified_base
<222>  (3)..(3)
<223>  am

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  misc_feature
<222>  (15)..(15)
<223>  n = uracil

<220>
<221>  misc_feature
<222>  (16)..(16)
<223>  n = uracil

<220>
<221>  modified_base
<222>  (17)..(17)
<223>  cm

<220>
<221>  modified_base
<222>  (18)..(18)
<223>  am

<220>
<221>  modified_base
<222>  (19)..(19)

<223>  cm

<400>  12
gcaggcangn ngacnncact t                                                21


<210>  13
<211>  21
<212>  DNA
<213>  Artificial Sequence

<220>
<223>  siRNA sense of Exp.2

<220>
<221>  misc_feature
<222>  (1)..(19)
<223>  RNA

<220>
<221>  misc_feature
<222>  (2)..(2)
<223>  n = uracil

<220>
<221>  misc_feature
<222>  (7)..(7)
<223>  n = uracil

<220>
<221>  misc_feature
<222>  (13)..(13)
<223>  n = uracil

<220>
<221>  misc_feature
<222>  (17)..(17)
<223>  n = uracil

<220>
<221>  modified_base
<222>  (17)..(17)
<223>  um

<220>
<221>  modified_base
<222>  (18)..(18)
<223>  gm

<220>
<221>  modified_base
<222>  (19)..(19)
<223>  cm

<400>  13
gngaagncaa cangccngct t                                                21


<210>  14
<211>  21
<212>  DNA
<213>  Artificial Sequence

<220>
<223>  siRNA antisense of Exp.2

<220>
<221>  misc_feature
<222>  (1)..(19)
<223>  RNA

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  misc_feature
<222>  (15)..(15)
<223>  n = uracil

<220>
<221>  misc_feature
<222>  (16)..(16)
<223>  n = uracil

<220>
<221>  modified_base
<222>  (17)..(17)
<223>  cm

<220>
<221>  modified_base
<222>  (18)..(18)
<223>  am

<220>
<221>  modified_base
<222>  (19)..(19)
<223>  cm

<400>  14
gcaggcangn ngacnncact t                                                21


<210>  15
<211>  21
<212>  DNA
<213>  Artificial Sequence

<220>
<223>  siRNA sense of Com.6

<220>
<221>  misc_feature
<222>  (1)..(19)

<223>  RNA

<220>
<221>  misc_feature
<222>  (2)..(2)
<223>  n = uracil

<220>
<221>  misc_feature
<222>  (7)..(7)
<223>  n = uracil

<220>
<221>  misc_feature
<222>  (13)..(13)
<223>  n = uracil

<220>
<221>  misc_feature
<222>  (17)..(17)
<223>  n = uracil

<220>
<221>  modified_base
<222>  (17)..(17)
<223>  um

<220>
<221>  modified_base
<222>  (18)..(18)
<223>  gm

<220>
<221>  modified_base
<222>  (19)..(19)
<223>  cm

<400>  15
gngaagncaa cangccngct t                                                21


<210>  16
<211>  21
<212>  DNA
<213>  Artificial Sequence

<220>
<223>  siRNA antisense of Com.6

<220>
<221>  misc_feature
<222>  (1)..(19)
<223>  RNA

<220>
<221>  modified_base
<222>  (5)..(5)
<223>  gm

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  misc_feature
<222>  (15)..(15)
<223>  n = uracil

<220>
<221>  modified_base
<222>  (15)..(15)
<223>  um

<220>
<221>  misc_feature
<222>  (16)..(16)
<223>  n = uracil

<400>  16
gcaggcangn ngacnncact t                                                21


<210>  17
<211>  21
<212>  DNA
<213>  Artificial Sequence

<220>
<223>  siRNA sense of Com.7

<220>
<221>  misc_feature
<222>  (1)..(19)
<223>  RNA

<220>
<221>  misc_feature
<222>  (2)..(2)
<223>  n = uracil

<220>
<221>  misc_feature
<222>  (7)..(7)
<223>  n = uracil

<220>
<221>  misc_feature
<222>  (13)..(13)
<223>  n = uracil

<220>
<221>  misc_feature
<222>  (17)..(17)
<223>  n = uracil

<220>
<221>  modified_base
<222>  (17)..(17)
<223>  um

<220>
<221>  modified_base
<222>  (18)..(18)
<223>  gm

<220>
<221>  modified_base
<222>  (19)..(19)
<223>  cm

<400>  17
gngaagncaa cangccngct t                                                21


<210>  18
<211>  21
<212>  DNA
<213>  Artificial Sequence

<220>
<223>  siRNA antisense of Com.7

<220>
<221>  misc_feature
<222>  (1)..(19)
<223>  RNA

<220>
<221>  modified_base
<222>  (5)..(5)
<223>  gm

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  modified_base
<222>  (12)..(12)
<223>  gm

<220>
<221>  misc_feature
<222>  (15)..(15)
<223>  n = uracil

<220>

<221>  modified_base
<222>  (15)..(15)
<223>  um

<220>
<221>  misc_feature
<222>  (16)..(16)
<223>  n = uracil

<400>  18
gcaggcangn ngacnncact t                                                21


<210>  19
<211>  21
<212>  DNA
<213>  Artificial Sequence

<220>
<223>  siRNA sense of Com.8

<220>
<221>  misc_feature
<222>  (1)..(19)
<223>  RNA

<220>
<221>  misc_feature
<222>  (2)..(2)
<223>  n = uracil

<220>
<221>  misc_feature
<222>  (7)..(7)
<223>  n = uracil

<220>
<221>  misc_feature
<222>  (13)..(13)
<223>  n = uracil

<220>
<221>  misc_feature
<222>  (17)..(17)
<223>  n = uracil

<220>
<221>  modified_base
<222>  (17)..(17)
<223>  um

<220>
<221>  modified_base
<222>  (18)..(18)
<223>  gm

<220>
<221>  modified_base
<222>  (19)..(19)
<223>  cm

<400>  19
gngaagncaa cangccngct t                                                21

<210>  20
<211>  21
<212>  DNA
<213>  Artificial Sequence

<220>
<223>  siRNA antisense of Com.8

<220>
<221>  misc_feature
<222>  (1)..(19)
<223>  RNA

<220>
<221>  modified_base
<222>  (1)..(1)
<223>  gm

<220>
<221>  modified_base
<222>  (3)..(3)
<223>  am

<220>
<221>  modified_base
<222>  (5)..(5)
<223>  gm

<220>
<221>  modified_base
<222>  (7)..(7)
<223>  am

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  modified_base
<222>  (13)..(13)
<223>  am

<220>
<221>  misc_feature
<222>  (15)..(15)
<223>  n = uracil

<220>
<221>  modified_base
<222>  (15)..(15)

<223>  um

<220>
<221>  misc_feature
<222>  (16)..(16)
<223>  n = uracil

<220>
<221>  modified_base
<222>  (17)..(17)
<223>  cm

<220>
<221>  modified_base
<222>  (19)..(19)
<223>  cm

<400>  20
gcaggcangn ngacnncact t                                                21


<210>  21
<211>  21
<212>  DNA
<213>  Artificial Sequence

<220>
<223>  siRNA sense of Com.9

<220>
<221>  misc_feature
<222>  (1)..(19)
<223>  RNA

<220>
<221>  misc_feature
<222>  (2)..(2)
<223>  n = uracil

<220>
<221>  misc_feature
<222>  (7)..(7)
<223>  n = uracil

<220>
<221>  misc_feature
<222>  (13)..(13)
<223>  n = uracil

<220>
<221>  misc_feature
<222>  (17)..(17)
<223>  n = uracil

<220>
<221>  modified_base
<222>  (17)..(17)
<223>  um

<220>
<221>  modified_base
<222>  (18)..(18)
<223>  gm

<220>
<221>  modified_base
<222>  (19)..(19)
<223>  cm

<400>  21
gngaagncaa cangccngct t                                                21


<210>  22
<211>  21
<212>  DNA
<213>  Artificial Sequence

<220>
<223>  siRNA antisense of Com.9


<220>
<221>  misc_feature
<222>  (1)..(19)
<223>  RNA

<220>
<221>  modified_base
<222>  (1)..(1)
<223>  gm

<220>
<221>  modified_base
<222>  (2)..(2)
<223>  cm

<220>
<221>  modified_base
<222>  (3)..(3)
<223>  am

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  misc_feature
<222>  (15)..(15)
<223>  n = uracil

<220>
<221>  misc_feature
<222>  (16)..(16)
<223>  n = uracil

<220>
<221>  modified_base
<222>  (17)..(17)
<223>  cm

<220>
<221>  modified_base
<222>  (18)..(18)
<223>  am

<220>
<221>  modified_base
<222>  (19)..(19)
<223>  cm

<400>  22
gcaggcangn ngacnncact t                                                21


<210>  23
<211>  21
<212>  DNA
<213>  Artificial Sequence

<220>
<223>  siRNA sense of Exp.3

<220>
<221>  misc_feature
<222>  (1)..(19)
<223>  RNA

<220>
<221>  misc_feature
<222>  (2)..(2)
<223>  n = uracil

<220>
<221>  modified_base
<222>  (2)..(2)
<223>  um

<220>
<221>  modified_base
<222>  (4)..(4)
<223>  am

<220>
<221>  modified_base
<222>  (6)..(6)
<223>  gm

<220>
<221>  misc_feature
<222>  (7)..(7)
<223>  n = uracil

<220>
<221>  modified_base
<222>  (8)..(8)
<223>  cm

<220>
<221>  modified_base
<222>  (12)..(12)
<223>  am

<220>
<221>  misc_feature
<222>  (13)..(13)
<223>  n = uracil

<220>
<221>  modified_base
<222>  (14)..(14)
<223>  gm

<220>
<221>  modified_base
<222>  (16)..(16)
<223>  cm

<220>
<221>  misc_feature
<222>  (17)..(17)
<223>  n = uracil

<220>
<221>  modified_base
<222>  (18)..(18)
<223>  gm

<400>  23
gngaagncaa cangccngct t                                                21


<210>  24
<211>  21
<212>  DNA
<213>  Artificial Sequence

<220>
<223>  siRNA antisense of Exp.3

<220>
<221>  misc_feature
<222>  (1)..(19)
<223>  RNA

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>

<221>  misc_feature
<222>  (15)..(15)
<223>  n = uracil

<220>
<221>  misc_feature
<222>  (16)..(16)
<223>  n = uracil

<220>
<221>  modified_base
<222>  (17)..(17)
<223>  cm

<220>
<221>  modified_base
<222>  (18)..(18)
<223>  am

<220>
<221>  modified_base
<222>  (19)..(19)
<223>  cm

<400>  24
gcaggcangn ngacnncact t                                                21


<210>  25
<211>  21
<212>  DNA
<213>  Artificial Sequence

<220>
<223>  siRNA sense of Exp.4

<220>
<221>  misc_feature
<222>  (1)..(19)
<223>  RNA

<220>
<221>  modified_base
<222>  (1)..(1)
<223>  gm

<220>
<221>  misc_feature
<222>  (2)..(2)
<223>  n = uracil

<220>
<221>  modified_base
<222>  (2)..(2)
<223>  um

<220>
<221>  modified_base
<222>  (3)..(3)
<223>  gm

<220>
<221>  misc_feature

<222>  (7)..(7)
<223>  n = uracil

<220>
<221>  misc_feature
<222>  (13)..(13)
<223>  n = uracil

<220>
<221>  misc_feature
<222>  (17)..(17)
<223>  n = uracil

<220>
<221>  modified_base
<222>  (17)..(17)
<223>  um

<220>
<221>  modified_base
<222>  (18)..(18)
<223>  gm

<220>
<221>  modified_base
<222>  (19)..(19)
<223>  cm

<400>  25
gngaagncaa cangccngct t                                                21


<210>  26
<211>  21
<212>  DNA
<213>  Artificial Sequence

<220>
<223>  siRNA antisense of Exp.4

<220>
<221>  misc_feature
<222>  (1)..(19)
<223>  RNA

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  misc_feature
<222>  (15)..(15)

<223>  n = uracil

<220>
<221>  misc_feature
<222>  (16)..(16)
<223>  n = uracil

<220>
<221>  modified_base
<222>  (17)..(17)
<223>  cm

<220>
<221>  modified_base
<222>  (18)..(18)
<223>  am

<220>
<221>  modified_base
<222>  (19)..(19)
<223>  cm

<400>  26
gcaggcangn ngacnncact t                                                21


<210>  27
<211>  20
<212>  DNA
<213>  Artificial Sequence

<220>
<223>  IS for siRNA sense of exp.1 to 4 and com.1 to 9

<220>
<221>  misc_feature
<222>  (1)..(18)
<223>  RNA

<220>
<221>  misc_feature
<222>  (2)..(2)
<223>  n = uracil

<220>
<221>  misc_feature
<222>  (7)..(7)
<223>  n = uracil

<220>
<221>  misc_feature
<222>  (13)..(13)
<223>  n = uracil

<220>
<221>  misc_feature
<222>  (17)..(17)
<223>  n = uracil

<400>  27
gngaagncaa cangccngtt                                                  20

<210>  28
<211>  20
<212>  DNA
<213>  Artificial Sequence

<220>
<223>  IS for siRNA antisense of exp.1 to 4 and com.1 to 9

<220>
<221>  misc_feature
<222>  (1)..(18)
<223>  RNA

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  misc_feature
<222>  (15)..(15)
<223>  n = uracil

<220>
<221>  misc_feature
<222>  (16)..(16)
<223>  n = uracil

<400>  28
caggcangnn gacnncactt                                                    20


<210>  29
<211>  25
<212>  RNA
<213>  Artificial Sequence

<220>
<223>  siRNA sense of Exp.5

<220>
<221>  modified_base
<222>  (1)..(1)
<223>  cm

<220>
<221>  modified_base
<222>  (2)..(2)
<223>  cm

<220>
<221>  modified_base
<222>  (3)..(3)

<223>  am

<220>
<221>  modified_base
<222>  (4)..(4)
<223>  cm

<220>
<221>  modified_base
<222>  (5)..(5)
<223>  am

<220>
<221>  modified_base
<222>  (6)..(6)
<223>  am

<220>
<221>  modified_base
<222>  (7)..(7)
<223>  gm

<220>
<221>  modified_base
<222>  (19)..(19)
<223>  um

<220>
<221>  modified_base
<222>  (20)..(20)
<223>  gm

<220>
<221>  modified_base
<222>  (21)..(21)
<223>  cm

<220>
<221>  modified_base
<222>  (22)..(22)
<223>  cm

<220>
<221>  modified_base
<222>  (23)..(23)
<223>  um

<220>
<221>  modified_base
<222>  (24)..(24)
<223>  gm

<220>
<221>  modified_base
<222>  (25)..(25)
<223>  cm

<400>  29
ccacaaguga agucaacaug ccugc                                          25


<210>  30
<211>  25

<212>  RNA
<213>  Artificial Sequence

<220>
<223>  siRNA antisense of Exp.5

<220>
<221>  modified_base
<222>  (19)..(19)
<223>  cm

<220>
<221>  modified_base
<222>  (20)..(20)
<223>  um

<220>
<221>  modified_base
<222>  (21)..(21)
<223>  um

<220>
<221>  modified_base
<222>  (22)..(22)
<223>  gm

<220>
<221>  modified_base
<222>  (23)..(23)
<223>  um

<220>
<221>  modified_base
<222>  (24)..(24)
<223>  gm

<220>
<221>  modified_base
<222>  (25)..(25)
<223>  gm

<400>  30
gcaggcaugu ugacuucacu ugugg                                          25


<210>  31
<211>  25
<212>  RNA
<213>  Artificial Sequence

<220>
<223>  siRNA sense of Com.10

<220>
<221>  modified_base
<222>  (1)..(1)
<223>  cm

<220>
<221>  modified_base
<222>  (2)..(2)
<223>  cm

<220>
<221>  modified_base
<222>  (3)..(3)
<223>  am

<220>
<221>  modified_base
<222>  (4)..(4)
<223>  cm

<220>
<221>  modified_base
<222>  (5)..(5)
<223>  am

<220>
<221>  modified_base
<222>  (6)..(6)
<223>  am

<220>
<221>  modified_base
<222>  (7)..(7)
<223>  gm

<220>
<221>  modified_base
<222>  (19)..(19)
<223>  um

<220>
<221>  modified_base
<222>  (20)..(20)
<223>  gm

<220>
<221>  modified_base
<222>  (21)..(21)
<223>  cm

<220>
<221>  modified_base
<222>  (22)..(22)
<223>  cm

<220>
<221>  modified_base
<222>  (23)..(23)
<223>  um

<220>
<221>  modified_base
<222>  (24)..(24)
<223>  gm

<220>
<221>  modified_base
<222>  (25)..(25)
<223>  cm

<400>  31
ccacaaguga agucaacaug ccugc                                          25

<210>  32
<211>  25
<212>  RNA
<213>  Artificial Sequence

<220>
<223>  siRNA antisense of Com.10

<400>  32
gcaggcaugu ugacuucacu ugugg                                          25


<210>  33
<211>  25
<212>  RNA
<213>  Artificial Sequence

<220>
<223>  siRNA sense of Com.11

<220>
<221>  modified_base
<222>  (1)..(1)
<223>  cm

<220>
<221>  modified_base
<222>  (2)..(2)
<223>  cm

<220>
<221>  modified_base
<222>  (3)..(3)
<223>  am

<220>
<221>  modified_base
<222>  (4)..(4)
<223>  cm

<220>
<221>  modified_base
<222>  (5)..(5)
<223>  am

<220>
<221>  modified_base
<222>  (6)..(6)
<223>  am

<220>
<221>  modified_base
<222>  (7)..(7)
<223>  gm

<400>  33
ccacaaguga agucaacaug ccugc                                          25


<210>  34
<211>  25
<212>  RNA

<213>  Artificial Sequence

<220>
<223>  siRNA antisense of Com.11


<220>
<221>  modified_base
<222>  (19)..(19)
<223>  cm

<220>
<221>  modified_base
<222>  (20)..(20)
<223>  um

<220>
<221>  modified_base
<222>  (21)..(21)
<223>  um

<220>
<221>  modified_base
<222>  (22)..(22)
<223>  gm

<220>
<221>  modified_base
<222>  (23)..(23)
<223>  um

<220>
<221>  modified_base
<222>  (24)..(24)
<223>  gm

<220>
<221>  modified_base
<222>  (25)..(25)
<223>  gm

<400>  34
gcaggcaugu ugacuucacu ugugg                                          25


<210>  35
<211>  25
<212>  RNA
<213>  Artificial Sequence

<220>
<223>  siRNA sense of Com.12

<220>
<221>  modified_base
<222>  (1)..(1)
<223>  cm

<220>
<221>  modified_base
<222>  (2)..(2)
<223>  cm

<220>
<221>  modified_base
<222>  (3)..(3)
<223>  am

<220>
<221>  modified_base
<222>  (4)..(4)
<223>  cm

<220>
<221>  modified_base
<222>  (5)..(5)
<223>  am

<220>
<221>  modified_base
<222>  (6)..(6)
<223>  am

<220>
<221>  modified_base
<222>  (7)..(7)
<223>  gm

<400>  35
ccacaaguga agucaacaug ccugc                                          25


<210>  36
<211>  25
<212>  RNA
<213>  Artificial Sequence

<220>
<223>  siRNA antisense of Com.12

<400>  36
gcaggcaugu ugacuucacu ugugg                                          25


<210>  37
<211>  25
<212>  RNA
<213>  Artificial Sequence

<220>
<223>  siRNA sense of Com.13

<220>
<221>  modified_base
<222>  (1)..(1)
<223>  cm

<220>
<221>  modified_base
<222>  (2)..(2)
<223>  cm

<220>
<221>  modified_base
<222>  (3)..(3)
<223>  am

<220>
<221>  modified_base
<222>  (4)..(4)
<223>  cm

<220>
<221>  modified_base
<222>  (22)..(22)
<223>  cm

<220>
<221>  modified_base
<222>  (23)..(23)
<223>  um

<220>
<221>  modified_base
<222>  (24)..(24)
<223>  gm

<220>
<221>  modified_base
<222>  (25)..(25)
<223>  cm

<400>  37
ccacaaguga agucaacaug ccugc                                          25


<210>  38
<211>  25
<212>  RNA
<213>  Artificial Sequence

<220>
<223>  siRNA antisense of Com.13

<400>  38
gcaggcaugu ugacuucacu ugugg                                          25


<210>  39
<211>  20
<212>  RNA
<213>  Artificial Sequence

<220>
<223>  IS for siRNA sense of exp.5 and com.10 to 13

<220>
<221>  misc_feature
<222>  (1)..(19)
<223>  RNA

<220>
<221>  misc_feature
<222>  (2)..(2)
<223>  n = uracil

<220>
<221>  modified_base
<222>  (2)..(2)

<223>  um

<220>
<221>  modified_base
<222>  (4)..(4)
<223>  am

<220>
<221>  modified_base
<222>  (6)..(6)
<223>  gm

<220>
<221>  misc_feature
<222>  (7)..(7)
<223>  n = uracil

<220>
<221>  modified_base
<222>  (8)..(8)
<223>  cm

<220>
<221>  modified_base
<222>  (10)..(10)
<223>  am

<220>
<221>  modified_base
<222>  (12)..(12)
<223>  am

<220>
<221>  misc_feature
<222>  (13)..(13)
<223>  n = uracil

<220>
<221>  modified_base
<222>  (14)..(14)
<223>  gm

<220>
<221>  modified_base
<222>  (16)..(16)
<223>  cm

<220>
<221>  misc_feature
<222>  (17)..(17)
<223>  n = uracil

<220>
<221>  modified_base
<222>  (18)..(18)
<223>  gm

<400>  39
gngaagncaa cangccngct                                                   20


<210>  40
<211>  20

<212>  DNA
<213>  Artificial Sequence

<220>
<223>  IS for siRNA antisense of exp.5 and com.10 to 13

<220>
<221>  misc_feature
<222>  (1)..(19)
<223>  RNA

<220>
<221>  misc_feature
<222>  (8)..(8)
<223>  n = uracil

<220>
<221>  misc_feature
<222>  (10)..(10)
<223>  n = uracil

<220>
<221>  misc_feature
<222>  (11)..(11)
<223>  n = uracil

<220>
<221>  misc_feature
<222>  (15)..(15)
<223>  n = uracil

<220>
<221>  misc_feature
<222>  (16)..(16)
<223>  n = uracil

<400>  40
gcaggcangn ngacnncact                                                   20


<210>  41
<211>  6492
<212>  DNA
<213>  Homo sapiens

<220>
<223>  bcl2 mRNA

<400>  41
tttctgtgaa gcagaagtct gggaatcgat ctggaaatcc tcctaatttt tactccctct      60

ccccgcgact cctgattcat tgggaagttt caaatcagct ataactggag agtgctgaag     120

attgatggga tcgttgcctt atgcatttgt tttggtttta caaaaaggaa acttgacaga     180

ggatcatgct gtacttaaaa aatacaacat cacagaggaa gtagactgat attaacaata     240

cttactaata ataacgtgcc tcatgaaata aagatccgaa aggaattgga ataaaaattt     300

cctgcatctc atgccaaggg ggaaacacca gaatcaagtg ttccgcgtga ttgaagacac     360

cccctcgtcc aagaatgcaa agcacatcca ataaaatagc tggattataa ctcctcttct     420

ttctctgggg gccgtggggt gggagctggg gcgagaggtg ccgttggccc ccgttgcttt 480
tcctctggga aggatggcgc acgctgggag aacagggtac gataaccggg agatagtgat 540
gaagtacatc cattataagc tgtcgcagag gggctacgag tgggatgcgg gagatgtggg 600
cgccgcgccc ccgggggccg cccccgcacc gggcatcttc tcctcccagc ccgggcacac 660
gccccatcca gccgcatccc gggacccggt cgccaggacc tcgccgctgc agaccccggc 720
tgcccccggc gccgccgcgg ggcctgcgct cagcccggtg ccacctgtgg tccacctgac 780
cctccgccag gccggcgacg acttctcccg ccgctaccgc cgcgacttcg ccgagatgtc 840
cagccagctg cacctgacgc ccttcaccgc gcggggacgc tttgccacgg tggtggagga 900
gctcttcagg gacggggtga actgggggag gattgtggcc ttctttgagt tcggtggggt 960
catgtgtgtg gagagcgtca accgggagat gtcgcccctg gtggacaaca tcgccctgtg 1020
gatgactgag tacctgaacc ggcacctgca cacctggatc caggataacg gaggctggga 1080
tgcctttgtg gaactgtacg gccccagcat gcggcctctg tttgatttct cctggctgtc 1140
tctgaagact ctgctcagtt tggccctggt gggagcttgc atcaccctgg gtgcctatct 1200
gggccacaag tgaagtcaac atgcctgccc caaacaaata tgcaaaaggt tcactaaagc 1260
agtagaaata atatgcattg tcagtgatgt accatgaaac aaagctgcag gctgtttaag 1320
aaaaaataac acacatataa acatcacaca cacagacaga cacacacaca cacaacaatt 1380
aacagtcttc aggcaaaacg tcgaatcagc tatttactgc caaagggaaa tatcatttat 1440
tttttacatt attaagaaaa aaagatttat ttatttaaga cagtcccatc aaaactcctg 1500
tctttggaaa tccgaccact aattgccaag caccgcttcg tgtggctcca cctggatgtt 1560
ctgtgcctgt aaacatagat tcgctttcca tgttgttggc cggatcacca tctgaagagc 1620
agacggatgg aaaaaggacc tgatcattgg ggaagctggc tttctggctg ctggaggctg 1680
gggagaaggt gttcattcac ttgcatttct ttgccctggg ggctgtgata ttaacagagg 1740
gagggttcct gtggggggaa gtccatgcct ccctggcctg aagaagagac tctttgcata 1800
tgactcacat gatgcatacc tggtgggagg aaaagagttg ggaacttcag atggacctag 1860
tacccactga gatttccacg ccgaaggaca gcgatgggaa aaatgccctt aaatcatagg 1920
aaagtatttt tttaagctac caattgtgcc gagaaaagca ttttagcaat ttatacaata 1980
tcatccagta ccttaagccc tgattgtgta tattcatata ttttggatac gcacccccca 2040
actcccaata ctggctctgt ctgagtaaga aacagaatcc tctggaactt gaggaagtga 2100
acatttcggt gacttccgca tcaggaaggc tagagttacc cagagcatca ggccgccaca 2160
agtgcctgct tttaggagac cgaagtccgc agaacctgcc tgtgtcccag cttggaggcc 2220
tggtcctgga actgagccgg ggccctcact ggcctcctcc agggatgatc aacagggcag 2280
tgtggtctcc gaatgtctgg aagctgatgg agctcagaat tccactgtca agaaagagca 2340

gtagaggggt gtggctgggc ctgtcaccct ggggccctcc aggtaggccc gttttcacgt 2400

ggagcatggg agccacgacc cttcttaaga catgtatcac tgtagaggga aggaacagag 2460

gccctgggcc cttcctatca gaaggacatg gtgaaggctg ggaacgtgag gagaggcaat 2520

ggccacggcc cattttggct gtagcacatg gcacgttggc tgtgtggcct tggcccacct 2580

gtgagtttaa agcaaggctt taaatgactt tggagagggt cacaaatcct aaaagaagca 2640

ttgaagtgag gtgtcatgga ttaattgacc cctgtctatg gaattacatg taaaacatta 2700

tcttgtcact gtagtttggt tttatttgaa aacctgacaa aaaaaaagtt ccaggtgtgg 2760

aatatggggg ttatctgtac atcctggggc attaaaaaaa aaatcaatgg tggggaacta 2820

taaagaagta acaaaagaag tgacatcttc agcaaataaa ctaggaaatt tttttttctt 2880

ccagtttaga atcagccttg aaacattgat ggaataactc tgtggcatta ttgcattata 2940

taccatttat ctgtattaac tttggaatgt actctgttca atgtttaatg ctgtggttga 3000

tatttcgaaa gctgctttaa aaaaatacat gcatctcagc gtttttttgt ttttaattgt 3060

atttagttat ggcctataca ctatttgtga gcaaaggtga tcgttttctg tttgagattt 3120

ttatctcttg attcttcaaa agcattctga gaaggtgaga taagccctga gtctcagcta 3180

cctaagaaaa acctggatgt cactggccac tgaggagctt tgtttcaacc aagtcatgtg 3240

catttccacg tcaacagaat tgtttattgt gacagttata tctgttgtcc ctttgacctt 3300

gtttcttgaa ggtttcctcg tccctgggca attccgcatt taattcatgg tattcaggat 3360

tacatgcatg tttggttaaa cccatgagat tcattcagtt aaaaatccag atggcaaatg 3420

accagcagat tcaaatctat ggtggtttga cctttagaga gttgctttac gtggcctgtt 3480

tcaacacaga cccacccaga gccctcctgc cctccttccg cgggggcttt ctcatggctg 3540

tccttcaggg tcttcctgaa atgcagtggt gcttacgctc caccaagaaa gcaggaaacc 3600

tgtggtatga agccagacct ccccggcggg cctcagggaa cagaatgatc agacctttga 3660

atgattctaa tttttaagca aaatattatt ttatgaaagg tttacattgt caaagtgatg 3720

aatatggaat atccaatcct gtgctgctat cctgccaaaa tcattttaat ggagtcagtt 3780

tgcagtatgc tccacgtggt aagatcctcc aagctgcttt agaagtaaca atgaagaacg 3840

tggacgtttt taatataaag cctgttttgt cttttgttgt tgttcaaacg ggattcacag 3900

agtatttgaa aaatgtatat atattaagag gtcacggggg ctaattgctg gctggctgcc 3960

ttttgctgtg gggttttgtt acctggtttt aataacagta aatgtgccca gcctcttggc 4020

cccagaactg tacagtattg tggctgcact tgctctaaga gtagttgatg ttgcattttc 4080

cttattgtta aaaacatgtt agaagcaatg aatgtatata aaagcctcaa ctagtcattt 4140

ttttctcctc ttcttttttt tcattatatc taattatttt gcagttgggc aacagagaac 4200

catccctatt ttgtattgaa gagggattca catctgcatc ttaactgctc tttatgaatg 4260

aaaaaacagt cctctgtatg tactcctctt tacactggcc agggtcagag ttaaatagag 4320

tatatgcact ttccaaattg gggacaaggg ctctaaaaaa agccccaaaa ggagaagaac 4380

atctgagaac ctcctcggcc ctcccagtcc ctcgctgcac aaatactccg caagagaggc 4440

cagaatgaca gctgacaggg tctatggcca tcgggtcgtc tccgaagatt tggcaggggc 4500

agaaaactct ggcaggctta agatttggaa taaagtcaca gaattaagga agcacctcaa 4560

tttagttcaa acaagacgcc aacattctct ccacagctca cttacctctc tgtgttcaga 4620

tgtggccttc catttatatg tgatctttgt tttattagta aatgcttatc atctaaagat 4680

gtagctctgg cccagtggga aaaattagga agtgattata aatcgagagg agttataata 4740

atcaagatta aatgtaaata atcagggcaa tcccaacaca tgtctagctt tcacctccag 4800

gatctattga gtgaacagaa ttgcaaatag tctctatttg taattgaact tatcctaaaa 4860

caaatagttt ataaatgtga acttaaactc taattaattc caactgtact tttaaggcag 4920

tggctgtttt tagactttct tatcacttat agttagtaat gtacacctac tctatcagag 4980

aaaaacagga aaggctcgaa atacaagcca ttctaaggaa attagggagt cagttgaaat 5040

tctattctga tcttattctg tggtgtcttt tgcagcccag acaaatgtgg ttacacactt 5100

tttaagaaat acaattctac attgtcaagc ttatgaaggt tccaatcaga tctttattgt 5160

tattcaattt ggatctttca gggatttttt ttttaaatta ttatgggaca aaggacattt 5220

gttggagggg tgggagggag gaagaatttt taaatgtaaa acattcccaa gtttggatca 5280

gggagttgga agttttcaga ataaccagaa ctaagggtat gaaggacctg tattggggtc 5340

gatgtgatgc ctctgcgaag aaccttgtgt gacaaatgag aaacattttg aagtttgtgg 5400

tacgaccttt agattccaga gacatcagca tggctcaaag tgcagctccg tttggcagtg 5460

caatggtata aatttcaagc tggatatgtc taatgggtat ttaaacaata aatgtgcagt 5520

tttaactaac aggatattta atgacaacct tctggttggt agggacatct gtttctaaat 5580

gtttattatg tacaatacag aaaaaaattt tataaaatta agcaatgtga aactgaattg 5640

gagagtgata atacaagtcc tttagtctta cccagtgaat cattctgttc catgtctttg 5700

gacaaccatg accttggaca atcatgaaat atgcatctca ctggatgcaa agaaaatcag 5760

atggagcatg aatggtactg taccggttca tctggactgc cccagaaaaa taacttcaag 5820

caaacatcct atcaacaaca aggttgttct gcataccaag ctgagcacag aagatgggaa 5880

cactggtgga ggatggaaag gctcgctcaa tcaagaaaat tctgagacta ttaataaata 5940

agactgtagt gtagatactg agtaaatcca tgcacctaaa ccttttggaa aatctgccgt 6000

gggccctcca gatagctcat ttcattaagt ttttccctcc aaggtagaat ttgcaagagt 6060

gacagtggat tgcatttctt ttggggaagc tttcttttgg tggttttgtt tattatacct 6120

tcttaagttt tcaaccaagg tttgcttttg ttttgagtta ctggggttat ttttgtttta 6180

aataaaaata agtgtacaat aagtgttttt gtattgaaag cttttgttat caagattttc 6240

atacttttac cttccatggc tctttttaag attgatactt ttaagaggtg gctgatattc 6300

tgcaacactg tacacataaa aaatacggta aggatacttt acatggttaa ggtaaagtaa 6360

gtctccagtt ggccaccatt agctataatg gcactttgtt tgtgttgttg gaaaaagtca 6420

cattgccatt aaactttcct tgtctgtcta gttaatattg tgaagaaaaa taaagtacag 6480

tgtgagatac tg 6492

## Description

### Technical Field

The present invention relates to a composition for suppressing the expression of a target gene, and the like.

### Background Art

As a method of suppressing the expression of a target gene, for example, a method of utilizing RNA interference (hereinafter referred to as "RNAi") and the like are known, and specifically, a phenomenon in which when a double-stranded RNA having a sequence identical to that of a target gene is introduced into Nematoda, thereby the expression of the target gene is specifically suppressed has been reported (see "Nature", Vol. 391, No. 6669, pp. 806-811, 1998). Further, it has been found that even when a double-stranded RNA having a length of 21 to 23 bases is introduced into Drosophila, instead of a long double-stranded RNA, the expression of a target gene is suppressed. This is named a short interfering RNA (siRNA) (see International Publication No. WO 01/75164).

In the case of mammalian cells, when a long double-stranded RNA was introduced, apoptosis took place as a result of the functions of virus defense mechanism, and thus the expression of a specific gene could not be suppressed. However, it has been found that when siRNA having a length of 20 to 29 bases is used, such a reaction does not take place, and that the expression of a specific gene can be suppressed. Among others, siRNA having 21 to 25 bases has a high effect of suppressing expression ("Nature", Vol. 411, No. 6836, pp. 494-498, 2001; "Nature Reviews Genetics", Vol. 3, No. 10, pp. 737-747, 2002; "Molecular Cell", (USA) Vol. 10, No. 3, pp. 549-561, 2002; "Nature Biotechnology", (USA) Vol. 20, No. 5, pp. 497-500, 2002).

RNAi has been frequently verified also in in vivo tests. The effect of siRNA with a length of 50 base pairs or less on fetal animals (see Patent document 1) and the effect thereof on adult mice (see Patent document 2) are reported. Moreover, the effect of suppressing the expression of a specific gene has been found in each of organs that are kidney, spleen, lung, pancreas, and liver, when siRNA is intravenously administered to a fetal mouse (see Non-patent document 1). Furthermore, it has been reported that also when siRNA is directly administered to brain cells, the expression of a specific gene is suppressed (see Non-patent document 2).

On the other hand, as means for delivering a nucleic acid into a cell, a method using cationic lipidparticle or cationic polymers is known. However, in the method, after intravenous administration of cationic lipidparticle or cationic polymers containing a nucleic acid is carried out, the nucleic acid is promptly removed from the blood, and when a target tissue is other than liver or lung, for example, when it is a tumor site or the like, the nucleic acid cannot be delivered to the target tissue, and therefore, the expression of a sufficient action has not been made possible yet. Accordingly, a nucleic acid-encapsulating lipidparticle (lipidparticle encapsulating a nucleic acid therein) with which the problem that a nucleic acid is promptly removed from the blood was solved has been reported (see Patent documents 3 to 6, and Non-patent document 3). In the Patent document 3, as a method of producing lipidparticle encapsulating a nucleic acid or the like, for example, a method of producing an oligodeoxynucleotide (ODN)-encapsulating lipidparticle by dissolving a cationic lipid in chloroform in advance, adding an aqueous solution of ODN and methanol thereto and mixing and centrifuging the mixture thereby transferring a complex of the cationic lipid and ODN to a chloroform layer, and then taking out the chloroform layer, adding a polyethylene glycolated phospholipid, a neutral lipid, and water to the chloroform layer to form a water-in-oil (w/o) emulsion and treating the emulsion by the reverse phase evaporation method has been reported. In Patent document 4 and Non-patent document 3, a method of producing an ODN-encapsulating lipidparticle by dissolving ODN in an aqueous solution of citric acid at pH 3.8, adding a lipid (in ethanol) to the solution, reducing the ethanol concentration to 20 v/v% to prepare an ODN-encapsulating lipidparticle, performing filtration for sizing, removing excess ethanol by dialysis, and then further performing dialysis of the sample at pH 7.5 to remove ODN adhering to the surface of the lipidparticle has been reported. In each method, a lipidparticle encapsulating an active ingredient such as a nucleic acid is produced.

On the other hand, in Patent documents 5 and 6, it has been reported that a lipidparticle encapsulating an active ingredient such as a nucleic acid is produced by a method of coating fine particles with a lipid bilayer membrane in a liquid. In the method, fine particles are coated with a lipid bilayer membrane in liquid by reducing the concentration of the polar organic solvent in a polar organic solvent-containing aqueous solution in which the fine particles are dispersed and a lipid is dissolved. In this method, for example, fine particles coated with a lipid bilayer membrane (coated fine particles) having a size suitable for fine particles for intravenous injection and the like are produced very efficiently. In addition, as examples of the fine particles to be coated, for example, a complex which consists of ODN or siRNA and a cationic lipid, and is formed by an electrostatic interaction is exemplified in Patent documents 5 and 6. It has been reported that the particle diameter of the coated fine particles obtained by coating the fine particles is small and suitable for using as an injection, and the coated fine particles show high retention in the blood and are much accumulated in a tumor tissue when they are intravenously administered.

It has been common procedure to modify the surface of lipidparticle with a water-soluble polymer such as polyethylene glycol (PEG). Such surface-modified lipidparticles are known to have a long retention time in blood, because of difficulties interacting with serum proteins such as opsonin, and the ability to avoid recognition by macrophage. There are also reports that the nucleic acid-encapsulating lipidparticle, when PEG-modified, can have higher retention in blood, and accumulate in large amounts in tumor tissue. However, it is known that the second administration of a PEG-modified lipidparticle at intervals of 3 to 7 days from the first administration greatly lowers the blood retention of the PEG-modified lipidparticle, and it is considered that the significant reduction in the blood retention of the PEG-modified lipidparticle at the second administration occurs as the anti-PEG-IgM antibodies induced at the first administration of the PEG lipidparticle bind to the PEG of the lipidparticle at the second administration, and activates the complement system to accelerate the intake by the hepatic macrophage (see Non-Patent Document 4).

Patent document 1: United States Publication No. US 2002-132788
Patent document 2: International Publication No. WO 03/10180
Patent document 3: Published Japanese translation of a PCT international application No. 2002-508765
Patent document 4: Published Japanese translation of a PCT international application No. 2002-501511
Patent document 5: International Publication No. WO 02/28367
Patent document 6: International Publication No. WO 2006/080118

Non-patent document 1: "Nature Genetics", Vol. 32, No. 1, pp. 107-108, 2002
Non-patent document 2: "Nature Biotechnology", Vol. 20, No. 10, pp. 1006-1010, 2002
Non-patent document 3: "Biochimica et Biophysica Acta", Vol. 1510, pp. 152-166, 2001
Non-patent document 4: "Journal of Controlled Release", Vol. 137, pp. 234-240, 2009

### Summary of the Invention

### Problems to be Solved by the Invention

An object of the present invention is to provide a composition for suppressing the expression of a target gene, and the like. Further, it is another object to provide a composition for suppressing the expression of a target gene, which composition contains a lipidparticle surface-modified with a water-soluble polymer such as polyethylene glycol (PEG), and that can show high blood retention by suppressing the significant reduction in blood retention at the second administration, and the like.

### Means for Solving the Problems

The present invention relates to the following (1) to (56).
(1) A composition that comprises a lipidparticle encapsulating a double-stranded nucleic acid molecule that contains a sense strand and an antisense strand,
   wherein the antisense strand is a polynucleotide of 17 to 30 bases in which a sequence (sequence a) of bases 1 to 17 in the 5' -end to 3' -end direction is complementary to the sequence of the 17 contiguous bases of a target gene's mRNA, and the sugars in the antisense strand are ribose, deoxyribose, or ribose whose hydroxyl group at the 2' position is substituted by a modifying group, and
   wherein the sense strand is a polynucleotide of 17 to 30 bases that contains a base sequence (sequence b) complementary to the base sequence of bases 1 to 17 in the 5' -end to 3'-end direction of the antisense strand, and the sugars in the sense strand are ribose, deoxyribose, or ribose whose hydroxyl group at the 2' position is substituted by a modifying group,
   (i) 0 to 30% of the sugars binding to the bases 1 to 8 in the 5'-end to 3'-end direction of sequence a are deoxyribose, or ribose whose hydroxyl group at the 2' position is substituted by a modifying group,
   (ii) 0 to 20% of the sugars binding to the bases 9 to 16 in the 5'-end to 3'-end direction of sequence a are deoxyribose, or a ribose whose hydroxyl group at the 2' position is substituted by a modifying group,
   (iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
   (iv) 10 to 70% of the sugars binding to the bases 1 to 17 in the 5'-end to 3'-end direction of sequence b are deoxyribose, or ribose whose hydroxyl group at the 2' position is substituted by a modifying group,
   (v) 30 to 100% of the sugars binding to the bases other than sequence b of the sense strand are deoxyribose, or ribose whose hydroxyl group at the 2' position is substituted by a modifying group, and
   the lipidparticle is a lipidparticle having a size that allows for intravenous administration, and contains a lipid bilayer membrane whose constituent component is a lipid conjugate, a fatty acid conjugate or an aliphatic hydrocarbon conjugate of a water-soluble substance.
(2) The composition according to the above (1), wherein (v) 50 to 70% of the sugars binding to the bases other than sequence b of the sense strand are deoxyribose, or ribose whose hydroxyl group at the 2' position is substituted by a modifying group.
(3) The composition according to the above (1) or (2), wherein the double-stranded nucleic acid molecule is a double-stranded nucleic acid molecule having an activity of suppressing the expression of the target gene by utilizing RNA interference (RNAi).
(4) The composition according to any one of the above (1) to (3), wherein the target gene is a gene associated with tumor or inflammation.
(5) The composition according to any one of the above (1) to (4), wherein the target gene is a gene associated with angiogenesis.
(6) The composition according to any one of the above (1) to (4), wherein the target gene is a gene of any one of a vascular endothelial growth factor, a vascular endothelial growth factor receptor, a fibroblast growth factor, a fibroblast growth factor receptor, a platelet-derived growth factor, a platelet-derived growth factor receptor, a hepatocyte growth factor, a hepatocyte growth factor receptor, a Kruppel-like factor, an Ets transcription factor, a nuclear factor and a hypoxia-inducible factor.
(7) The composition according to any one of the above (1) to (6), wherein the mRNA is either human mRNA or mouse mRNA.
(8) The composition according to any one of the above (1) to (7), wherein the lipidparticle encapsulating the double-stranded nucleic acid molecule is a lipidparticle that comprises:
   a complex particle that contains a lead particle and the double-stranded nucleic acid molecule as constituent components; and
   a lipid bilayer membrane coating the complex particle,
   wherein constituent components of the lipid bilayer membrane are soluble in a certain polar organic solvent, and wherein the constituent components of the lipid bilayer membrane, and the complex particle are dispersible in a liquid that contains the polar organic solvent in a certain concentration.
(9) The composition according to the above (8), wherein the polar organic solvent is an alcohol.
(10) The composition according to the above (8), wherein the polar organic solvent is ethanol.
(11) The composition according to any one of the above (8) to (10), wherein the lead particle is a lead particle that contains a cationic substance, and wherein the lipid bilayer membrane coating the complex particle contains, as constituent components, a neutral lipid, and a lipid conjugate, a fatty acid conjugate or an aliphatic hydrocarbon conjugate of a water-soluble substance.
(12) The composition according to any one of the above (1) to (7), wherein the lipidparticle encapsulating the double-stranded nucleic acid molecule is a lipidparticle that comprises: a complex particle that contains a cationic substance-containing lead particle and the double-stranded nucleic acid molecule as constituent components; and a lipid bilayer membrane coating the complex particle,
   wherein the lipid bilayer membrane coating the complex particle contains, as constituent components, a neutral lipid, and a lipid conjugate, a fatty acid conjugate or an aliphatic hydrocarbon conjugate of a water-soluble substance.
(13) The composition according to any one of the above (1) to (12), wherein the lipid conjugate, the fatty acid conjugate or the aliphatic hydrocarbon conjugate of a water-soluble substance is polyethylene glycol phosphatidyl ethanolamine.

(14) A therapeutic agent for cancer or inflammatory disease, the therapeutic agent comprising a lipidparticle that comprises:
   a complex particle that contains, as constituent components, a lead particle and a double-stranded nucleic acid molecule that contains a sense strand and an antisense strand; and
   a lipid bilayer membrane coating the complex particle,
   wherein the antisense strand is a polynucleotide of 17 to 30 bases in which a sequence (sequence a) of bases 1 to 17 in the 5' -end to 3' -end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
   wherein the sense strand is a polynucleotide of 17 to 30 bases that contains a base sequence (sequence b) complementary to the base sequence of bases 1 to 17 in the 5' -end to 3'-end direction of the antisense strand, and the sugars in the sense strand are ribose, deoxyribose, or ribose whose hydroxyl group at the 2' position is substituted by a modifying group,
      (i) 0 to 30% of the sugars binding to the bases 1 to 8 in the 5'-end to 3'-end direction of sequence a are deoxyribose, or ribose whose hydroxyl group at the 2' position is substituted by a modifying group,
      (ii) 0 to 20% of the sugars binding to the bases 9 to 16 in the 5'-end to 3'-end direction of sequence a are deoxyribose, or a ribose whose hydroxyl group at the 2' position is substituted by a modifying group,
      (iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
      (iv) 10 to 70% of the sugars binding to the bases 1 to 17 in the 5'-end to 3'-end direction of sequence b are deoxyribose, or ribose whose hydroxyl group at the 2' position is substituted by a modifying group,
      (v) 30 to 100% of the sugars binding to the bases other than sequence b of the sense strand are deoxyribose, or ribose whose hydroxyl group at the 2' position is substituted by a modifying group, and
   the lipidparticle is a lipidparticle having a size that allows for intravenous administration,
   wherein the constituent components of the lipid bilayer membrane are soluble in a certain polar organic solvent, and wherein the constituent components of the lipid bilayer membrane, and the complex particle are dispersible in a liquid that contains the polar organic solvent in a certain concentration, and the lipid bilayer membrane contains as constituent component, a lipid conjugate, a fatty acid conjugate, or an aliphatic hydrocarbon conjugate of a water-soluble substance.
(15) The therapeutic agent for cancer or inflammatory disease according to the above (14), wherein the polar organic solvent is an alcohol.
(16) The therapeutic agent for cancer or inflammatory disease according to the above (14), wherein the polar organic solvent is ethanol.
(17) The therapeutic agent for cancer or inflammatory disease according to any one of the above (14) to (16), wherein the lead particle is a lead particle that contains a cationic substance, and wherein the lipid bilayer membrane contains, as constituent components, a neutral lipid, and a lipid conjugate, a fatty acid conjugate or an aliphatic hydrocarbon conjugate of a water-soluble substance.
(18) A therapeutic agent for cancer or inflammatory disease, the therapeutic agent comprising a lipidparticle that comprises:
   a complex particle that contains, as constituent components, a cationic substance-containing lead particle and a double-stranded nucleic acid molecule that contains a sense strand and an antisense strand; and
   a lipid bilayer membrane coating the complex particle,
   wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
   wherein the sense strand is a polynucleotide of 17 to 30 bases that contains a base sequence (sequence b) complementary to the base sequence of bases 1 to 17 in the 5' -end to 3' -end direction of the antisense strand, and the sugars in the sense strand are ribose, deoxyribose, or ribose whose hydroxyl group at the 2' position is substituted by a modifying group,
   (i) 0 to 30% of the sugars binding to the bases 1 to 8 in the 5'-end to 3'-end direction of sequence a are deoxyribose, or ribose whose hydroxyl group at the 2' position is substituted by a modifying group,
   (ii) 0 to 20% of the sugars binding to the bases 9 to 16 in the 5'-end to 3'-end direction of sequence a are deoxyribose, or a ribose whose hydroxyl group at the 2' position is substituted by a modifying group,
   (iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
   (iv) 10 to 70% of the sugars binding to the bases 1 to 17 in the 5'-end to 3'-end direction of sequence b are deoxyribose, or ribose whose hydroxyl group at the 2' position is substituted by a modifying group,
   (v) 30 to 100% of the sugars binding to the bases other than sequence b of the sense strand are deoxyribose, or ribose whose hydroxyl group at the 2' position is substituted by a modifying group, and
   the lipidparticle is a lipidparticle having a size that allows for intravenous administration, and the lipid bilayer membrane contains, as constituent components, a neutral lipid, and a lipid conjugate, a fatty acid conjugate or an aliphatic hydrocarbon conjugate of a water-soluble substance.
(19) The therapeutic agent for cancer or inflammatory disease according to any one of the above (14) to (18), wherein the lipid conjugate, the fatty acid conjugate, or the aliphatic hydrocarbon conjugate of a water-soluble substance is polyethylene glycol phosphatidyl ethanolamine.
(20) The therapeutic agent for cancer or inflammatory disease according to any one of the above (14) to (19), wherein (v) 50 to 70% of the sugars binding to the bases other than sequence b of the sense strand are deoxyribose, or ribose whose hydroxyl group at the 2' position is substituted by a modifying group.
(21) The therapeutic agent for cancer or inflammatory disease according to any one of the above (14) to (20), wherein the target gene associated with tumor or inflammation is a gene associated with angiogenesis.
(22) The therapeutic agent for cancer or inflammatory disease according to any one of the above (14) to (20), wherein the target gene associated with tumor or inflammation is a gene of any one of a vascular endothelial growth factor, a vascular endothelial growth factor receptor, a fibroblast growth factor, a fibroblast growth factor receptor, a platelet-derived growth factor, a platelet-derived growth factor receptor, a hepatocyte growth factor, a hepatocyte growth factor receptor, a Krüppel-like factor, an Ets transcription factor, a nuclear factor, and a hypoxia-inducible factor.
(23) The therapeutic agent for cancer or inflammatory disease according to any one of the above (14) to (22), wherein the mRNA is either human mRNA or mouse mRNA.

(24) A method for treating cancer or inflammatory disease, which comprises administering to a mammal a composition that comprises a lipidparticle that comprises:
   a complex particle that contains, as constituent components, a lead particle and a double-stranded nucleic acid molecule that contains a sense strand and an antisense strand; and
   a lipid bilayer membrane coating the complex particle,
   wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
   wherein the sense strand is a polynucleotide of 17 to 30 bases that contains a base sequence (sequence b) complementary to the base sequence of bases 1 to 17 in the 5' -end to 3' -end direction of the antisense strand, and the sugars in the sense strand are ribose, deoxyribose, or ribose whose hydroxyl group at the 2' position is substituted by a modifying group,
      (i) 0 to 30% of the sugars binding to the bases 1 to 8 in the 5'-end to 3'-end direction of sequence a are deoxyribose, or ribose whose hydroxyl group at the 2' position is substituted by a modifying group,
      (ii) 0 to 20% of the sugars binding to the bases 9 to 16 in the 5'-end to 3'-end direction of sequence a are deoxyribose, or a ribose whose hydroxyl group at the 2' position is substituted by a modifying group,
      (iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
      (iv) 10 to 70% of the sugars binding to the bases 1 to 17 in the 5'-end to 3'-end direction of sequence b are deoxyribose, or ribose whose hydroxyl group at the 2' position is substituted by a modifying group,
      (v) 30 to 100% of the sugars binding to the bases other than sequence b of the sense strand are deoxyribose, or ribose whose hydroxyl group at the 2' position is substituted by a modifying group, and
   the lipidparticle is a lipidparticle having a size that allows for intravenous administration,
   wherein the constituent components of the lipid bilayer membrane are soluble in a certain polar organic solvent, and wherein the constituent components of the lipid bilayer membrane, and the complex particle are dispersible in a liquid that contains the polar organic solvent in a certain concentration, and the lipid bilayer membrane contains as constituent component, a lipid conjugate, a fatty acid conjugate, or an aliphatic hydrocarbon conjugate of a water-soluble substance.
(25) The method for treating cancer or inflammatory disease according to the above (24), wherein the polar organic solvent is an alcohol.
(26) The method for treating cancer or inflammatory disease according to the above (24), wherein the polar organic solvent is ethanol.
(27) The method for treating cancer or inflammatory disease according to any one of the above (24) to (26), wherein the lead particle is a lead particle that contains a cationic substance, and wherein the lipid bilayer membrane contains, as constituent components, a neutral lipid, and a lipid conjugate, a fatty acid conjugate or an aliphatic hydrocarbon conjugate of a water-soluble substance.
(28) A method for treating cancer or inflammatory disease, which comprises administering to a mammal a composition that comprises a lipidparticle that comprises:
   a complex particle that contains, as constituent components, a cationic substance-containing lead particle and a double-stranded nucleic acid molecule that contains a sense strand and an antisense strand; and
   a lipid bilayer membrane coating the complex particle,
   wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
   wherein the sense strand is a polynucleotide of 17 to 30 bases that contains a base sequence (sequence b) complementary to the base sequence of bases 1 to 17 in the 5' -end to 3'-end direction of the antisense strand, and the sugars in the sense strand are ribose, deoxyribose, or ribose whose hydroxyl group at the 2' position is substituted by a modifying group,
      (i) 0 to 30% of the sugars binding to the bases 1 to 8 in the 5'-end to 3'-end direction of sequence a are deoxyribose, or ribose whose hydroxyl group at the 2' position is substituted by a modifying group,
      (ii) 0 to 20% of the sugars binding to the bases 9 to 16 in the 5'-end to 3'-end direction of sequence a are deoxyribose, or a ribose whose hydroxyl group at the 2' position is substituted by a modifying group,
      (iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
      (iv) 10 to 70% of the sugars binding to the bases 1 to 17 in the 5'-end to 3'-end direction of sequence b are deoxyribose, or ribose whose hydroxyl group at the 2' position is substituted by a modifying group,
      (v) 30 to 100% of the sugars binding to the bases other than sequence b of the sense strand are deoxyribose, or ribose whose hydroxyl group at the 2' position is substituted by a modifying group, and
   the lipidparticle is a lipidparticle having a size that allows for intravenous administration, and the lipid bilayer membrane contains, as constituent components, a neutral lipid, and a lipid conjugate, a fatty acid conjugate or an aliphatic hydrocarbon conjugate of a water-soluble substance.
(29) The method for treating cancer or inflammatory disease according to any one of the above (24) to (28), wherein the lipid conjugate, the fatty acid conjugate, or the aliphatic hydrocarbon conjugate of a water-soluble substance is polyethylene glycol phosphatidyl ethanolamine.
(30) The method for treating cancer or inflammatory disease according to any one of the above (24) to (29), wherein (v) 50 to 70% of the sugars binding to the bases other than sequence b of the sense strand are deoxyribose, or ribose whose hydroxyl group at the 2' position is substituted by a modifying group.
(31) The method for treating cancer or inflammatory disease according to any one of the above (24) to (30), wherein the target gene associated with tumor or inflammation is a gene associated with angiogenesis.
(32) The method for treating cancer or inflammatory disease according to any one of the above (24) to (30), wherein the target gene associated with tumor or inflammation is a gene of any one of a vascular endothelial growth factor, a vascular endothelial growth factor receptor, a fibroblast growth factor, a fibroblast growth factor receptor, a platelet-derived growth factor, a platelet-derived growth factor receptor, a hepatocyte growth factor, a hepatocyte growth factor receptor, a Krüppel-like factor, an Ets transcription factor, a nuclear factor, and a hypoxia-inducible factor.
(33) The method for treating cancer or inflammatory disease according to any one of the above (24) to (32), wherein the mRNA is either human mRNA or mouse mRNA.

(34) Use of a composition for a manufacture of a therapeutic agent for cancer or inflammatory disease, the composition comprising a lipidparticle that comprises:
   a complex particle that contains, as constituent components, a lead particle and a double-stranded nucleic acid molecule that contains a sense strand and an antisense strand; and
   a lipid bilayer membrane coating the complex particle,
   wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
   wherein the sense strand is a polynucleotide of 17 to 30 bases that contains a base sequence (sequence b) complementary to the base sequence of bases 1 to 17 in the 5'-end to 3' -end direction of the antisense strand, and the sugars in the sense strand are ribose, deoxyribose, or ribose whose hydroxyl group at the 2' position is substituted by a modifying group,
      (i) 0 to 30% of the sugars binding to the bases 1 to 8 in the 5'-end to 3'-end direction of sequence a are deoxyribose, or ribose whose hydroxyl group at the 2' position is substituted by a modifying group,
      (ii) 0 to 20% of the sugars binding to the bases 9 to 16 in the 5'-end to 3'-end direction of sequence a are deoxyribose, or a ribose whose hydroxyl group at the 2' position is substituted by a modifying group,
      (iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
      (iv) 10 to 70% of the sugars binding to the bases 1 to 17 in the 5'-end to 3'-end direction of sequence b are deoxyribose, or ribose whose hydroxyl group at the 2' position is substituted by a modifying group,
      (v) 30 to 100% of the sugars binding to the bases other than sequence b of the sense strand are deoxyribose, or ribose whose hydroxyl group at the 2' position is substituted by a modifying group, and
   the lipidparticle is a lipidparticle having a size that allows for intravenous administration,
   wherein the constituent components of the lipid bilayer membrane are soluble in a certain polar organic solvent, and wherein the constituent components of the lipid bilayer membrane, and the complex particle are dispersible in a liquid that contains the polar organic solvent in a certain concentration, and the lipid bilayer membrane contains as constituent component, a lipid conjugate, a fatty acid conjugate, or an aliphatic hydrocarbon conjugate of a water-soluble substance.
(35) The use according to the above (34), wherein the polar organic solvent is an alcohol.
(36) The use according to the above (34), wherein the polar organic solvent is ethanol.
(37) The use according to any one of the above (34) to (36), wherein the lead particle is a lead particle that contains a cationic substance, and wherein the lipid bilayer membrane contains, as constituent components, a neutral lipid, and a lipid conjugate, a fatty acid conjugate or an aliphatic hydrocarbon conjugate of a water-soluble substance.
(38) Use of a composition for a manufacture of a therapeutic agent for cancer or inflammatory disease, the composition comprising a lipidparticle that comprises:
   a complex particle that contains, as constituent components, a cationic substance-containing lead particle and a double-stranded nucleic acid molecule that contains a sense strand and an antisense strand; and
   a lipid bilayer membrane coating the complex particle,
   wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
   wherein the sense strand is a polynucleotide of 17 to 30 bases that contains a base sequence (sequence b) complementary to the base sequence of bases 1 to 17 in the 5' -end to 3'-end direction of the antisense strand, and the sugars in the sense strand are ribose, deoxyribose, or ribose whose hydroxyl group at the 2' position is substituted by a modifying group,
      (i) 0 to 30% of the sugars binding to the bases 1 to 8 in the 5'-end to 3'-end direction of sequence a are deoxyribose, or ribose whose hydroxyl group at the 2' position is substituted by a modifying group,
      (ii) 0 to 20% of the sugars binding to the bases 9 to 16 in the 5'-end to 3'-end direction of sequence a are deoxyribose, or a ribose whose hydroxyl group at the 2' position is substituted by a modifying group,
      (iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
      (iv) 10 to 70% of the sugars binding to the bases 1 to 17 in the 5'-end to 3'-end direction of sequence b are deoxyribose, or ribose whose hydroxyl group at the 2' position is substituted by a modifying group,
      (v) 30 to 100% of the sugars binding to the bases other than sequence b of the sense strand are deoxyribose, or ribose whose hydroxyl group at the 2' position is substituted by a modifying group, and
   the lipidparticle is a lipidparticle having a size that allows for intravenous administration, and the lipid bilayer membrane contains, as constituent components, a neutral lipid, and a lipid conjugate, a fatty acid conjugate or an aliphatic hydrocarbon conjugate of a water-soluble substance.
(39) The use according to any one of the above (34) to (38), wherein the lipid conjugate, the fatty acid conjugate, or the aliphatic hydrocarbon conjugate of a water-soluble substance is polyethylene glycol phosphatidyl ethanolamine.
(40) The use according to any one of the above (34) to (39), wherein (v) 50 to 70% of the sugars binding to the bases other than sequence b of the sense strand are deoxyribose, or ribose whose hydroxyl group at the 2' position is substituted by a modifying group.
(41) The use according to any one of the above (34) to (40), wherein the target gene associated with tumor or inflammation is a gene associated with angiogenesis.
(42) The use according to any one of the above (34) to (40), wherein the target gene associated with tumor or inflammation is a gene of any one of a vascular endothelial growth factor, a vascular endothelial growth factor receptor, a fibroblast growth factor, a fibroblast growth factor receptor, a platelet-derived growth factor, a platelet-derived growth factor receptor, a hepatocyte growth factor, a hepatocyte growth factor receptor, a Krüppel-like factor, an Ets transcription factor, a nuclear factor, and a hypoxia-inducible factor.
(43) The use according to any one of the above (34) to (42), wherein the mRNA is either human mRNA or mouse mRNA.

(44) A method for suppressing the expression of a target gene, which comprises administering to mammals a composition that comprises:
   a lipidparticle encapsulating a double-stranded nucleic acid molecule that contains a sense strand and an antisense strand,
   wherein the antisense strand is a polynucleotide of 17 to 30 bases in which a sequence (sequence a) of bases 1 to 17 in the 5'-end to 3'-end direction is complementary to the sequence of the 17 contiguous bases of a target gene mRNA, and the sugars in the antisense strand are ribose, deoxyribose, or ribose whose hydroxyl group at the 2' position is substituted by a modifying group, and
   wherein the sense strand is a polynucleotide of 17 to 30 bases that contains a base sequence (sequence b) complementary to the base sequence of bases 1 to 17 in the 5'-end to 3'-end direction of the antisense strand, and the sugars in the sense strand are ribose, deoxyribose, or ribose whose hydroxyl group at the 2' position is substituted by a modifying group,
      (i) 0 to 30% of the sugars binding to the bases 1 to 8 in the 5'-end to 3'-end direction of sequence a are deoxyribose, or ribose whose hydroxyl group at the 2' position is substituted by a modifying group,
      (ii) 0 to 20% of the sugars binding to the bases 9 to 16 in the 5' -end to 3'-end direction of sequence a are deoxyribose, or a ribose whose hydroxyl group at the 2' position is substituted by a modifying group,
      (iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
      (iv) 10 to 70% of the sugars binding to the bases 1 to 17 in the 5' -end to 3'-end direction of sequence b are deoxyribose, or ribose whose hydroxyl group at the 2' position is substituted by a modifying group,
      (v) 30 to 100% of the sugars binding to the bases other than sequence b of the sense strand are deoxyribose, or ribose whose hydroxyl group at the 2' position is substituted by a modifying group, and
   the lipidparticle is a lipidparticle having a size that allows for intravenous administration, and contains a lipid bilayer membrane whose constituent component is a lipid conjugate, a fatty acid conjugate or an aliphatic hydrocarbon conjugate of a water-soluble substance.
(45) The method for suppressing the expression of the target geneaccording to the above (44), wherein (v) 50 to 70% of the sugars binding to the bases other than sequence b of the sense strand are deoxyribose, or ribose whose hydroxyl group at the 2' position is substituted by a modifying group.
(46) The method for suppressing the expression of the target geneaccording to the above (44) or (45), wherein the double-stranded nucleic acid molecule is a double-stranded nucleic acid molecule having an activity of suppressing the expression of the target gene by utilizing RNA interference (RNAi).
(47) The method for suppressing the expression of the target geneaccording to any one of the above (44) to (46), wherein the target gene is a gene associated with tumor or inflammation.
(48) The method for suppressing the expression of the target geneaccording to any one of the above (44) to (47), wherein the target gene is a gene associated with angiogenesis.
(49) The method for suppressing the expression of the target geneaccording to any one of the above (44) to (47), wherein the target gene is a gene of any one of a vascular endothelial growth factor, a vascular endothelial growth factor receptor, a fibroblast growth factor, a fibroblast growth factor receptor, a platelet-derived growth factor, a platelet-derived growth factor receptor, a hepatocyte growth factor, a hepatocyte growth factor receptor, a Krüppel-like factor, an Ets transcription factor, a nuclear factor and a hypoxia-inducible factor.
(50) The method for suppressing the expression of the target geneaccording to any one of the above (44) to (49), wherein the mRNA is either human mRNA or mouse mRNA.
(51) The method for suppressing the expression of the target geneaccording to any one of the above (44) to (50), wherein the lipidparticle encapsulating the double-stranded nucleic acid molecule is a lipidparticle that comprises:
   a complex particle that contains a lead particle and the double-stranded nucleic acid molecule as constituent components; and
   a lipid bilayer membrane coating the complex particle,
   wherein constituent components of the lipid bilayer membrane are soluble in a certain polar organic solvent, and wherein the constituent components of the lipid bilayer membrane, and the complex particle are dispersible in a liquid that contains the polar organic solvent in a certain concentration.
(52) The method for suppressing the expression of the target geneaccording to the above (51), wherein the polar organic solvent is an alcohol.
(53) The method for suppressing the expression of the target geneaccording to the above (51), wherein the polar organic solvent is ethanol.
(54) The method for suppressing the expression of the target geneaccording to any one of the above (51) to (53), wherein the lead particle is a lead particle that contains a cationic substance, and wherein the lipid bilayer membrane coating the complex particle contains, as constituent components, a neutral lipid, and a lipid conjugate, a fatty acid conjugate or an aliphatic hydrocarbon conjugate of a water-soluble substance.
(55) The method for suppressing the expression of the target geneaccording to any one of the above (44) to (50), wherein the lipidparticle encapsulating the double-stranded nucleic acid molecule is a lipidparticle that comprises: a complex particle that contains a cationic substance-containing lead particle and the double-stranded nucleic acid molecule as constituent components; and a lipid bilayer membrane coating the complex particle,
   wherein the lipid bilayer membrane coating the complex particle contains, as constituent components, a neutral lipid, and a lipid conjugate, a fatty acid conjugate or an aliphatic hydrocarbon conjugate of a water-soluble substance.
(56) The method for suppressing the expression of the target geneaccording to any one of the above (44) to (55), wherein the lipid conjugate, the fatty acid conjugate or the aliphatic hydrocarbon conjugate of a water-soluble substance is polyethylene glycol phosphatidyl ethanolamine.

### Effects of the Invention

Target gene expression can be suppressed by administering the composition of the present invention to mammals and the like.

### Brief Description of the Drawings

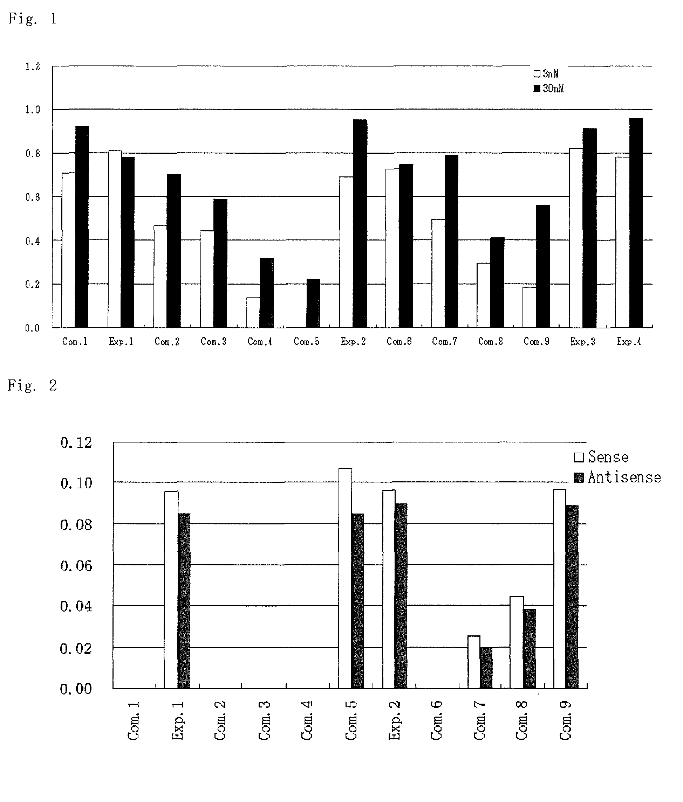

Fig. 1 shows the siRNA activities of double-stranded nucleic acid molecules used in Examples 1 to 4 and Comparative Examples 1 to 9; the vertical axis represents BCL-2 mRNA expression suppressing rate (ratio).
Fig. 2 shows the concentrations of double-stranded nucleic acid molecules in blood measured after 3 hours from the administration of the preparation obtained from Comparative Example 1 as a secondly administered PEG-modified lipidparticle 7 days after the administration of the preparations obtained from Examples 1 and 2 and Comparative Examples 1 to 9 to mice; the vertical axis represents the blood concentration of the double-stranded nucleic acid molecule (µmol/L).

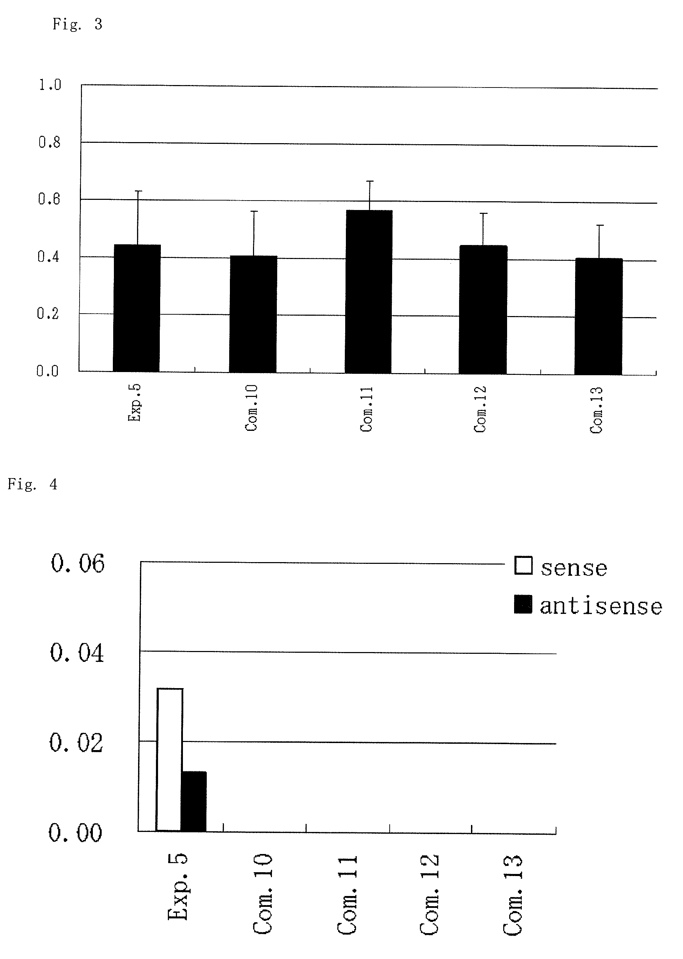

Fig. 3 shows the siRNA activities of double-stranded nucleic acid molecules used in Example 5 and Comparative Examples 10 to 13; the vertical axis represents BCL2 mRNA expression level ratio (ratio).
Fig. 4 shows the concentrations of double-stranded nucleic acid molecules in blood measured after 3 hours from the administration of the preparations obtained from Example 5 and Comparative Examples 10 to 13 as secondly administered PEG-modified lipidparticles 7 days after the administration of the preparations obtained from Example 5 and Comparative Examples 10 to 13 to mice, receptivity; the vertical axis represents the blood concentration of the double-stranded nucleic acid molecule (µmol/L).

### [Mode for Carrying Out the Invention]

The target gene used in the present invention is not particularly limited as long as it is a gene which produces and expresses mRNA in mammals. For example, the target gene is preferably a gene associated with tumor or inflammation, more preferably a gene associated with angiogenesis, and the like. Examples include genes that code for proteins such as a vascular endothelial growth factor (hereinafter referred to as "VEGF" ), a vascular endothelial growth factor receptor (hereinafter referred to as "VEGFR" ), a fibroblast growth factor, a fibroblast growth factor receptor, a platelet-derived growth factor, a platelet-derived growth factor receptor, a hepatocyte growth factor, a hepatocyte growth factor receptor, a Krüppel-like factor (hereinafter referred to as "KLF"), an Ets transcription factor, a nuclear factor, and a hypoxia-inducible factor, and the like. Specific examples include VEGF gene, VEGFR gene, fibroblast growth factor gene, fibroblast growth factor receptor gene, platelet-derived growth factor gene, platelet-derived growth factor receptor gene, hepatocyte growth factor gene, hepatocyte growth factor receptor gene, KLF gene, Ets transcription factor gene, nuclear factor gene, hypoxia-inducible factor gene, and the like. The preferred target gene is, for example, VEGF gene, VEGFR gene, and KLF gene, more preferably KLF gene, further preferably KLF5 gene.
The KLF family is a family of transcriptional factors, which is characterized in that it has a zinc finger motif at the C-terminus thereof, and examples thereof that have been known include KLF1, KLF2, KLF3, KLF4, KLF5, KLF6, KLF7, KLF8, KLF9, KLF10, KLF11, KLF12, KLF13, KLF14, KLF15, KLF 16 and the like. It has been reported that, in mammals, the KLF family plays an important role in differentiation of various types of tissues or cells, such as erythrocytes, vascular endothelial cells, smooth muscle, skin, and lymphocytes, and also in formation of the pathologic conditions of various types of diseases such as cancer, cardiovascular diseases, cirrhosis, renal diseases, and immune-mediated diseases (The Journal of Biological Chemistry, Vol. 276, No. 37, pp. 34355-34358, 2001; Genome Biology, Vol. 4, No. 2, p. 206, 2003).

Among the KLF family members, KLF5 is also referred to as BTEB2 (basic transcriptional element binding protein 2) or IKLF (intestinal-enriched Krüppel-like factor). The expression of KLF5 in vascular smooth muscle is controlled at the development stage thereof. KLF5 is highly expressed in the vascular smooth muscle of a fetus, whereas its expression is not found in the vascular smooth muscle of a healthy adult. In addition, in the case of the smooth muscle of intima of a blood vessel regenerated after denudation by a balloon catheter, KLF5 is highly expressed. Also, in the smooth muscle in lesions due to arteriosclerosis or restenosis, KLF5 is expressed (Circulation, Vol. 102, No. 20, pp. 2528-2534, 2000).

VEGF, discovered by Ferrara and others in 1983, is a growth factor specific to vascular endothelial cells. In the same year, Senger and Dvorak, with several others, discovered a factor having vascular permeability activity, and they named this factor a VPF (vascular permeability factor). Amino acid sequence analysis of the proteins revealed that these were the same. VEGF facilitates growth by binding to receptors on the endothelial cells lining inside blood vessels. VEGF has activity not only in the formation of blood vessels during the fetal period, but also in the formation of pathologic blood vessels. For example, when cancer grows to a certain size and becomes deficient in oxygen, VEGF and VFGF receptor production increases and induces angiogenesis. Further, the vascular permeability increasing effect is also considered to be a cause of cancerous ascites. VEGF also play a role in the formation of new blood vessels in the retina during the propagation of diabetes mellitus. Specifically, VEGF is a protein that forms new blood vessels. VEGF expression induced by a low-oxygen state thus has an important role in angiogenesis. Aside from its role in angiogenesis, involvement of VEGF factor is strongly indicated in explaining the mechanism of edema seen in tumor or inflammatory lesions and the like.

On the other hand, VEGFRs are present in vascular endothelial cells or cancer cells themselves. Upon binding of VEGF to VEGFR, the receptors themselves are phosphorylated (activated), and signaling the cells to, for example, grow or migrate. It is known that inhibiting the receptor phosphorylation inhibits the signaling in the cells, and thus inhibits angiogenesis.

Examples of the target gene include B-CELL CLL/LYMPHOMA (hereinafter referred to as "bcl") genes, and the preferred target gene is, for example, bcl2 gene.
BCL2 is a mitochondria inner membrane protein that inhibits apoptotic cell death in some type of cell. Inhibition of apoptosis by high expression of bcl2 gene is considered to be a cause of diseases such as cancer and hematological malignant disease. In fact, large production of BCL2 is found in various solid cancers, including lymphatic sarcoma, prostate cancer, breast cancer, lung cancer, colon cancer, rectum cancer, and the like (T. J. McDonnell et al., Cancer Research, December 15, 1992, Vol. 52, No. 24, p. 6940-6944). Involvement of bcl-2 gene expression in apoptosis in the thymus gland is also indicated (Kanavaros et al., Histol. Histopathol. 16(4) : 1005-12 (Oct. 2001)).
Because of the apoptosis suppressing effect, cell death is not induced in cells that produce high levels of BCL2, and as a result drug resistance to various anticancer agents occurs. On the other hand, suppressing bcl-2 gene production in prostate cancer cells is known to suppress cell growth and helps induce apoptosis (Shi et al., Cancer Biother. Radiopharm., 16(5): 421-9 (Oct. 2001)). Thus, a method that suppresses bcl-2 gene expression can be an effective therapeutic or preventive method in diseases that require the promotion of apoptosis for the cure, such as in solid cancers and hematological malignant diseases.

The target gene used in the present invention is preferably, for example, a gene expressed in the liver, lungs, kidneys, or spleen. Examples include a gene associated with tumor or inflammation, hepatitis B virus genome, hepatitis C virus genome, and genes that encode proteins such as apolipoprotein (APO), hydroxymethylglutaryl (HMG) CoA reductase, kexin type 9 serine protease (PCSK9), factor 12, glucagon receptor, glucocorticoid receptor, leukotriene, thromboxane A2 receptor, histamine H1 receptor, carbonic anhydrase, angiotensin converting enzyme, renin, p53, tyrosine phosphatase (PTP), sodium-dependent glucose transport carrier, tumor necrosis factor, and interleukin and the like.

The double-stranded nucleic acid molecule used in the present invention may be a double-stranded nucleic acid molecule that contains a sense strand and an antisense strand,
wherein the antisense strand is a polynucleotide of 17 to 30, preferably 19 to 25 bases in which a sequence (sequence a) of bases 1 to 17 in the 5'-end to 3'-end direction is complementary to the sequence of the 17 contiguous bases of a target gene's mRNA, and the sugars in the antisense strand are ribose, deoxyribose, or ribose whose hydroxyl group at the 2' position is substituted by a modifying group, and
wherein the sense strand is a polynucleotide of 17 to 30, preferably 19 to 25 bases that contains a base sequence (sequence b) complementary to the base sequence of bases 1 to 17 in the 5' -end to 3' -end direction of the antisense strand, and the sugars in the sense strand are ribose, deoxyribose, or ribose whose hydroxyl group at the 2' position is substituted by a modifying group,
(i) 0 to 30% of the sugars binding to the bases 1 to 8 in the 5'-end to 3'-end direction of sequence a are deoxyribose, or ribose whose hydroxyl group at the 2' position is substituted by a modifying group,
(ii) 0 to 20% of the sugars binding to the bases 9 to 16 in the 5'-end to 3'-end direction of sequence a are deoxyribose, or a ribose whose hydroxyl group at the 2' position is substituted by a modifying group, (Preferably, 0 to 20% of the sugars binding to the bases 9 to 16 in the 5'-end to 3'-end direction of sequence a are deoxyribose, or ribose whose hydroxyl group at the 2' position is substituted by a modifying group, and 0% of the sugars binding to the bases 9 to 11 are deoxyribose, or ribose whose hydroxyl group at the 2' position is substituted by a modifying group),
(iii) 30 to 100%, preferably 40% or more of the sugars binding to the bases from base 17 to the 3'-end in the 5'-end to 3'-end direction of the antisense strand are deoxyribose, or ribose whose hydroxyl group at the 2' position is substituted by a modifying group,
(iv) 10 to 70%, preferably, 30 to 60% of the sugars binding to the bases 1 to 17 in the 5' -end to 3' -end direction of sequence b are deoxyribose, or ribose whose hydroxyl group at the 2' position is substituted by a modifying group, (Preferably, 0% of the sugars binding to the bases 9 to 11 are deoxyribose, or ribose whose hydroxyl group at the 2' position is substituted by a modifying group), and
(v) 30 to 100%, preferably 40% or more of the sugars binding to the bases other than sequence b of the sense strand are deoxyribose, or ribose whose hydroxyl group at the 2' position is substituted by a modifying group.

Note that, as used herein, "0% of the sugars binding to the bases m to n (where m and n are arbitrary numbers) are deoxyribose, or ribose whose hydroxyl group at the 2' position is substituted by a modifying group" means that the sugars binding to the bases m to n do not contain deoxyribose, or ribose whose hydroxyl group at the 2' position is substituted by a modifying group, namely the sugars binding to the bases m to n are all ribose.

The double-stranded nucleic acid molecule used in the present invention may be preferably a double-stranded nucleic acid molecule having an activity of suppressing the expression of the target gene by utilizing RNA interference (RNAi).
The base sequence of the nucleotides added adjacent to the 3'-end of sequence of the antisense strand may be a base sequence complementary to the target gene's mRNA base sequence adjacent to sequence a. This structure is preferred from the standpoint of the target gene expression suppressing action utilizing RNA interference (RNAi). Specifically, the antisense strand is a strand in which the sequence of at least bases 1 to 17 in the 5'-end to 3'-end direction is complementary to the sequence of 17 contiguous bases of the target gene's mRNA. Preferably, the antisense strand is any one of a strand in which the sequence of bases 1 to 19 in the 5'-end to 3'-end direction is complementary to the sequence of 19 contiguous bases of the target gene's mRNA, a strand in which the sequence of bases 1 to 21 in the 5'-end to 3' -end direction is complementary to the sequence of 21 contiguous bases of the target gene's mRNA, and a strand in which the sequence of bases 1 to 25 is complementary to the sequence of 25 contiguous bases of the target gene's mRNA.

When bases other than sequence b of the sense strand are present face to face with the bases of the antisense strand, it is preferable that the bases other than sequence b of the sense strand form complementary base pairs with the opposite bases of the antisense strand.
Further, in the double-stranded nucleic acid molecule used in the present invention, 10 to 70%, preferably 15 to 60%, more preferably 20 to 50% of the sugars in the double-stranded nucleic acid molecule are ribose substituted by a modifying group at 2' position. As used herein, the substitution by a modifying group at the 2' position of the ribose means substitution of the hydroxyl group by a modifying group at 2' position. The modifying group may have the same or different configuration as the hydroxyl group at the 2' position of the ribose, and preferably has the same configuration as the hydroxyl group at the 2' position of the ribose.

The double-stranded nucleic acid molecule used in the present invention encompasses derivatives in which the oxygen atoms and the like contained in the phosphate moiety, ester moiety, and the like in the nucleic acid structure is substituted with other atoms, for example, such as a sulfur atom.

Examples of the modifying group in the present invention include 2'-cyano, 2' -alkyl, 2'-substituted alkyl, 2'-alkenyl, 2'-substituted alkenyl, 2'-halogen, 2'-O-cyano, 2'-O-alkyl, 2'-O-substituted alkyl, 2'-O-alkenyl, 2'-O-substituted alkenyl, 2'-S-alkyl, 2'-S-substituted alkyl, 2'-S-alkenyl, 2' -S-substituted alkenyl, 2'-amino, 2'-NH-alkyl, 2'-NH-substituted alkyl, 2' -NH-alkenyl, 2' -NH-substituted alkenyl, 2' -SO-alkyl, 2' -SO-substituted alkyl, 2'-carboxy, 2' -CO-alkyl, 2'-CO-substituted alkyl, 2'-Se-alkyl, 2'-Se-substituted alkyl, 2'-SiH₂-alkyl, 2' -SiH₂-substituted alkyl, 2'-ONO₂, 2'-NO₂, 2'-N₃, 2'-amino acid residue (amino acid with the hydroxyl group removed from the carboxylic acid), and 2'-O-amino acid residue (having the same definition as above), and include a peptide nucleic acid (PNA) [Acc. Chem. Res., 32, 624 (1999)], an oxy-peptide nucleic acid (OPNA) [J. Am. Chem. Soc., 123, 4653 (2001)], a peptide ribonucleic acid (PRNA) [J. Am. Chem. Soc., 122, 6900 (2000)], and the like. The ribose with the substitution by a modifying group at 2' position in the present invention also encompasses bridged nucleic acids (BNAs) of a structure in which the modifying group at 2' position is bridged to the 4' carbon atom, specifically, locked nucleic acids (LNAs) in which the oxygen atom at 2' position is bridged to the 4' carbon atom via methylene, ethylene bridged nucleic acids (ENAs) [Nucleic Acid Research, 32, e175 (2004)], and the like.
The preferred modifying group in the present invention include 2'-cyano, 2'-halogen, 2'-O-cyano, 2'-alkyl, 2'-substituted alkyl, 2'-O-alkyl, 2'-O-substituted alkyl, 2'-O-alkenyl, 2'-O-substituted alkenyl, -Se-alkyl, and -Se-substituted alkyl. More preferred examples include 2' -cyano, 2' -fluoro, 2'-chloro, 2' -bromo, 2'-trifluoromethyl, 2'-O-methyl, 2'-O-ethyl, 2'-O-isopropyl, 2'-O-trifluoromethyl, 2'-O-[2-(methoxy) ethyl], 2'-O-(3-aminopropyl), 2'-O-(2-[N, N-dimethyl]aminooxy) ethyl, 2'-O-[3-(N,N-dimethylamino)propyl], 2'-O-[2-[2-(N, N-dimethylamino)ethoxy]ethyl], 2'-O-[2-(methylamino)-2-oxoethyl], 2'-Se-methyl, and the like. Even more preferred are 2'-O-methyl, 2'-O-ethyl, 2'-fluoro, and the like. 2'-O-methyl and 2'-O-ethyl are most preferable.
The preferred range of the modifying group in the present invention may also be defined based on its size. Modifying groups of a size corresponding to from the size of fluoro to the size of -O-butyl are preferable, and modifying groups of a size corresponding to the size of -O-methyl to the size of -O-ethyl are more preferable.

Examples of the alkyl in the modifying group include linear or branched alkyl having 1 to 6 carbon atoms, for example, such as methyl, ethyl, propyl, isopropyl, butyl, isobutyl, sec-butyl, tert-butyl, pentyl, isopentyl, neopentyl, tert-pentyl, hexyl and the like. Preferred examples include methyl, ethyl, propyl, isopropyl, isobutyl, sec-butyl, tert-butyl, pentyl, neopentyl, tert-pentyl, and the like. Examples of the alkenyl in the modifying group include linear or branched alkenyl having 1 to 6 carbon atoms, for example, such as vinyl, allyl, isopropenyl and the like.
Examples of the halogen include a fluorine atom, a chlorine atom, a bromine atom, and an iodine atom.
Examples of the amino acid include aliphatic amino acids (specifically, glycine, alanine, valine, leucine, isoleucine, and the like), hydroxy amino acids (specifically, serine, threonine, and the like), acidic amino acids (specifically, aspartic acid, glutamic acid, and the like), acidic amino acid amides (specifically, asparagine, glutamine, and the like), basic amino acids (specifically, lysine, hydroxylysine, arginine, ornithine, and the like), sulfur-containing amino acids (specifically, cysteine, cystine, methionine, and the like), imino acids (specifically, proline, 4-hydroxy proline, and the like), and the like.
Examples of the substituents in the substituted alkyl and the substituted alkenyl include halogen (having the same definition as above), hydroxy, sulfanyl, amino, oxo, -O-alkyl (the alkyl moiety of the -0-alkyl has the same definition as above), -S-alkyl (the alkyl moiety of the -S-alkyl has the same definition as above), -NH-alkyl (the alkyl moiety of the -NH-alkyl has the same definition as above), dialkylaminooxy (the two alkyls of the dialkylaminooxy may be the same or different, and have the same definition as above), dialkylamino (the two alkyls of the dialkylamino may be the same or different, and have the same definition as above), dialkylaminoalkyleneoxy (the two alkyls of the dialkylaminoalkyleneoxy may be the same or different, and have the same definition as above; the alkylene means a group wherein the hydrogen atom is removed from the above-defined alkyl), and the like, and the number of the substituent is preferably 1 to 3.
Note that, in the present invention, the ribose substituted by a modifying group at 2' position in the sugars of the double-stranded nucleic acid molecule is encompassed in the double-stranded nucleic acid molecule used in the present invention, regardless of the method of production, raw material, or intermediate, as long as the end products have the same structure. Accordingly, those using DNA or deoxyribose as the raw material or intermediate are also encompassed in the double-stranded nucleic acid molecule used in the present invention, as long as the end products have the same structure. Therefore, in the present invention, the ribose substituted by a modifying group at 2' position encompasses a deoxyribose in which the hydrogen at the 2' position is replaced with a modifying group.

In the double-stranded nucleic acid molecule used in the present invention, it is more preferable to control the distribution of the riboses substituted by a modifying group at 2' position in order to minimize the number of adjacent of these riboses. It is preferable, however, that the sugars binding to 2 to 7 bases at the 3'-end of the antisense strand and at the 5'-end and 3'-end of the sense strand occur adjacently as deoxyribose, or as ribose whose hydroxyl group at the 2' position is substituted by a modifying group.
It is also preferable that the ribose substituted by a modifying group is only one of the bases forming an opposing complementary base pair. It is preferable, however, that the both bases of an opposing complementary base pair at the 5'-end and/or 3'-end of the antisense strand or sense strand are deoxyribose, or ribose whose hydroxyl group at the 2' position is substituted by a modifying group.

The double-stranded nucleic acid molecule used in the present invention may have (A) blunt ends at the 5'-end of the antisense strand and the 3'-end of the sense strand, and at the 3' -end of the antisense strand and the 5'-end of the sense strand, forming complementary base pairs on the opposite strands, (b) overhangs of 1 to 6, preferably 2 to 4 nucleotides, the same or different, added at the 3'-ends of the antisense strand and sense strand without forming base pairs on the opposite strands, or (C) a combination of a blunt end and an overhang. The nucleotide bases added may be one or more bases selected from guanine, adenine, cytosine, thymine, and uracil, and the sugar binding to each base may be any of ribose, deoxyribose, and ribose whose hydroxyl group at the 2' position is substituted by a modifying group. More preferably, the nucleotides added are one or two of urydylic acid (U) and deoxythymidylic acid (dT). The base sequence of the nucleotides added adjacent to the 3'-end of the sense strand may be the same as the base sequence adjoining the sequence a in mRNA, and this structure is more preferable.
The base sequence of the nucleotides added adjacent to the 3'-end of the antisense strand may be a base sequence complementary to the base sequence corresponding to the mRNA of the target gene, and this structure is more preferable. In the present invention, it is most preferable that the base sequence of the antisense strand be a base sequence completely complementary to the base sequence corresponding to the mRNA of the target gene.

The sugar binding to the bases at the 5'-end of the antisense strand and sense strand may be that in which the hydroxyl group at the 5' position is modified with a phosphate group, the modifying group, or a group that becomes the phosphate group or the modifying group by the action of a nucleolytic enzyme or the like in the body.
The sugar binding to the bases at the 3'-end of the antisense strand and sense strand may be that in which the hydroxyl group at the 3' position is modified with a phosphate group, the modifying group, or a group that becomes the phosphate group or the modifying group by the action of a nucleolytic enzyme or the like in the body.

The double-stranded nucleic acid molecule of the present invention may be one that occurs after being decomposed by a nucleolytic enzyme or the like in the body. The double-stranded nucleic acid molecule before being decomposed represents a prodrug of the double-stranded nucleic acid molecule of the present invention.
The prodrug of the double-stranded nucleic acid molecule may be, for example, a double-stranded nucleic acid molecule that contains:
4 to 8, preferably 5 to 6 nucleotides, the same or different, added to the 5'-end of sequence a of the antisense strand;
the same number of bases added at the 3'-end of sequence b of the sense strand, complementary to the base sequence of the antisense strand;
two bases added to the 3'-end of sequence a of the antisense strand in the same sequence as the base sequence corresponding to the mRNA of the target gene;
two bases added to the 5' -end of sequence b of the sense strand in the sequence complementary to the base sequence of the antisense strand; and
overhangs of 1 to 6, preferably 2 to 4 nucleotides, the same or different, added to the 3'-end of the antisense strand without base pairing, preferably, the hydroxyl group at the 5' position of the sugar binding to the base at the 5'-end of the sense strand is phosphorylated.
The prodrug becomes the double-stranded nucleic acid molecule of the present invention after removing all the nucleotides added to the 5'-end of sequence a of the antisense strand, and all the nucleotides, except for the first and second nucleotides, added to the 3'-end of sequence b of the sense strand, using a dicer.

Another example of the prodrug is a single-stranded nucleic acid molecule with a hairpin structure in which the antisense strand and the sense strand are joined by a spacer oligonucleotide, and in which 1 to 6, preferably 2 to 4 nucleotides are added to the 3'-end. Preferably, the spacer oligonucleotide is a single-stranded nucleic acid molecule of 6 to 12 bases, preferably with two uracils representing the sequence at the 5'-end. An example of the spacer oligonucleotide is a double-stranded nucleic acid molecule with the sequence UUCAAGAGA. Either of the two double-stranded nucleic acid molecules joined by the spacer oligonucleotide may be on the 5'-end.

The double-stranded nucleic acid molecule used in the present invention may be produced using known RNA or DNA synthesis methods or known RNA or DNA modification methods. For example, the double-stranded nucleic acid molecule can be obtained by using chemical synthesis services provided by, for example, Hokkaido System Science Co., Ltd.

The lipidparticle in the composition of the present invention (hereinafter referred to as "lipidparticle A" ) is preferably a lipidparticle that encapsulates a double-stranded nucleic acid molecule that contains a sequence consisting of 17 to 30 contiguous bases of a target gene's mRNA and a base sequence complementary to the sequence. The lipidparticle A is not particularly limited, as long as it is capable of reaching a tissue or an organ containing an expression site of the target gene. Examples include a lipidparticle that comprises a lipid bilayer membrane whose constituent component is a lipid conjugate, a fatty acid conjugate, or an aliphatic hydrocarbon conjugate of a water-soluble substance, specifically, a lipidparticle surface-modified with a water-soluble polymer such as polyethylene glycol (PEG).
Examples of lipidparticle A used in the present invention include liposome, lipid micelle and the like. Examples of lipid micelle include lipidsphere and emulsion particle, which the surface with the external aqueous phase is preferred lipid monolayer membrane or lipid bilayer membrane.

Examples of lipidparticle A include a lipidparticle produced by reverse-phase evaporation treatment of a water-in-oil type (W/O) emulsion formed by adding a polyethylene glycolated phospholipid, a neutral lipid, and water to a cationic lipid/double-stranded nucleic acid molecule complex in chloroform layer, the complex in chloroform layer being prepared by dissolving cationic lipid in chloroform, adding a double-stranded nucleic acid molecule aqueous solution and methanol, and separating the chloroform layer (see Japanese translation of PCT international application, No. 2002-508765), a lipidparticle produced from a double-stranded nucleic acid molecule-encapsulating lipidparticle prepared by lowering ethanol concentration to 20 v/v% after adding lipid (in ethanol) to a solution of double-stranded nucleic acid molecule dissolved in an acidic electrolyte aqueous solution, which double-stranded nucleic acid molecule-encapsulating lipidparticle is then subjected to filtration for sizing, dialyzed to remove the excess ethanol, and subjected again to dialysis at an increased sample pH to remove the double-stranded nucleic acid molecule adhering to the lipidparticle surface (see Japanese translation of PCT international application, No. 2002-501511, and Biochimica et Biophysica Acta, 2001, Vol. 1510, p. 152-166), a lipidparticle including complex particles that contain a lead particle and the double-stranded nucleic acid molecule, and a lipid bilayer membrane for encapsulating the complex particles (see WO02/28367, and WO2006/080118), and the like. The lipidparticle A is preferably a lipidparticle including complex particles that include a lead particle and the double-stranded nucleic acid molecule, and a lipid bilayer membrane for encapsulating the complex particles. It is more preferable that the constituent components of the lipid bilayer membrane be soluble in a certain polar organic solvent, and that the constituent components of the lipid bilayer membrane and the complex particles be dispersible in a liquid that contains the polar organic solvent in a certain concentration. It is also preferable that the lipidparticle A be a lipidparticle that comprises complex particles containing a cationic substance-containing lead particle and the double-stranded nucleic acid molecule as constituent components, and a lipid bilayer membrane coating the complex particles, and in which the lipid bilayer membrane contains, as constituent components, a neutral lipid, and a lipid conjugate, a fatty acid conjugate, or an aliphatic hydrocarbon conjugate of a water-soluble substance. More preferably, the constituent components of the lipid bilayer membrane are soluble in a certain polar organic solvent, and the constituent components of the lipid bilayer membrane and the complex particles are dispersible in a liquid that contains the polar organic solvent in a certain concentration.
As used herein, the terms "dispersed" means dispersing insolubly.

It has been reported that the lipidparticles exemplified above can be delivered to tumor- or inflammation-bearing tissues or organs, specifically, to solid tumors, solid cancers, or inflammation sites in blood vessels or in the vicinity of blood vessels, and the like. The foregoing lipidparticles can therefore be preferably used when the target gene is a gene associated with tumor or inflammation.
Further, the lipidparticles exemplified above are reported to have high retention in the blood. Thus, these lipidparticles have a high possibility of being delivered to any tissue or organ through systemic circulation, and thus a gene that can be targeted is not limited.

The lead particle in the present invention is a fine particle of, for example, lipid assembly, liposome (hereinafer refferd to as "liposome B"), polymeric micelle, and the like, preferably a fine particle of liposome B. The lead particle in the present invention may be a complex as a combination of two or more of a lipid assembly, a liposome B, a polymeric micelle, and the like. For example, the lead particle may be a polymeric micelle as a complex that contains the constituent component lipids of a lipid assembly, a liposome B, and the like, or a lipid assembly, liposome B, and the like as a complex that contains the constituent component polymer of a polymeric micelle.

The lipid assembly or liposome B as the lead particle is preferably one configured from a polar lipid or the like that is amphiphatic (having both hydrophilic and hydrophobic properties), and that assumes a lipid bilayer structure in water. The lipid is not particularly limited, and may be any of a simple lipid, a complex lipid and a derived lipid, and examples thereof include a phospholipid, a glyceroglycolipid, a sphingoglycolipid, a sphingoid, a sterol, a cationic lipid, and the like. Preferred examples include a phospholipid and a cationic lipid.

Examples of the phospholipid as the constituting lipid of the lead particle include natural and synthetic phospholipids such as phosphatidylcholine (specifically, soybean phosphatidylcholine, egg yolk phosphatidylcholine (EPC), distearoyl phosphatidylcholine, dipalmitoyl phosphatidylcholine, palmitoyloleoyl phosphatidylcholine (POPC), dimyristoyl phosphatidylcholine, dioleoyl phosphatidylcholine and the like), phosphatidylethanolamine (specifically, distearoyl phosphatidylethanolamine (DSPE), dipalmitoyl phosphatidylethanolamine (DPPE), dioleoyl phosphatidylethanolamine (DORE), dimyristoylphosphoethanolamine (DMPE), 16-0-monomethyl PE, 16-0-dimethyl PE, 18-1-trans PE, palmitoyloleoyl-phosphatidylethanolamine (POPE), 1-stearoyl-2-oleoyl-phosphatidylethanolamine (SOPE) and the like), glycerophospholipid (specifically, phosphatidylserine, phosphatidic acid, phosphatidylglycerol, phosphatidylinositol, palmitoyloleoylphosphatidylglycerol (POPG), lysophosphatidylcholine and the like), sphingophospholipid (specifically sphingomyelin, ceramide phosphoethanolamine, ceramide phosphoglycerol, ceramide phosphoglycerophosphate and the like), glycerophosphono lipid, sphingophosphonolipid, natural lecithin (specifically, egg yolk lecithin, soybean lecithin and the like), and hydrogenated phospholipid (specifically hydrogenated soybean phosphatidylcholine and the like).

Examples of the glyceroglycolipid as the constituting lipid of the lead particle include sulfoxyribosyl glyceride, diglycosyl diglyceride, digalactosyl diglyceride, galactosyl diglyceride, glycosyl diglyceride and the like.

Examples of the sphingoglycolipid as the constituting lipid of the lead particle include galactosyl cerebroside, lactosyl cerebroside, ganglioside, and the like.

Examples of the sphingoid as the constituting lipid of the lead particle include sphingan, icosasphingan, sphingosine, derivatives thereof, and the like. Examples of the derivatives thereof include those in which -NH₂ of sphingan, icosasphingan, sphingosine or the like is replaced with -NHCO(CH₂)ₓCH₃ (in the formula, x represents an integer of 0 to 18, in particular, 6, 12 or 18 is preferred), and the like.

Examples of the sterol as the constituting lipid of the lead particle include cholesterol, dihydrocholesterol, lanosterol, β-sitosterol, campesterol, stigmasterol, brassicasterol, ergocasterol, fucosterol, 3β-[N-(N',N'- dimethylaminoethyl)carbamoyl]cholesterol (DC-Chol), and the like.

Concerning the cationic lipid in the lipid forming the lead particle, the amphiphatic polar lipid that has both hydrophilic and hydrophobic properties, and assumes a lipid bilayer structure in water may have a structure that includes a primary amine, secondary amine, tertiary amine, quaternary ammonium, or nitrogen atom-containing heterocyclic ring or the like at the hydrophilic moiety. Examples include *N*-[1-(2, 3-dioleoylpropyl)]-*N*,*N*,*N*-trimethylammonium chloride (DOTAP), *N*-[1-(2,3-dioleoylpropyl)]-*N*,*N*-dimethylamine (DODAP), *N*-[1-(2, 3-dioleyloxypropyl)]-*N*,*N*,*N*-trimethylammonium chloride (DOTMA), 2,3-dioleyloxy-*N*-[2-(sperminecarboxyamide)ethyl]-*N*,*N*-dimethyl-1-propanaminium trifluoroacetate (DOSPA), *N*-[1-(2,3-ditetradecyloxypropyl)]-*N*,*N*-dimethyl-*N-*hydroxyethyl ammonium bromide (DMRIE), *N*-[1-(2,3-dioleyloxypropyl)]-*N*,*N-*dimethyl-*N*-hydroxyethyl ammonium bromide (DORIE), 1,2-dilinoleyloxy-*N*,*N-*dimethylaminopropane (DLinDMA), 1,2-dilinolenyloxy-*N*,*N*-dimethylaminopropane (DLenDMA), didecyldimethylammonium chloride, distearyldimethylammonium chloride, and DC-Chol. Preferably, the amphiphatic polar lipid is one or more selected from *N*-[1-(2,3-dioleoylpropyl)]-*N*,*N*,*N*-trimethylammonium chloride, *N*-[1-(2,3-dioleoylpropyl)]-*N*,*N*-dimethylamine, *N*-[1-(2,3-dioleyloxypropyl)]-*N*,*N*,*N-*trimethylammonium chloride, *N*-[1-(2,3-ditetradecyloxypropyl)]-*N*,*N*-dimethyl-*N-*hydroxyethyl ammonium bromide, and DC-Chol.

The liposome B may contain a membrane stabilizer such as a sterol including cholesterol, an antioxidant such as tocopherol or the like, as needed. The stabilizers may be used either alone or in combinations of two or more.

Examples of the lipid assembly include a spherical micelle, a spherical reversed micelle, a sausage-shaped micelle, a sausage-shaped reversed micelle, a plate-shaped micelle, a plate-shaped reversed micelle, hexagonal I, hexagonal II and an associated product of two or more lipid molecules.

The polymer micelle may be one or more micelles selected from, for example, protein, albumin, dextran, polyfect, chitosan, dextran sulfate; and polymers, for example, such as poly-L-lysine, polyethyleneimine, polyaspartic acid, a copolymer of styrene and maleic acid, a copolymer of isopropylacrylamide and acrylpyrrolidone, polyethylene glycol (PEG)-modified dendrimer, polylactic acid, polylactic acid polyglycolic acid, and polyethylene glycolated polylactic acid, and salts thereof.

Here, the salt of the polymer includes, for example, a metal salt, an ammonium salt, an acid addition salt, an organic amine addition salt, an amino acid addition salt, and the like. Examples of the metal salt include alkali metal salts such as a lithium salt, a sodium salt and a potassium salt; alkaline earth metal salts such as a magnesium salt and a calcium salt; an aluminum salt; a zinc salt, and the like. Examples of the ammonium salt include salts of ammonium, tetramethylammonium, and the like. Examples of the acid addition salt include inorganates such as a hydrochloride, a sulfate, a nitrate, and a phosphate, and organates such as an acetate, a maleate, a fumarate, and a citrate. Examples of the organic amine addition salt include addition salts of morpholine, piperidine, and the like, and examples of the amino acid addition salt include addition salts of glycine, phenylalanine, aspartic acid, glutamic acid, lysine, and the like.

The lead particle in the present invention may contain a lipid conjugate or a fatty acid conjugate of one or more substance(s) selected from, for example, sugars, peptides, nucleic acids and water-soluble polymers; or a surfactant; or the like. The lipid conjugate or the fatty acid conjugate of one or more substance(s) selected from sugars, peptides, nucleic acids and water-soluble polymers; or the surfactant may be used as a constituent component of the lead particle or may be used by adding it to the lead particle.

Preferred examples of the lipid conjugate or the fatty acid conjugate of one or more substance(s) selected from sugars, peptides, nucleic acids and water-soluble polymers; or the surfactant include a glycolipid or a lipid conjugate or a fatty acid conjugate of a water-soluble polymer, and more preferred examples thereof include a lipid conjugate or a fatty acid conjugate of a water-soluble polymer. The lipid conjugate or the fatty acid conjugate of one or more substance(s) selected from sugars, peptides, nucleic acids and water-soluble polymers; or the surfactant is preferably a substance having a dual character that a part of the molecule has a property of binding to another constituent component(s) of the lead particle due to, for example, hydrophobic affinity, electrostatic interaction or the like, and other part has a property of binding to a solvent used in the production of the lead particle due to, for example, hydrophilic affinity, electrostatic interaction or the like.

Examples of the lipid conjugate or the fatty acid conjugate of a sugar, a peptide or a nucleic acid include those comprising a sugar such as sucrose, sorbitol or lactose, a peptide such as a casein-derived peptide, an egg white-derived peptide, a soybean-derived peptide or glutathione, a nucleic acid such as DNA, RNA, plasmid, siRNA or ODN, which are bonded to and any of the lipid illustrated in the above-mentioned definition of the lead particle or a fatty acid such as stearic acid, palmitic acid, myristic acid or lauric acid, and the like.

Examples of the lipid conjugate or the fatty acid conjugate of a sugar include the glyceroglycolipids and the sphingoglycolipids illustrated in the above-mentioned definition of the lead particle and the like.

Examples of the lipid conjugate or fatty acid conjugate of a water-soluble polymer include products formed by the binding of the lipid exemplified above in the definition of the lead particles, or a fatty acid, for example, such as stearic acid, palmitic acid, myristic acid, or lauric acid with a water-soluble polymer such as polyethylene glycol, polyglycerin, polyethyleneimine, polyvinyl alcohol, polyacrylic acid, polyacrylamide, oligosaccharide, dextrin, water-soluble cellulose, dextran, chondroitin sulfate, polyglycerin, chitosan, polyvinylpyrrolidone, polyaspartic acid amide, poly-L-lysine, mannan, pullulan, oligoglycerol, and derivatives thereof (preferably, the water-soluble polymer is a linear water-soluble polymer). More preferred examples include lipid conjugates or fatty acid conjugates such as polyethylene glycol derivatives and polyglycerin derivatives. Even more preferred examples include lipid conjugates or fatty acid conjugates such as polyethylene glycol derivatives.

Examples of the lipid conjugate or the fatty acid conjugate of a polyethylene glycol derivative include a polyethylene glycolated lipid [specifically, polyethylene glycol phosphatidyl ethanolamine (more specifically, 1,2-distearoyl-sn-glycero-3-phosphoethanolamine-N-[methoxy(polyethylene glycol)-2000] (PEG-DSPE) and the like), polyoxyethylene hydrogenated castor oil 60, Cremophor EL and the like], a polyethylene glycol sorbitan fatty acid ester (specifically, polyoxyethylene sorbitan monooleate and the like), a polyethylene glycol fatty acid ester and the like, and more preferred examples include a polyethylene glycolated lipid.

Examples of the lipid conjugate or the fatty acid conjugate of a polyglycerol derivative include a polyglycerolated lipid (specifically, polyglycerol phosphatidyl ethanolamine and the like), a polyglycerol fatty acid ester and the like, and more preferred examples include a polyglycerolated lipid.

Examples of the surfactant include polyoxyethylene sorbitan monooleate (specifically, polysorbate 80 and the like), polyoxyethylene polyoxypropylene glycol (specifically Pluronic F68 and the like), a sorbitan fatty acid ester (specifically, sorbitan monolaurate, sorbitan monooleate and the like), a polyoxyethylene derivative (specifically, polyoxyethylene hydrogenated castor oil 60, polyoxyethylene lauryl alcohol and the like), a glycerin fatty acid ester, a polyethylene glycolalkyl ether and the like. Preferred examples include polyoxyethylene polyoxypropylene glycol, a glycerin fatty acid ester, and a polyethylene glycolalkyl ether and the like.

The lead particle preferably has a positive electric charge. The "positive electric charge" as used herein includes an electric charge, surface polarization and the like which generate electrostatic attraction to an electric charge in the above-mentioned double-stranded nucleic acid molecule, intramolecular polarization and the like. In order for the lead particle to have a positive electric charge, the lead particle preferably contains a cationic substance.

The cationic substance to be contained in the lead particle is a substance exhibiting a cationic nature, however, even if it is an amphoteric substance having both cationic group and anionic group, the relative electronegativity varies depending on the pH, bonding to another substance or the like, therefore, the amphoteric substance can be classified into a cationic substance as the case may be. These cationic substances may be used as a constituent component of the lead particle or may be used by adding it to the lead particle.

Examples of the cationic substance include the cationic substances exemplified above in the definition of the lead particle [specifically, the lipid cationic substance, the cationic polymer and the like], proteins or peptides that are cationic at pH values at or below the isoelectric point and the like. More preferred examples include the lipid cationic substances. Further preferably, the cationic substance is one or more selected from *N*-[1-(2,3-dioleoylpropyl)]-*N*,*N*,*N*-trimethylammonium chloride, *N*-[1-(2,3-dioleoylpropyl)]-*N*,*N*-dimethylamine, *N*-[1-(2,3-dioleyloxypropyl)]-*N*,*N*,*N-*trimethylammonium chloride, *N*-[1-(2,3-ditetradecyloxypropyl)]-*N*,*N*-dimethyl-*N-*hydroxyethyl ammonium bromide, and 3β-[*N-*(*N'*,*N'* dimethylaminoethyl)carbamoyl]cholesterol.
Examples of the lipid cationic substances include cationic lipids (DOTAP, DODAP, DOTMA, DOSPA, DMRIE, DORIE and the like), DC-Chol and the like.
Examples of the cationic polymer include poly-L-lysine, polyethyleneimine, polyfect, chitosan and the like.

The protein or the peptide which shows cationic nature at a pH equal to or less than an isoelectric point is not particularly limited as long as it is a protein or a peptide which shows cationic nature at a pH equal to or less than the isoelectric point of the substance. Examples thereof include albumin, orosomucoid, globulin, fibrinogen, pepsin, ribonuclease T1 and the like.

The lead particle in the present invention can be produced by or in accordance with a known production method or a method similar to that, and a lead particle produced by any production method can be used. For example, liposome B, which is one type of the lead particle, a known liposome preparation method can be applied. As the known liposome preparation method, for example, liposome preparation method by Bangham, et al. [see "Journal of Molecular Biology" (J. Mol. Biol.), Vol. 13, pp. 238-252 (1965)], an ethanol injection method [see "Journal of Cell Biology" (J. Cell Biol.), Vol. 66, pp. 621-634 (1975)], a French press method [see "FEBS Letters" (FEBS Lett.), Vol. 99, pp. 210-214 (1979)], a freeze-thaw method [see "Archives of Biochemistry and Biophysics" (Arch. Biochem. Biophys.), Vol. 212, pp. 186-194 (1981)], a reverse phase evaporation method [see "Proceedings of the National Academy of Science United States of America" (Proc. Natl. Acad. Sci. USA), Vol. 75, pp. 4194-4198 (1978)], a pH gradient method (see, for example, Japanese Patent No. 2,572,554, Japanese Patent No. 2,659,136, etc.) and the like. As a solution for dispersing liposome B in the production of the liposome B, for example, water, an acid, an alkali, any of various buffers, a physiological saline solution, an amino acid infusion or the like can be used. Further, in the production of the liposome B, it is also possible to add an antioxidant such as citric acid, ascorbic acid, cysteine or ethylenediamine tetraacetic acid (EDTA), an isotonic agent such as glycerol, glucose, sodium chloride or the like. Further, the liposome can also be produced by dissolving a lipid or the like in, for example, an organic solvent such as ethanol, distilling off the solvent, adding a physiological saline solution or the like and stirring the mixture by shaking, thereby forming the liposome B.

For example, the cationic substance, polymer, polyoxyethylene derivative and the like can be used for the surface improvement of the lead particle, including liposome B [see ed. D.D. Lasic, F. Martin, Stealth Liposomes, CRC Press Inc., US, 1995, p.93-102]. Examples of the polymer usable for the surface improvement include dextran, pullulan, mannan, amylopectin, hydroxyethyl starch and the like. Examples of the polyoxyethylene derivative include polysorbate 80, Pluronic F68, polyoxyethylene hydrogenated castor oil 60, polyoxyethylenelauryl alcohol, PEG-DSPE and the like. The surface improvement of the lead particle, including liposome B and the like, represents one of the methods of containing the lipid conjugate or fatty acid conjugate of one or more substances selected from sugar, peptide, nucleic acid, and water-soluble polymer, or a surfactant in the lead particle.

An average particle diameter of the liposome B can be freely selected upon demand. It is preferable to adjust the average particle diameter to a diameter of the lead particle shown below. Examples of a method of adjusting the average particle diameter include an extrusion method and a method in which a large multilamellar liposome vesicle (MLV) is mechanically pulverized (specifically using Manton-gaulin, a microfluidizer or the like) (see "Emulsion and Nanosuspensions for the Formulation of Poorly Soluble Drugs", edited by R. H. Muller, S. Benita and B. Bohm, Scientific Publishers, Stuttgart, Germany, pp. 267-294, 1998) and the like.

In addition, the method of producing a complex obtained by combining two or more substances selected from, for example, a lipid assembly, liposome B, a polymer micelle, and the like, which constitute the lead particle, may be, for example, a production method in which, for example, a lipid, a polymer or the like are only mixed in water. At this time, a granulation step, a sterilization step or the like can be further added as needed. It is also possible to perform the formation of the complex in any of various solvents such as acetone or ether.

As for the size of the lead particle in the present invention, an average particle diameter is preferably several nanometers to several tens micrometers, more preferably about 10 nm to 1000 nm, further more preferably about 50 nm to 300 nm.

The lipid bilayer membrane coating the complex particles that contain the lead particles and the double-stranded nucleic acid molecule in the present invention may contain constituent components, for example, such as the lipids exemplified above in the definition of the lead particles, preferably neutral lipids, in addition to the lipid conjugate, fatty acid conjugate, or aliphatic hydrocarbon conjugate of a water-soluble substance. As used herein, the neutral lipid refers to lipids excluding the lipid cationic substance exemplified for the cationic substance for the positively charged lead particle, and anionic lipids exemplified below in conjunction with the adhesion-competitive agent. More preferred examples of the neutral lipid include phospholipid, glycoglycerolipid, glycosphingolipid, and the like. The phospholipid is even more preferable, and EPC is further preferable. These lipids may be used either alone or in a combination of two or more.

The constituent components of the lipid bilayer membrane coating the complex particles are preferably soluble in a polar organic solvent, and are preferably dispersible in a liquid that contains the polar organic solvent in a certain concentration. The concentration of the polar solvent in a liquid that contains the polar solvent in a certain concentration is preferably such that the constituent components of the lipid bilayer membrane are dispersible, and that the complex particles are also dispersible. Examples of the polar organic solvent include alcohols such as methanol, ethanol, n-propanol, 2-propanol, n-butanol, 2-butanol, and tert-butanol, glycols such as glycerol, ethylene glycol, and propylene glycol, polyalkylene glycols such as polyethylene glycol, and the like. Alcohol is preferable, and ethanol is more preferable.
Examples of solvents other than the polar organic solvent contained in the polar organic solvent-containing liquid in the present invention include water, liquid carbon dioxide, liquid hydrocarbon, halogenated carbon, halogenated hydrocarbon, and the like, of which water is preferable. The solvent may include other components, including ions and buffers. One or two or more solvents may be used. When two or more solvents are used, the solvents combined are preferably compatible to each other.

The lipid bilayer membrane of the lipidparticle in the composition of the present invention, and the lipid bilayer membrane coating the complex particles contain, as constituent components, a lipid conjugate, a fatty acid conjugate, or an aliphatic hydrocarbon conjugate of a water-soluble substance, or the surfactant. Preferably, a lipid conjugate, a fatty acid conjugate, or an aliphatic hydrocarbon conjugate of a water-soluble substance is contained as the constituent component. Examples of the lipid conjugate, fatty acid conjugate, or aliphatic hydrocarbon conjugate of a water-soluble substance include a lipid conjugate or a fatty acid conjugate of one or more substances selected from the sugar, peptide, nucleic acid, and water-soluble polymer, and an aliphatic hydrocarbon conjugate of one or more substances selected from the sugar, peptide, nucleic acid, and water-soluble polymer. The lipid conjugate, fatty acid conjugate, or aliphatic hydrocarbon conjugate of a water-soluble substance is more preferably a lipid conjugate or a fatty acid conjugate of the water-soluble polymer, further preferably the polyethylene glycolated phospholipid, most preferably polyethylene glycol phosphatidyl ethanolamine. Examples of the aliphatic hydrocarbon conjugate of a water-soluble substance in the present invention include products formed by the binding of a water-soluble substance with, for example, long-chain aliphatic alcohol, or with an alcoholic residue of polyoxypropylenealkyl or glycerin fatty acid ester.

Examples of the aliphatic hydrocarbon conjugate of a sugar, a peptide or a nucleic acid include an aliphatic hydrocarbon conjugate of a sugar such as sucrose, sorbitol or lactose, a peptide such as a casein-derived peptide, an egg white-derived peptide, a soybean-derived peptide or glutathione, a nucleic acid such as DNA, RNA, plasmid, siRNA or ODN.

Examples of the aliphatic hydrocarbon conjugate of a water-soluble polymer include an aliphatic hydrocarbon conjugate of polyethylene glycol, polyglycerol, polyethyleneimine, polyvinyl alcohol, polyacrylic acid, polyacrylamide, oligosaccharide, dextrin, a water-soluble cellulose, dextran, chondroitin sulfate, polyglycerol, chitosan, polyvinylpyrrolidone, polyaspartate amide, poly-L-lysine, mannan, pullulan, oligoglycerol or the like, or a derivative thereof, and preferred examples thereof include an aliphatic hydrocarbon conjugate of a polyethylene glycol derivative or a polyglycerol derivative, and more preferred examples thereof include an aliphatic hydrocarbon derivative of a polyethylene glycol derivative.

In the case where the lead particle is a fine particle containing liposome B as a constituent component, a substance which contains complex particles containing the liposome B and the above-mentioned double-stranded nucleic acid molecule as constituent components and a lipid bilayer membrane coating the complex particles becomes lipidparticle A, which is classified into liposome in a narrow sense based on its structure. Even if the lead particle is different from a fine particle containing the liposome B as a constituent component, the lead particle is coated with a lipid bilayer membrane, therefore, the resulting substance is classified into liposome in a wide sense. In the present invention, it is more preferred that the lead particle is also a fine particle containing the liposome B.

The complex particles containing the lead particle and the double-stranded nucleic acid molecule as constituent components in the present invention can be produced by adhering or encapsulating the double-stranded nucleic acid molecule to or into the lead particle after or concurrently with the production of the lead particle. Further, lipidparticle A can be produced by coating the complex particles with the lipid bilayer membrane after or concurrently with the production of the complex particles. The lipidparticle A can be produced by or in accordance with a known production method described in, for example, Published Japanese translation of a PCT international application No. 2002-508765, Published Japanese translation of a PCT international application No. 2002-501511, "Biochimica et Biophysica Acta", Vol. 1510, pp. 152-166 (2001), and International Publication No. WO 02/28367, or can be produced by a production method including a step of dispersing the complex particles and coating layer components in a liquid which contains a polar organic solvent in which the coating layer components are soluble at a concentration at which the complex particles are not dissolved and the coating layer components are present in a dispersed state after the complex particles are produced by adhering or encapsulating the double-stranded nucleic acid molecule to or into the lead particle, and a step of coating the complex particles with the coating layer components. It is preferred that the complex particles containing the lead particle and the double-stranded nucleic acid molecule as constituent components in the present invention is produced after or concurrently with the production of the lead particle in water, by mixing double-stranded nucleic acid molecule which is dispersed or dissolved in water with the lead particle, then by adhering or encapsulating the double-stranded nucleic acid molecule to or into the lead particle, or produced after the production of the lead particle in optional iquid, and the lead partiles were dispersed in water, by mixing the double-stranded nucleic acid molecule which is dispersed or dissolved in the water to the lead particle, then by adhering the double-stranded nucleic acid molecule to the lead particle, and it is more preferred that the complex particles is produced after the production of the lead particle in water, by mixing the double-stranded nucleic acid molecule which is dispersed or dissolved in the water to the lead particle, then by adhering the double-stranded nucleic acid molecule to the lead particle.

As a preferred production method of the lipidparticle A in the composition of the present invention, the following production method including a step of producing complex particles containing as constituent components a lead particle and the double-stranded nucleic acid molecule (step 1) and a step of coating the complex particles with a lipid bilayer membrane (step 2 or step 3) can be exemplified.

### Step 1) Step of producing complex particles containing as constituent components a lead particle and the double-stranded nucleic acid molecule

Lead particles are dispersed in a solvent such as water, the double-stranded nucleic acid molecule dispersed or dissolved and mixed so as to be contained in the liquid in which the lead particles are dispersed, and the double-stranded nucleic acid molecule is adhered to the lead particles. In the step 1, in order to suppress the aggregation of the lead particles, the lead particles are preferably lead particles containing an aggregation-suppressing substance. As the aggregation-suppressing substance, the above-mentioned lipid conjugate or fatty acid conjugate of one or more substance(s) selected from sugars, peptides, nucleic acids and water-soluble polymers or the surfactant. In the case where the lead particles have a positive electric charge, the double-stranded nucleic acid molecule and an adhesion-competitive agent are allowed to coexist in the liquid in which the lead particles are dispersed, and the adhesion-competitive agent may be adhered to the lead particles as well as the double-stranded nucleic acid molecule. Also in the case where the lead particles are lead particles containing the aggregation-suppressing substance, in order to further suppress the aggregation of the lead particles, the adhesion-competitive agent may be used. In the combination of the lead particles and the double-stranded nucleic acid molecule, it is preferred that a combination in which the complex particles are dispersible in the liquid containing a polar organic solvent is selected, and it is more preferred that the solubility of the complex particles in the polar organic solvent is lower than that of the constituent components of the lipid bilayer membrane to be used in the step 2 or 3. It is further more preferred that a combination in which the polar organic solvent can be contained in a liquid at such a concentration that the constituent components of the lipid bilayer membrane are dispersible and the complex particles are dispersible is selected.

Examples of the adhesion-competitive agent include an anionic substance and the like, and the anionic substance includes a substance electrostatically adhered to the lead particles due to the electrostatic attraction by an electric charge, intramolecular polarization or the like in the molecule. The anionic substance as the adhesion-competitive agent is a substance exhibiting an anionic nature, however, even if it is an amphoteric substance having both cationic group and anionic group, the relative electronegativity varies depending on the pH, binding to another substance(s) or the like, therefore, the amphoteric substance can be classified into an anionic substance on a moment-to-moment basis.

Examples of the anionic substance include anionic lipid, an anionic surfactant, an anionic polymer, a protein or a peptide or a nucleic acid which shows an anionic nature at a pH equal to or greater than an isoelectric point, and the like. Preferred examples thereof include dextran sulfate, sodium dextran sulfate, chondroitin sulfate, sodium chondroitin sulfate, hyaluronic acid, chondroitin, dermatan sulfate, heparan sulfate, heparin, keratan sulfate, dextran fluorescein anionic, and the like. The anionic substances may be used alone, or two or more anionic substances may be used in combination.

Examples of the anionic lipid include phosphatidyl serine, phosphatidylglycerol, phosphatidylinositol, phosphatidic acid, and the like.
Examples of the anionic surfactant include acylsarcosine, sodium alkyl sulfonate, alkylbenzene sulfonate, fatty acid sodium of 7 to 22 carbon atoms, and the like. Specific examples include sodium dodecylsulfonate, sodium lauryl sulfate, sodium cholate, sodium deoxycholate, sodium taurodeoxycholate, and the like.

Examples of the anionic polymer include polyaspartic acid, a copolymer of styrene with maleic acid, a copolymer of isopropylacrylamide with acrylpyrrolidone, PEG-modified dendrimer, polylactic acid, polylactic acid polyglycolic acid, polyethylene glycolated polylactic acid, dextran sulfate, sodium dextran sulfate, chondroitin sulfate, sodium chondroitin sulfate, hyaluronic acid, chondroitin, dermatan sulfate, heparan sulfate, heparin, keratan sulfate, dextran fluorescein anionic and the like.
The protein or the peptide which shows an anionic nature at a pH equal to or greater than an isoelectric point is not particularly limited as long as it is a protein or a peptide which shows an anionic nature at a pH equal to or greater than the isoelectric point of the substance. Examples thereof include albumin, orosomucoid, globulin, fibrinogen, histone, protamine, ribonuclease, lysozyme and the like.

Examples of the nucleic acid as an anionic substance include DNA, RNA, plasmid, siRNA, ODN and the like. It may have any length and any sequence as long as it does not exhibit a physiological activity.

The adhesion-competitive agent is preferably electrostatically adhered to the lead particles, and is preferably a substance with a size which does not allow the crosslinking formation to aggregate the lead particles even if the substance is adhered to the lead particles, or a substance having in its molecule, a moiety which is adhered to the lead particles and a moiety which repels the adhesion and suppresses the aggregation of the lead particles.

More specifically, the step 1 can be carried out, for example, in a production method including a step of producing a liquid in which lead particles containing an aggregation-suppressing substance are dispersed, and a step of dispersing or dissolving the double-stranded nucleic acid molecule so as to be contained in the liquid in which the lead particles are dispersed (for example, a step of adding the double-stranded nucleic acid molecule to the liquid in which the lead particles are dispersed and dispersing or dissolving the double-stranded nucleic acid molecule therein, a step of adding a liquid in which the double-stranded nucleic acid molecule is dispersed or dissolved to the liquid in which the lead particles are dispersed or the like). Here, specific examples of the complex particles obtained by the step of dispersing or dissolving the double-stranded nucleic acid molecule so as to be contained in the liquid in which the lead particles are dispersed, contain complex particles formed by adhering the double-stranded nucleic acid molecule to fine particles containing as a constituent component, liposome B containing the cationic substance, complex particles formed by adhering the double-stranded nucleic acid molecule to fine particles containing as a constituent component, a lipid assembly containing the cationic substance, and complex particles formed by adhering the double-stranded nucleic acid molecule to fine particles containing as a constituent component, a polymer containing a cationic polymer such as poly-L-lysine. The step of dispersing or dissolving the double-stranded nucleic acid molecule so as to be contained in the liquid in which the lead particles are dispersed is preferably a step of further incorporating the adhesion-competitive agent in the liquid in which the double-stranded nucleic acid molecule is dispersed or dissolved and adding the resulting liquid to the liquid in which the lead particles are dispersed. In this case, the complex particles are produced by adhering both of the double-stranded nucleic acid molecule and the adhesion-competitive agent to the lead particles, and the production can be carried out by further suppressing aggregation of the lead particles during the production of the complex particles and aggregation of the complex particles after the production.

The ratio of the lead particles to the liquid in which the lead particles are dispersed is not particularly limited as long as the double-stranded nucleic acid molecule can be adhered to the lead particles, however, it is preferably about 1 µg/mL to 1 g/mL, more preferably about 0.1 to 500 mg/mL.

### Step 2) Step of coating complex particles with lipid bilayer membrane (part 1)

Lipidparticle A can be produced by, for example, a production method including a step of preparing a liquid (liquid A) containing a polar organic solvent in which the complex particles obtained from the step 1 are dispersed and the constituent components of the lipid bilayer membrane are dissolved, and a step of coating the complex particles with the lipid bilayer membrane by reducing the ratio of the polar organic solvent in the liquid A. In this case, the lipidparticle A is obtained in the form of a dispersion (liquid B). The solvent in the liquid A is a solvent which contains a polar organic solvent at such a concentration that the constituent components of the lipid bilayer membrane are soluble and the complex particles are dispersible. In the liquid B in which the ratio of the polar organic solvent to the liquid A is reduced, the constituent components of the lipid bilayer membrane are dispersible and the complex particles are also dispersible. In the case where the solvent in the liquid A is a liquid mixture of a polar organic solvent and a solvent different from a polar organic solvent, for example, by adding a solvent containing a solvent different from a polar organic solvent mixable with the polar organic solvent (liquid C), and/or selectively removing the polar organic solvent by distillation by evaporation, semipermeable membrane separation, fractional distillation or the like, the ratio of the polar organic solvent can be reduced. Here, the liquid C is preferably a solvent containing a solvent different from a polar organic solvent, and may also contain a polar organic solvent as long as the ratio of the polar organic solvent in liquid C is lower than that of the polar organic solvent contained in the liquid A.

Examples of the solvent different from a polar organic solvent in the step 2 include water, liquid carbon dioxide, a liquid hydrocarbon, a halogenated carbon, a halogenated hydrocarbon and the like, and preferred examples thereof include water. The liquid A and the liquid C may contain an ion, a buffer component or the like. These may be used alone, or two or more may be used in combination.

The combination of a polar organic solvent with a solvent different from a polar organic solvent is preferably a combination of solvents that are mixable with each other and can be selected by considering the solubility of the complex particles and the constituent components of the lipid bilayer membrane in the solvents in the liquid A and the liquid B, and the liquid C. The complex particles preferably have a low solubility in any of the solvents in the liquid A and the liquid B, and the liquid C, and also preferably have a low solubility in any of a polar organic solvent and a solvent different from a polar organic solvent. The constituent components of the lipid bilayer membrane preferably have a low solubility in the solvent in the liquid B, and the liquid C, and preferably have a high solubility in the solvent in the liquid A, and preferably have a high solubility in a polar organic solvent and preferably have a low solubility in a solvent different from a polar organic solvent. Here, "the complex particles having a low solubility" means that the elution of each component contained in the complex particles such as the lead particles, the double-stranded nucleic acid molecule and adhesion-competitive agent in the solvent is low, and even if the respective solubility of the components are high, it is sufficient that the elution of each component becomes low due to the binding or the like between the respective components. For example, even in the case where the solubility of any of the components contained in the lead particles in the solvent of the liquid A is high, if the lead particles have a positive electric charge, and an electrostatic bond is formed due to an electric charge, intramolecular polarization or the like in the double-stranded nucleic acid molecule, and the solubility of the component(s) in the solvent of the liquid A becomes low, the elution of the components in the complex particles is suppressed, whereby the solubility of the complex particles in the solvent of the liquid A can be lowered. That is, if the lead particles have a positive electric charge, the elution of the components of the complex particles is suppressed in the production of the lipidparticle A, and an effect of improving the productivity and yield is imparted.

The concentration of the polar organic solvent in the liquid A is not particularly limited as long as it is a concentration at which the constituent components of the lipid bilayer membrane are soluble and the complex particles are dispersible, and varies depending on the solvent or the complex particles to be used, the type of constituent components of the lipid bilayer membrane or the like. However, it is preferably about 30 v/v% or more, more preferably 60 to 90 v/v%. The concentration of the polar organic solvent in the liquid B is not particularly limited as long as the liquid B contains the polar organic solvent at a concentration lower than the liquid A and it is a concentration at which the constituent components of the lipid bilayer membrane are dispersible and the complex particles are also dispersible, however, it is preferably about 50 v/v% or less.

The step of preparing the liquid A may be a step of preparing the liquid A by mixing the polar organic solvent, the complex particles and the constituent components of the lipid bilayer membrane, further the solvent different from the polar organic solvent, if necessary. The polar organic solvent, the complex particles, the constituent components of the lipid bilayer membrane and the solvent different from the polar organic solvent can be added in any order as long as the complex particles are not dissolved. Preferably, a step of preparing a liquid (liquid D) containing a polar organic solvent in which the complex particles are dispersed, preparing a liquid (liquid E) in which the constituent components of the lipid bilayer membrane are dissolved in a solvent containing a polar organic solvent that is the same as or different from the polar organic solvent in the liquid D, and mixing the liquid D and the liquid E can be exemplified. When the liquid A is prepared by mixing the liquid D and the liquid E, it is preferred to mix them gradually.

### Step 3) Step of coating complex particles with lipid bilayer membrane (part 2)

Lipidparticle A can be produced by a production method including a step of dispersing the complex particles obtained from the step 1 and the constituent components of the lipid bilayer membrane in a liquid (liquid F) which contains a polar organic solvent in which the constituent components of the lipid bilayer membrane are soluble at a concentration at which the constituent components of the lipid bilayer membrane are present in a dispersed state. In this case, the lipidparticle A can be obtained in a dispersion liquid state. Although the constituent components of the lipid bilayer membrane are soluble in a polar organic solvent contained in liquid F, liquid F contains the polar organic solvent, wherein the polar organic solvent can be contained in liquid F at such a concentration that the constituent components of the lipid bilayer membrane are dispersible and the complex particles are dispersible.

As a method of preparing the liquid F, any embodiment can be employed. For example, the liquid F may be prepared by preparing a dispersion of complex particles and a solution or a dispersion of the constituent components of the lipid bilayer membrane and mixing both liquids, or the liquid F may be prepared by preparing either one of the dispersions of the complex particles and the constituent components of the lipid bilayer membrane, and adding and dispersing the other remaining complex particles or constituent components of the lipid bilayer membrane in the form of a solid in the resulting dispersion. In the case where a dispersion of the complex particles and a solution or a dispersion of the constituent components of the lipid bilayer membrane are mixed, a dispersion medium of the complex particles may contain a polar organic solvent in advance, and a solvent or a dispersion medium of the constituent components of the lipid bilayer membrane may be a liquid containing a polar organic solvent or a liquid composed only of a polar organic solvent. On the other hand, in the case where the dispersions of either one of the complex particles and the constituent components of the lipid bilayer membrane is prepared, and the other remaining complex particles or constituent components of the lipid bilayer membrane in the form of a solid are added to the resulting dispersion, the resulting dispersion is preferably a liquid containing a polar organic solvent. Incidentally, in the case where the complex particles are not dissolved and the constituent components of the lipid bilayer membrane are dispersed after the liquid F is prepared, a polar organic solvent may be added in a concentration range of the polar organic solvent in which the complex particles are not dissolved and the constituent components of the lipid bilayer membrane are dispersed, or the organic solvent may be removed or the concentration thereof may be reduced. On the other hand, in the case where the complex particles are not dissolved and the constituent components of the lipid bilayer membrane are dissolved after the liquid F is prepared, the polar organic solvent may be removed or the concentration thereof may be reduced in a concentration range of the polar organic solvent in which the complex particles are not dissolved and the constituent components of the lipid bilayer membrane are dispersed. Alternatively, the complex particles and the constituent components of the lipid bilayer membrane are mixed in a solvent different from a polar organic solvent in advance, and a polar organic solvent may be added thereto in a concentration range of the polar organic solvent in which the complex particles are not dissolved and the constituent components of the lipid bilayer membrane are dispersed. In this case, the complex particles and the constituent components of the lipid bilayer membrane are separately dispersed in solvents different from a polar organic solvent, and both dispersions are mixed, and then, a polar organic solvent may be added thereto, or either one of the complex particles and the constituent components of the lipid bilayer membrane are dispersed in a solvent different from a polar organic solvent, and the other remaining complex particles or constituent components of the lipid bilayer membrane in the form of a solid are added to the resulting dispersion, and then a polar organic solvent may be added thereto.
It is preferred to comprise a step of letting a liquid, in which the complex particles and the constituent components of the lipid bilayer membrane are dispersed and a polar organic solvent is contained, stand or mixing the liquid for a time sufficient to coat the complex particles with the lipid bilayer membrane. The time for letting the liquid stand or mixing the liquid after the complex particles and the constituent components of the lipid bilayer membrane are dispersed in the liquid containing the polar organic solvent is not limited as long as it is not completed immediately after the complex particles and the constituent components of the lipid bilayer membrane are dispersed in the liquid containing the polar organic solvent, however, it can arbitrarily be set depending on the constituent components of the lipid bilayer membrane or the type of the liquid containing the polar organic solvent, and it is preferably to set to a time which keeps the yield of the obtained lipidparticle A constant, for example, about 3 seconds to 30 minutes. Note that dispersing the complex particles and the constituent components of the lipid bilayer membrane in a polar organic solvent-containing liquid starts the coating of the complex particles with the lipid bilayer membrane, and the coating of the complex particles with the lipid bilayer membrane may be quickly completed. For example, in preparing the liquid F by mixing the complex particle dispersion and a dissolving solution of the constituent components of the lipid bilayer membrane after preparing the dissolving solution, there are cases where the coating of the complex particles with the lipid bilayer membrane completes substantially as soon as the constituent components of the lipid bilayer membrane are dispersed in a certain polar organic solvent-containing liquid, when the constituent components of the lipid bilayer membrane have a low solubility for liquid F.

Examples of the solvent different from a polar organic solvent in the liquid F contain those illustrated in the solvent different from a polar organic solvent in the step 2, and preferred examples thereof contain water.

The concentration of the polar organic solvent in the liquid F is not particularly limited as long as only the requirement that both of the complex particles and the constituent components of the lipid bilayer membrane are dispersed is met, and varies depending on the solvent or the complex particles to be used, the type of the constituent components of the lipid bilayer membrane or the like. However, it is preferably about 1 to 80 vol%, more preferably about 10 to 60 vol%, more preferably about 20 to 50 vol%, and the most preferably about 30 to 40 vol%.

In the present invention, the description of "the constituent components of the lipid bilayer membrane being soluble in a certain polar organic solvent" include a case in which the constituent components of the lipid bilayer membrane have a property of being dissolved in a certain polar organic solvent, a case in which the constituent components of the lipid bilayer membrane have a property of being dissolved in a certain polar organic solvent with the help of a solubilizer or the like, a case in which the constituent components of the lipid bilayer membrane have a property capable of being emulsified or formed into an emulsion by forming aggregates, micelles or the like in a certain polar organic solvent and the like. The description of "the constituent components of the lipid bilayer membrane being dispersible" includes a state in which the whole of the constituent components of the lipid bilayer membrane form aggregates, micelles or the like and are emulsified or formed into an emulsion, a state in which a part of the constituent components of the lipid bilayer membrane form aggregates, micelles or the like and are emulsified or formed into an emulsion, and the rest of the components are dissolved, a state in which a part of the constituent components of the lipid bilayer membrane form aggregates, micelles or the like and are emulsified or formed into an emulsion, and the rest of the components are precipitated and the like. Incidentally, the description of "the constituent components of the lipid bilayer membrane being dissolved" does not include a state in which the whole of the constituent components of the lipid bilayer membrane form aggregates, micelles or the like and are emulsified or formed into an emulsion.

In the present invention, the description of "the complex particles being dispersed" means a state in which the complex particles are suspended or emulsified or formed into an emulsion, and includes a state in which a part of the complex particles are suspended or emulsified or formed into an emulsion, and the rest of the particles are dissolved, a state in which a part of the complex particles are emulsified or formed into an emulsion, and the rest of the particles are precipitated and the like. The description of "the complex particles being not dissolved" is synonymous with the above-mentioned definition of "the complex particles being dispersed".

The concentration of the complex particles in the aqueous solution containing a polar organic solvent to be used in the method of producing lipidparticle A according to the present invention is not particularly limited, as long as it allows the complex particles to be coated with the lipid bilayer membrane, however, it is preferably about 1 µg/mL to 1 g/mL, more preferably about 0.1 to 500 mg/mL. The concentration of the constituent components of the lipid bilayer membrane to be used is not particularly limited as long as it allows the complex particles to be coated, however, it is preferably about 1 µg/mL to 1 g/mL, more preferably about 0.1 to 400 mg/mL.

As for the size of the lipidparticle A of the present invention, an average particle diameter is more preferably about 30 nm to 300 nm, even more preferably about 50 nm to 200 nm. Specifically, for example, an injectable size is preferred.

Further, the lipidparticle A obtained above can be modified with a substance such as a protein including an antibody and the like, a saccharide, a glycolipid, an amino acid, a nucleic acid, or any of various low-molecular compounds and polymers, and such coated complex particles obtained by modification is included in the lipidparticle A. For example, in order to apply to targeting, it is possible that the lipidparticle A obtained above is further subjected to a surface modification of the lipid bilayer membrane using a protein such as an antibody, a peptide, a fatty acid or the like [see Stealth Lipidparticles, edited by D. D. Lasic and F. Martin, CRC Press Inc., USA, pp. 93-102, (1995)]. Surface improvement can also be optionally carried out to the lipidparticle A by using, for example, a lipid conjugate, a fatty acid conjugate, or an aliphatic hydrocarbon conjugate of a water-soluble substance. The lipid conjugate, the fatty acid conjugate, and the aliphatic hydrocarbon conjugate of a water-soluble substance to be used in the surface modification have the same definitions as the lipid conjugate, the fatty acid conjugate, and the aliphatic hydrocarbon derivative of a water-soluble substance as the constituent components of the lipid bilayer membrane. The water-soluble substance can be contained as a constituent component in the lipid bilayer membrane of the lipidparticle by the surface improvement of the lipidparticle.

By administering the composition of the present invention to a mammal including humans, the double-stranded nucleic acid molecule can be delivered to an expression site of a target gene, and, for example, an RNA or the like capable of suppressing the expression of the gene can be introduced into a mammalian cell in vivo, making it possible to suppress expression of the gene or the like. By the intravenous administration of the composition of the present invention to mammals including humans, the composition is delivered to, for example, an organ or a site involving cancer or inflammation, and the nucleic acid in the composition of the present invention can be introduced into the cells of the organ or site. The organ or site involving cancer or inflammation is not particularly limited. Examples include stomach, large intestine, liver, lungs, spleen, pancreas, kidneys, bladder, skin, blood vessel, eye ball, and the like. Further, by the intravenous administration of the composition of the present invention to mammals including humans, the composition can be delivered to, for example, blood vessel, liver, lungs, spleen, and/or kidneys, and the nucleic acid in the composition of the present invention can be introduced into the cells of the organ or site. The liver, lung, spleen, and/or kidney cells may be any of normal cells, cells associated with cancer or inflammation, and cells associated with other diseases.
Specifically, the present invention provides a method for suppressing the expression of the target gene, by administering the composition of the present invention to mammals. Preferably, the administration target is human.

For example, when the target gene of the composition of the present invention is a gene associated with tumor or inflammation, the composition of the present invention can be used as a therapeutic or preventive agent for cancer or inflammatory disease, preferably a therapeutic or preventive agent for solid cancer, or inflammation in blood vessels or in the vicinity of blood vessels. Specifically, for example, when the target gene of the composition of the present invention is a gene associated with angiogenesis or the like, the growth of the vascular smooth muscle, angiogenesis, or the like can be suppressed, and the composition of the present invention can be used, for example, as a therapeutic or preventive agent for cancer or inflammatory disease involving growth of the vascular smooth muscle or angiogenesis.
In other words, the present invention also provides a method for treating cancer or inflammatory disease, by which the composition of the present invention described above is administered to a mammal. Preferably the subject of administration is human, preferably human individuals affected by cancer or inflammatory disease.
Further, the composition of the present invention can also be used as a tool for acquiring P0C (proof of concept) in an in vivo screening system concerning a therapeutic or preventive agent for cancer or inflammatory disease.

The composition of the present invention can be used as a preparation intended for stabilization of the double-stranded nucleic acid molecule in a living body component such as a blood component (for example, blood, gastrointestinal tract or the like), reduction of side effects, increase in drug accumulation in tissues or organs containing the expression site of the target gene, and the like.

In the case where the composition of the present invention is used as a therapeutic or preventive agent for diseases such as cancer and inflammation, it is preferred that an administration route that is most effective for treatment be used. Examples of the administration route include parenteral administration routes such as intraoral administration, tracheobronchial administration, intrarectal administration, subcutaneous administration, intramuscular administration, and intravenous administration, and oral administration routes. Preferred examples thereof include intravenous administration and intramuscular administration, and more preferred examples thereof include intravenous administration.
The doses may vary depending upon conditions and age of the subject, administration route, and the like. For example, a dose of about 0.1 µg to 1000 mg in terms of RNA is administered daily.

As a preparation suitable for intravenous administration or intramuscular administration, for example, an injection can be exemplified, and it is also possible to use the dispersion of the lipidparticle A prepared by the above-mentioned method as it is in the form of, for example, an injection or the like. However, it can also be used after removing the solvent from the dispersion by, for example, filtration, centrifugation or the like, or after lyophilizing the dispersion or the dispersion supplemented with an excipient such as mannitol, lactose, trehalose, maltose or glycine.
In the case of an injection, it is preferred that an injection is prepared by mixing, for example, water, an acid, an alkali, any of various buffers, a physiological saline solution, an amino acid infusion or the like with the dispersion of the lipidparticle A or the lipidparticle A obtained by removing the solvent or lyophilization. It is possible to prepare an injection by adding an antioxidant such as citric acid, ascorbic acid, cysteine or EDTA, an isotonic agent such as glycerol, glucose or sodium chloride or the like. It can also be cryopreserved by adding a cryopreservation agent such as glycerol.

Among the compositions of the present invention, the therapeutic agent for cancer or inflammatory disease of the present invention may be a composition that comprises:
a double-stranded nucleic acid molecule that contains a sense strand and an antisense strand,
wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5' -end to 3'-end direction of the antisense strand, and the sugars in the sense strand are ribose, deoxyribose, or ribose whose hydroxyl group at the 2' position is substituted by a modifying group,
   (i) 0 to 30% of the sugars binding to the bases 1 to 8 in the 5'-end to 3'-end direction of sequence a being deoxyribose, or ribose whose hydroxyl group at the 2' position is substituted by a modifying group,
   (ii) 0 to 20% of the sugars binding to the bases 9 to 16 in the 5' -end to 3'-end direction of sequence a being deoxyribose, or a ribose whose hydroxyl group at the 2' position is substituted by a modifying group,
   (iii) 30 to 100% of the sugars binding to the bases from base 17 to the 3'-end base in the 5'-end to 3'-end direction of the antisense strand being deoxyribose, or ribose whose hydroxyl group at the 2' position is substituted by a modifying group,
   (iv) 10 to 70% of the sugars binding to the bases 1 to 17 in the 5'-end to 3'-end direction of sequence b being deoxyribose, or ribose whose hydroxyl group at the 2' position is substituted by a modifying group,
   (v) 30 to 100% of the sugars binding to the bases other than sequence b of the sense strand being deoxyribose, or ribose whose hydroxyl group at the 2' position is substituted by a modifying group; and
lipidparticle A which may be either (1) a lipidparticle including a complex particle that contains a lead particle and the double-stranded nucleic acid molecule as constituent components, and a lipid bilayer membrane coating the complex particle, the constituent components of the lipid bilayer membrane being soluble in a certain polar organic solvent, the constituent components of the lipid bilayer membrane and the complex particle being dispersible in a liquid that contain the polar organic solvent in a certain concentration, and the lipid bilayer membrane including as constituent components a lipid conjugate, a fatty acid conjugate, or an aliphatic hydrocarbon conjugate of a water-soluble substance, or (2) a lipidparticle including a complex particle that contains a cationic substance-containing lead particle and the double-stranded nucleic acid molecule as constituent components, and a lipid bilayer membrane coating the complex particle, the lipid bilayer membrane containing, as constituent components, a neutral lipid, and a lipid conjugate, a fatty acid conjugate, or an aliphatic hydrocarbon conjugate of a water-soluble substance. In the therapeutic agent for cancer or inflammatory disease of the present invention, the cancer is preferably a solid cancer, and the inflammatory disease is preferably inflammation in blood vessels or in the vicinity of blood vessels.

Further, the present invention provides use of the composition of the present invention for the manufacture of a therapeutic agent for cancer or inflammatory disease, preferably a therapeutic agent for solid cancer, or inflammation in blood vessels or in the vicinity of blood vessels. The composition may be a composition that comprises:
a double-stranded nucleic acid molecule that contains a sense strand and an antisense strand,
wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5'-end to 3' -end direction of the antisense strand, and the sugars in the sense strand are ribose, deoxyribose, or ribose whose hydroxyl group at the 2' position is substituted by a modifying group,
   (i) 0 to 30% of the sugars binding to the bases 1 to 8 in the 5'-end to 3'-end direction of sequence a being deoxyribose, or ribose whose hydroxyl group at the 2' position is substituted by a modifying group,
   (ii) 0 to 20% of the sugars binding to the bases 9 to 16 in the 5'-end to 3'-end direction of sequence a being deoxyribose, or a ribose whose hydroxyl group at the 2' position is substituted by a modifying group,
   (iii) 30 to 100% of the sugars binding to the bases from base 17 to the 3'-end base in the 5'-end to 3'-end direction of the antisense strand being deoxyribose, or ribose whose hydroxyl group at the 2' position is substituted by a modifying group,
   (iv) 10 to 70% of the sugars binding to the bases 1 to 17 in the 5'-end to 3'-end direction of sequence b being deoxyribose, or ribose whose hydroxyl group at the 2' position is substituted by a modifying group,
   (v) 30 to 100% of the sugars binding to the bases other than sequence b of the sense strand being deoxyribose, or ribose whose hydroxyl group at the 2' position is substituted by a modifying group; and
lipidparticle A which may be either (1) a lipidparticle including a complex particle that contains a lead particle and the double-stranded nucleic acid molecule as constituent components, and a lipid bilayer membrane coating the complex particle, the constituent components of the lipid bilayer membrane being soluble in a certain polar organic solvent, the constituent components of the lipid bilayer membrane and the complex particle being dispersible in a liquid that contain the polar organic solvent in a certain concentration, and the lipid bilayer membrane including as constituent components a lipid conjugate, a fatty acid conjugate, or an aliphatic hydrocarbon conjugate of a water-soluble substance, or (2) a lipidparticle including a complex particle that contains a cationic substance-containing lead particle and the double-stranded nucleic acid molecule as constituent components, and a lipid bilayer membrane coating the complex particle, the lipid bilayer membrane containing, as constituent components, a neutral lipid, and a lipid conjugate, a fatty acid conjugate, or an aliphatic hydrocarbon conjugate of a water-soluble substance.

The present invention will be specifically described below with reference to Examples and Test examples. However, the present invention is not limited to these Examples and Test examples.
Examples 1 to 4 and Comparative Examples 1 to 9 used double-stranded nucleic acid molecules that contained the sequence of 19 contiguous bases 5'-GUG AAG UCA ACA UGC CUG C-3' of BCL2 gene mRNA with the sense strand and the antisense strand shown in Table 1 (the sugars binding to the base prefaced by d are deoxyribose, and the sugars binding to the base prefaced by m are 2'-0-methyl-substituted ribose). The sense strand and the antisense strand were obtained from Hokkaido System Science Co., Ltd., and were annealed to prepare the double-stranded nucleic acid molecules.

### Example 1

DOTAP (manufactured by Avanti Polar Lipids Inc.), PEG-DSPE (manufactured by NOF Corporation) and distilled water (manufactured by Otsuka Pharmaceutical Co., Ltd.) were mixed such that the amounts of DOTAP/PEG-DSPE/distilled water was 40 mg/16 mg/1 mL, and the mixture was stirred by shaking with a vortex mixer. The obtained suspension was passed, at 70°C, through a 0.4-µm polycarbonate membrane filter (manufactured by Costar) 10 times and through a 0.2-µm polycarbonate membrane filter (manufactured by Whatman) 3 times and then through a 0.1 µm polycarbonate membrane filter (manufactured by Corning) 10 times and a 0.05 µm polycarbonate membrane filter (manufactured by Whatman) 20 times. The obtained lead particles had an average particle diameter of 70.71 nm as measured by Dynamic light scattering (DLS).
Meanwhile, EPC (NOF Corporation)/PEG-DSPE (NOF Corporation)/ethanol (Wako Pure Chemical Industries, Ltd.) /water (15 mg/3. 125 mg/0.625 mL/0.375 mL) were mixed, thereby a solution containing the constituent components of the lipid bilayer membrane was prepared.
The obtained lead particle dispersion (0.0125 mL) was mixed with an aqueous solution (0.00417 mL) obtained by mixing the BCL2siRNA-Exp. 1 in water presented in Table 1 in a proportion of 24 mg/1 mL, so as to prepare the complex particles. The obtained dispersion of complex particles was added to the solution of lipid bilayer membrane constituent components (0.06667 mL), and 0.02083 mL of distilled water was added. Then, after adding a solution (0.00667 mL) of EPC/PEG-DSPE dissolved in 40 vol% ethanol in 62.5 mg/62.5 mg/mL, distilled water (0.7758 mL) was gradually added to adjust the ethanol concentration to 5% or less and prepare the lipidparticle. The resulting lipidparticle suspension was isotonized with brine. A preparation was obtained by adjusting the BCL2siRNA-Exp. 1 concentration to 0.1 mg/mL by making the final liquid volume 1 mL with physiological saline (Otsuka Pharmaceutical Co., Ltd.).
The lipidparticle in the preparation had an average particle diameter of 82.59 nm as measured by DLS.

### Example 2

A preparation was obtained in the same manner as in Example 1, except that BCL2siRNA-Exp. 2 was used instead of BCL2siRNA-Exp. 1. The lipidparticle in the preparation had an average particle diameter of 83.94 nm as measured by DLS.

### Comparative Examples 1 to 9

Preparations were obtained in the same manner as in Example 1, except that BCL2siRNA-Com. 1 to 9 were used instead of BCL2siRNA-Exp. 1.
The average particle diameter of the lipidparticle in each preparation was measured by DLS. Table 1 presents the average particle diameter of the lipidparticle in each preparation.

### Example 3

A preparation was obtained in the same manner as in Example 1, except that BCL2siRNA-Exp. 3 was used instead of BCL2siRNA-Exp. 1. The lipidparticle in the preparation had an average particle diameter of 82.42 nm as measured by DLS.

### Example 4

A preparation was obtained in the same manner as in Example 1, except that BCL2siRNA-Exp. 4 was used instead of BCL2siRNA-Exp. 1. The lipidparticle in the preparation had an average particle diameter of 83.47 nm as measured by DLS.

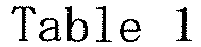

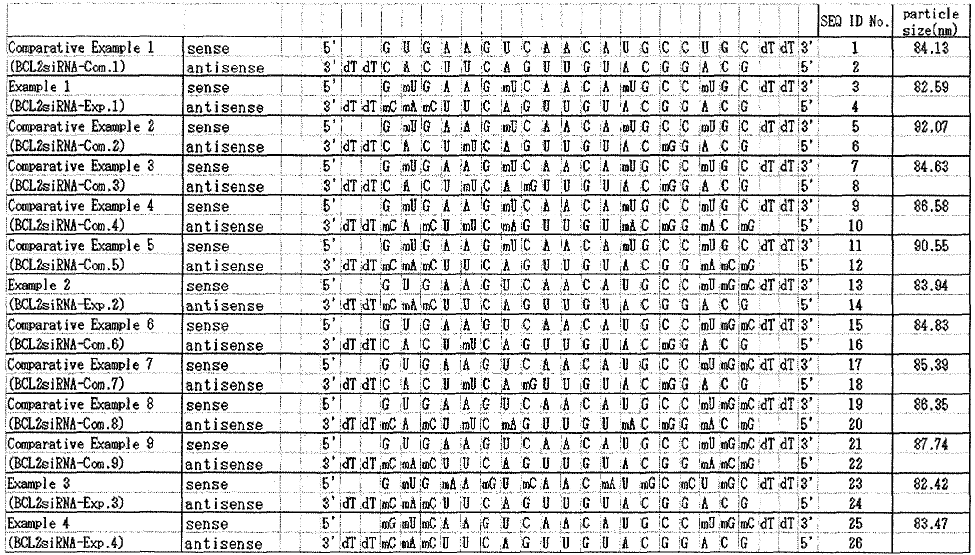

### Test Example 1

The RNAi activities of BCL2siRNA-Exp. 1 to 4 and BCL2siRNA-Com. 1 to 9 were evaluated by measuring the expression suppressing effect on Bcl2mRNA, as follows.
Human prostate cancer cells PC-3 were seeded in a 6 cm-diameter culture dish (2 × 10⁵ cells/dish), and cultured overnight under 37°C, 5% CO₂ conditions in F-12 Kaighn's medium (GIBCO, 21127) that contained 10% fetal bovine serum. On the next day, the medium was suctioned from the culture dish, and exchanged with 0.8-mL of OPTI-MEM (GIBCO, 31985), a low serum basal medium. Then, 0.2 mL of a siRNA-Oligofectamine complex solution mixed in OPTI-MEM was added to introduce siRNA into PC-3. Two final concentrations were set for the siRNA at 3 nM and 30 nM.
The human prostate cancer cells PC-3 after siRNA transfection were cultured in a 5% CO₂ incubator at 37°C for 48 hours, washed twice with PBS, and transferred to a 1.5-mL tube using a cell scraper. After removing the supernatant by centrifugation (1,000 × g, 2 min), the cells were collected by being dissolved in RLT buffer (attached to the RNA collection kit "RNeasy", Qiagen). Total RNA was then collected according to the protocol attached to the kit.
cDNA was produced by reverse-transcription reaction with Superscript VILO (Invitrogen), using the total RNA (1 µg) as the template. The cDNA was used as the template of PCR reaction, and PCR amplification was performed specific to the bcl-2 gene and to the housekeeping gene GADPH (D-glyceraldehyde-3-phosphate dehydrogenase) gene using a Taqman probe method with ABI7900HT Fast (ABI) to quantify mRNA levels. For the PCR amplification of each gene, 250 ng of total RNA-derived cDNA was used as the template. The sample mRNA levels were represented as the relative ratio with respect to the mRNA level 1 of the bcl-2 or GADPH in the siRNA non-introduced group (untreated). The difference of the expression level ratio of each sample subtracted from 1 was represented as expression suppressing rate, and presented in Fig. 1.

### Test Example 2

The preparations obtained from Examples 1 and 2 and Comparative Examples 1 to 9 were evaluated for the influence of the secondly administered PEG-modified lipidparticle on blood retention, as follows. The preparations obtained from Examples 1 and 2 and Comparative Examples 1 to 9 were administered to mice as the firstly administered PEG-modified lipidparticles. After 7 days, the preparation obtained in Comparative Example 1 was administered as the secondly administered PEG-modified lipidparticle. The blood concentration of BCL2siRNA-Com. 1 was then measured 3 hours after the administration.
Drug solutions (siRNA concentration, 50 µg/mL; 100 µL) containing the preparations obtained from Examples 1 and 2 and Comparative Examples 1 to 9 were administered to male Balb/c mice (6 weeks of age, CLEA Japan, Inc.) through the tail vein (dose, 5 µg/mouse). After 7 days, a drug solution (siRNA concentration, 50 µg/mL; 100 µL) containing the preparation obtained from Comparative Example 1 was administered through the tail vein (dose, 5 µg/mouse). After 3 hours from the second administration, blood (10 µL) was collected from the tail artery, and mixed with 90 µL of a denaturing solution (4 mol/L guanidine thiocyanate, 25 mmol/L sodium citrate, 0.1 v/v% 2-mercaptoethanol, 0.5 w/v% sodium *N*-lauroyl sarcosine; hereinafter referred to as "D solution"). As a result, a 10 v/v% blood was obtained.

The 10 v/v% blood (50 µL) was mixed with 5 µL of a diethylpyrocarbonate aqueous solution (a 0.1 v/v% mixture of diethylpyrocarbonate in ultrapure water), 10 µL of I.S. solution (0.3 µmol/L of the diethylpyrocarbonate aqueous solution as I.S.), 50 µL of D solution, 10 µL of 2 mmol/L sodium acetate (pH 4.0), and 150 µL of saturated phenol/chloroform aqueous solution. The mixture was then centrifuged. The supernatant (65 µL) was mixed with 15 µL of a GenTLE solution (GenTLE precipitation carrier (Takara Bio Inc.) diluted 15 times with the diethylpyrocarbonate aqueous solution), and ethanol was added. After centrifugation, the supernatant was discarded, and 75 v/v% ethanol was added to the precipitate. After centrifugation, the supernatant was discarded, and the precipitate was air-dried and dissolved in 50 µL of a redissolving solution (a 0.1/0.4/30/1,000 volume ratio mixture of diethylpyrocarbonate/triethylamine/hexafluoroisopropanol/water). The mixture was then quantified using HPLC. The results are shown in Fig. 2.

### Apparatus

HPLC apparatus: ACQUITY HPLC system (Waters)
Mass spectroscope: API4000 Q TRAP (Applied Biosystems/MDS Sciex)

HPLC Conditions

### Internal standard substance (I.S.)

5'-GUG AAG UCA ACA UGC CUG dTdT-3' (the sugars binding to the base prefaced by d are deoxyribose)(SEQ ID NO: 27)
5'-CAG GCA UGU UGA CUU CAC dTdT-3' (the sugars binding to the base prefaced by d are deoxyribose)(SEQ ID NO: 28)

Column: Xbridge C18 (3.5 µm, 2.1 mm I.D. x 50 mm, Waters)
Mobile phase: triethylamine/hexafluoroisopropanol/water (0.4/30/1000):methanol = 93:7 to 75:25

It can be seen from Fig. 1 that the double-stranded nucleic acid molecules used in Examples 1 to 4 (Exp. 1 to Exp. 4) show siRNA activities comparable to that of the double-stranded nucleic acid molecule used in Comparative Example 1, and that the siRNA activities are higher than those of the double-stranded nucleic acid molecules used in Comparative Examples 2 to 9. It can be seen from Fig. 2 that the double-stranded nucleic acid molecule cannot be observed in the blood and that the blood retention lowers greatly in the second administration of the PEG-modified lipidparticle in mice administered with the preparation of Comparative Example 1, and after 7 days with the preparation of Comparative Example 1 as the secondly administered PEG-modified lipidparticle. In contrast, in mice administered with the preparations of Examples 1 and 2, and after 7 days with the preparation of Comparative Example 1 as the secondly administered PEG-modified lipidparticle, the blood concentration of the double-stranded nucleic acid molecule was high, demonstrating that a decrease in blood retention in the second administration of the PEG-modified lipidparticle was suppressed.
Therefore, it was found that the composition of the present invention below had high siRNA activity, and was capable of reducing side reaction by suppressing a decrease in blood retention in the second administration of the PEG-modified lipidparticle, and increasing the accumulation of the drug in tissues or organs that contain a target gene expression site.
The composition comprises a lipidparticle encapsulating a double-stranded nucleic acid molecule that contains a sense strand and an antisense strand,
wherein the antisense strand is a polynucleotide of 17 to 30 bases in which a sequence of bases 1 to 17 in the 5'-end to 3'-end direction (sequence a) is complementary to the sequence of the 17 contiguous bases of a target gene's mRNA, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5'-end to 3'-end direction of the antisense strand, and the sugars in the sense strand are ribose, deoxyribose, or ribose whose hydroxyl group at the 2' position is substituted by a modifying group,
(i) 0 to 30% of the sugars binding to the bases 1 to 8 in the 5'-end to 3'-end direction of sequence a are deoxyribose, or ribose whose hydroxyl group at the 2' position is substituted by a modifying group,
(ii) 0 to 20% of the sugars binding to the bases 9 to 16 in the 5'-end to 3'-end direction of sequence a are deoxyribose, or a ribose whose hydroxyl group at the 2' position is substituted by a modifying group,
(iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
(iv) 10 to 70% of the sugars binding to the bases 1 to 17 in the 5'-end to 3'-end direction of sequence b are deoxyribose, or ribose whose hydroxyl group at the 2' position is substituted by a modifying group,
(v) 30 to 100% of the sugars binding to the bases other than sequence b of the sense strand are deoxyribose, or ribose whose hydroxyl group at the 2' position is substituted by a modifying group, and
the lipidparticle is a lipidparticle having a size that allows for intravenous administration, and comprises a lipid bilayer membrane whose constituent component is a lipid conjugate, a fatty acid conjugate, or an aliphatic hydrocarbon conjugate of a water-soluble substance.

Example 5 and Comparative Examples 10 to 13 used double-stranded nucleic acid molecules that contained the sequence of 25 contiguous bases 5'-CCA CAA GUG AAG UCA ACA UGC CUG C-3' of BCL2 gene mRNA with the sense strand and the antisense strand shown in Table 2 (the sugars binding to the base prefaced by m are 2'-0-methyl-substituted ribose). The sense strand and the antisense strand were obtained from Hokkaido System Science Co., Ltd., and were annealed to prepare the double-stranded nucleic acid molecules.

### Example 5

DOTAP (manufactured by Avanti Polar Lipids Inc.), PEG-DSPE (manufactured by NOF Corporation) and distilled water (manufactured by Otsuka Pharmaceutical Co., Ltd.) were mixed such that the amounts of DOTAP/PEG-DSPE/distilled water was 40 mg/16 mg/1 mL, and the mixture was stirred by shaking with a vortex mixer. The obtained suspension was passed, at 70°C, through a 0.4-µm polycarbonate membrane filter (manufactured by Costar) 10 times and through a 0.2-µm polycarbonate membrane filter (manufactured by Whatman) 3 times and then through a 0.1-µm polycarbonate membrane filter (manufactured by Corning) 10 times and a 0.05-µm polycarbonate membrane filter (manufactured by Whatman) 20 times. The obtained lead particle had an average particle diameter of 71.44 nm as measured by Dynamic light scattering (DLS).
Meanwhile, EPC (NOF Corporation)/PEG-DSPE (NOF Corporation)/ethanol (Wako Pure Chemical Industries, Ltd.)/water (15 mg/3. 125 mg/0.625 mL/0.375 mL) were mixed, thereby a solution containing the constituent components of the lipid bilayer membrane was prepared.
The obtained lead particle dispersion (0.025 mL) was mixed with an aqueous solution (0.00833 mL) obtained by mixing the BCL2siRNA-Exp. 5 in 24 mg/ mL, so as to prepare the complex particles. The obtained dispersion of complex particles was added to the solution of lipid bilayer membrane constituent components (0.13334 mL), and 0.04166 mL of distilled water was added. Then, after adding a solution (0.01334 mL) of EPC/PEG-DSPE dissolved in 40 vol% ethanol in 62.5 mg/62.5 mg/mL, distilled water (1.5517 mL) was gradually added to adjust the ethanol concentration to 5% or less and prepare the lipidparticle. The resulting lipidparticle suspension was isotonized with brine. A preparation was obtained by adjusting the BCL2siRNA-Exp. 5 concentration to 0.1 mg/mL by making the final liquid volume 2 mL with physiological saline (Otsuka Pharmaceutical Co., Ltd.).
The lipidparticle in the preparation had an average particle diameter of 77.26 nm as measured by DLS.

### Comparative Examples 10 to 13

Preparations were obtained in the same manner as in Example 5, except that BCL2siRNA-Com. 10 to 13 were used instead of BCL2siRNA-Exp. 5.
The average particle diameter of the lipidparticle in each preparation was measured by DLS. Table 2 presents the average particle diameter of the lipidparticle in each preparation.

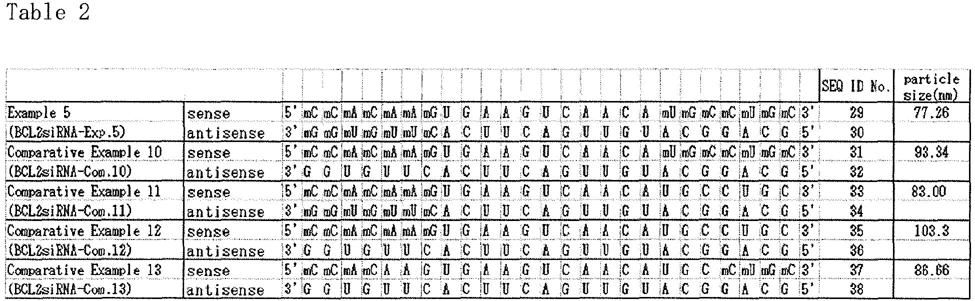

### Test Example 3

The RNAi activities of BCL2siRNA-Exp. 5 and BCL2siRNA-Com. 10 to 13 were evaluated by measuring the expression suppressing effect on Bcl2mRNA, as follows.
PC-3 cells were inoculated in a 6 cm-diameter culture dish (2 × 10⁵ cells/dish), and cultured overnight under 37°C, 5% CO₂ conditions in F-12 Kaighn's medium (GIBCO, 21127) that contained 10% fetal bovine serum. On the next day, the medium was suctioned from the culture dish, and exchanged with 0.8-mL of OPTI-MEM (GIBCO, 31985), a low serum basal medium. Then, 0.2 mL of a siRNA-Oligofectamine complex solution mixed in OPTI-MEM was added to transfect siRNA into PC-3. The final concentration of the siRNA was set to 10 nM.
The cells after siRNA transfection were cultured in a 5% CO₂ incubator at 37°C for 48 hours, washed twice with PBS, and transferred to a 1.5-mL tube using a cell scraper. After removing the supernatant by centrifugation (1,000 × g, 2 min), the cells were collected by being dissolved in RLT buffer (attached to the RNA collection kit "RNeasy", Qiagen). Total RNA was then collected according to the protocol attached to the kit.
cDNA was produced by reverse-transcription reaction with Superscript VILO (Invitrogen), using the total RNA (1 µg) as the template. The cDNA was used as the template of PCR reaction, and PCR amplification was performed specific to the bcl-2 gene and to the housekeeping gene GADPH (D-glyceraldehyde-3-phosphate dehydrogenase) gene using a Taqman probe method with ABI7900HT Fast (ABI) to quantify mRNA levels. For the PCR amplification of each gene, 250 ng of total RNA-derived cDNA was used as the template. The sample mRNA levels were represented as the relative ratio with respect to the mRNA level 1 of the bcl-2 or GADPH in the siRNA non-introduced group (untreated), and presented in Fig. 3.

### Test Example 4

The preparations obtained from Example 5 and Comparative Examples 10 to 13 were evaluated as in Test Example 2 with respect to the influence of the secondly administered PEG-modified lipidparticle on blood retention. The preparations obtained from Example 5 and Comparative Examples 10 to 13 were used as the firstly administered PEG-modified lipidparticles, and also as the secondly administered PEG-modified lipidparticles.
Fig. 4 represents the blood concentrations of the BCL2siRNA-Exp. 5 (Example 5) and BCL2siRNA-Com. 10 to 13 (Comparative Examples 10 to 13) measured after 3 hours from the second administration.
As the internal standard substances (I.S.), 5'-GmUG mAAmG UmCA mACmA UmGC mCUmG CdT-3' (the sugars binding to the 2nd, 4th, 6th, 8th, 10th, 12th, 14th, 16th, and 18th bases prefaced by m relative from the 5' -end are 2'-0-methyl-substituted ribose, and the sugars binding to the base prefaced by d are deoxyribose) (SEQ ID NO: 39), and 5'-GCA GGC AUG UUG ACU UCA CdT-3' (the sugars binding to the base prefaced by d are deoxyribose) (SEQ ID NO: 40) were used.

It can be seen from Fig. 3 that the double-stranded nucleic acid molecule used in Example 5 shows a siRNA activity comparable to those of the double-stranded nucleic acid molecules used in Comparative Examples 10 to 13. It can be seen from Fig. 4 that the double-stranded nucleic acid molecule cannot be observed in the blood and that the blood retention lowers greatly in the second administration of the PEG-modified lipidparticle in mice administered with the preparations of Comparative Examples 10 to 13 twice 7 days apart. In contrast, in mice administered with the preparation of Example 5 twice 7 days apart, the blood concentration of the double-stranded nucleic acid molecule was high, demonstrating that a decrease in blood retention in the second administration of the PEG-modified lipidparticle was suppressed.
Therefore, it was found that the composition of the present invention below had high siRNA activity, and was capable of reducing side reaction by suppressing a decrease in blood retention in the second administration of the PEG-modified lipidparticle, and increasing the accumulation of the drug in tissues or organs that contain a target gene expression site.
The composition comprises a lipidparticle encapsulating a double-stranded nucleic acid molecule that contains a sense strand and an antisense strand,
wherein the antisense strand is a polynucleotide of 17 to 30 bases in which a sequence (sequence a) of bases 1 to 17 in the 5'-end to 3'-end direction is complementary to the sequence of the 17 contiguous bases of a target gene's mRNA, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5'-end to 3'-end direction of the antisense strand, and the sugars in the sense strand are ribose, deoxyribose, or ribose whose hydroxyl group at the 2' position is substituted by a modifying group,
(i) 0 to 30% of the sugars binding to the bases 1 to 8 in the 5'-end to 3'-end direction of sequence a are deoxyribose, or ribose whose hydroxyl group at the 2' position is substituted by a modifying group,
(ii) 0 to 20% of the sugars binding to the bases 9 to 16 in the 5' -end to 3'-end direction of sequence a are deoxyribose, or a ribose whose hydroxyl group at the 2' position is substituted by a modifying group,
(iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
(iv) 10 to 70% of the sugars binding to the bases 1 to 17 in the 5'-end to 3'-end direction of sequence b are deoxyribose, or ribose whose hydroxyl group at the 2' position is substituted by a modifying group,
(v) 30 to 100% of the sugars binding to the bases other than sequence b of the sense strand are deoxyribose, or ribose whose hydroxyl group at the 2' position is substituted by a modifying group, and
the lipidparticle is a lipidparticle having a size that allows for intravenous administration, and comprises a lipid bilayer membrane whose constituent component is a lipid conjugate, a fatty acid conjugate, or an aliphatic hydrocarbon conjugate of a water-soluble substance.

### Industrial Applicability

By administering the composition of the present invention to a mammal or the like, the expression of the target gene can be suppressed.

[SEQUENCE LISTING FREE TEXT]
SEQ ID NO.1: siRNA sense of Com. 1
SEQ ID NO.2: siRNA antisense of Com. 1
SEQ ID NO.3: siRNA sense of Exp. 1
SEQ ID NO.4: siRNA antisense of Exp. 1
SEQ ID NO.5: siRNA sense of Com. 2
SEQ ID NO.6: siRNA antisense of Com. 2
SEQ ID NO.7: siRNA sense of Com. 3
SEQ ID NO.8: siRNA antisense of Com. 3
SEQ ID N0.9 siRNA sense of Com. 4
SEQ ID NO.10: siRNA antisense of Com. 4
SEQ ID NO.11: siRNA sense of Com. 5
SEQ ID NO.12: siRNA antisense of Com. 5
SEQ ID NO.13: siRNA sense of Exp. 2
SEQ ID NO.14: siRNA antisense of Exp. 2
SEQ ID NO.15: siRNA sense of Com. 6
SEQ ID NO.16: siRNA antisense of Com. 6
SEQ ID NO.17: siRNA sense of Com. 7
SEQ ID NO.18: siRNA antisense of Com. 7
SEQ ID NO. 19 siRNA sense of Com. 8
SEQ ID NO.20: siRNA antisense of Com. 8
SEQ ID NO.21: siRNA sense of Com. 9
SEQ ID NO.22: siRNA antisense of Com. 9
SEQ ID NO.23: siRNA sense of Exp. 3
SEQ ID NO.24: siRNA antisense of Exp. 3
SEQ ID NO.25: siRNA sense of Exp. 4
SEQ ID NO.26: siRNA antisense of Exp. 4
SEQ ID NO.27: IS for siRNA sense of exp. 1 to 4 and com.1 to 9
SEQ ID NO.28: IS for siRNA antisense of exp. 1 to 4 and com. 1 to 9
SEQ ID NO.29: siRNA sense of Exp. 5
SEQ ID NO.30: siRNA antisense of Exp. 5
SEQ ID NO.31: siRNA sense of Com. 10
SEQ ID NO.32: siRNA antisense of Com. 10
SEQ ID NO.33: siRNA sense of Com. 11
SEQ ID NO.34: siRNA antisense of Com. 11
SEQ ID NO.35 siRNA sense of Com. 12
SEQ ID NO.36: siRNA antisense of Com. 12
SEQ ID NO.37: siRNA sense of Com. 13
SEQ ID NO.38: siRNA antisense of Com. 13
SEQ ID NO.39: IS for siRNA sense of exp. 5 and com. 10 to 13
SEQ ID NO.40: IS for siRNA antisense of exp. 5 and com. 10 to 13
SEQ ID NO.41: bc12 mRNA

## Claims

1. A composition that comprises a lipidparticle encapsulating a double-stranded nucleic acid molecule that contains a sense strand and an antisense strand,
wherein the antisense strand is a polynucleotide of 17 to 30 bases in which a sequence (sequence a) of bases 1 to 17 in the 5'-end to 3'-end direction is complementary to the sequence of the 17 contiguous bases of a target gene's mRNA, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5'-end to 3'-end direction of the antisense strand, and the sugars in the sense strand are ribose, deoxyribose, or ribose whose hydroxyl group at the 2' position is substituted by a modifying group,
(i) 0 to 30% of the sugars binding to the bases 1 to 8 in the 5'-end to 3'-end direction of sequence a are deoxyribose, or ribose whose hydroxyl group at the 2' position is substituted by a modifying group,
(ii) 0 to 20% of the sugars binding to the bases 9 to 16 in the 5'-end to 3'-end direction of sequence a are deoxyribose, or a ribose whose hydroxyl group at the 2' position is substituted by a modifying group,
(iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
(iv) 10 to 70% of the sugars binding to the bases 1 to 17 in the 5'-end to 3'-end direction of sequence b are deoxyribose, or ribose whose hydroxyl group at the 2' position is substituted by a modifying group,
(v) 30 to 100% of the sugars binding to the bases other than sequence b of the sense strand are deoxyribose, or ribose whose hydroxyl group at the 2' position is substituted by a modifying group, and
the lipidparticle is a lipidparticle having a size that allows for intravenous administration, and contains a lipid bilayer membrane whose constituent component is a lipid conjugate, a fatty acid conjugate or an aliphatic hydrocarbon conjugate of a water-soluble substance.

2. The composition according to Claim 1, wherein (v) 50 to 70% of the sugars binding to the bases other than sequence b of the sense strand are deoxyribose, or ribose whose hydroxyl group at the 2' position is substituted by a modifying group.

3. The composition according to Claim 1 or 2, wherein the double-stranded nucleic acid molecule is a double-stranded nucleic acid molecule having an activity of suppressing the expression of the target gene by utilizing RNA interference (RNAi).

4. The composition according to any one of Claim 1 to 3, wherein the target gene is a gene associated with tumor or inflammation.

5. The composition according to any one of Claim 1 to 4, wherein the target gene is a gene associated with angiogenesis.

6. The composition according to any one of Claim 1 to 4, wherein the target gene is a gene of any one of a vascular endothelial growth factor, a vascular endothelial growth factor receptor, a fibroblast growth factor, a fibroblast growth factor receptor, a platelet-derived growth factor, a platelet-derived growth factor receptor, a hepatocyte growth factor, a hepatocyte growth factor receptor, a Krüppel-like factor, an Ets transcription factor, a nuclear factor and a hypoxia-inducible factor.

7. The composition according to any one of Claim 1 to 6, wherein the mRNA is either human mRNA or mouse mRNA.

8. The composition according to any one of Claim 1 to 7, wherein the lipidparticle encapsulating the double-stranded nucleic acid molecule is a lipidparticle that comprises:
a complex particle that contains a lead particle and the double-stranded nucleic acid molecule as constituent components; and
a lipid bilayer membrane coating the complex particle,
wherein constituent components of the lipid bilayer membrane are soluble in a certain polar organic solvent, and wherein the constituent components of the lipid bilayer membrane, and the complex particle are dispersible in a liquid that contains the polar organic solvent in a certain concentration.

9. The composition according to Claim 8, wherein the polar organic solvent is an alcohol.

10. The composition according to Claim 8, wherein the polar organic solvent is ethanol.

11. The composition according to any one of Claim 8 to 10, wherein the lead particle is a lead particle that contains a cationic substance, and wherein the lipid bilayer membrane coating the complex particle contains, as constituent components, a neutral lipid, and a lipid conjugate, a fatty acid conjugate or an aliphatic hydrocarbon conjugate of a water-soluble substance.

12. The composition according to any one of Claim 1 to 7, wherein the lipidparticle encapsulating the double-stranded nucleic acid molecule is a lipidparticle that comprises: a complex particle that contains a cationic substance-containing lead particle and the double-stranded nucleic acid molecule as constituent components; and a lipid bilayer membrane coating the complex particle,
wherein the lipid bilayer membrane coating the complex particle contains, as constituent components, a neutral lipid, and a lipid conjugate, a fatty acid conjugate or an aliphatic hydrocarbon conjugate of a water-soluble substance.

13. The composition according to any one of Claim 1 to 12, wherein the lipid conjugate, the fatty acid conjugate or the aliphatic hydrocarbon conjugate of a water-soluble substance is polyethylene glycol phosphatidyl ethanolamine.

14. A therapeutic agent for cancer or inflammatory disease, the therapeutic agent comprising a lipidparticle that comprises:
a complex particle that contains, as constituent components, a lead particle and a double-stranded nucleic acid molecule that contains a sense strand and an antisense strand; and
a lipid bilayer membrane coating the complex particle,
wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5'-end to 3' -end direction of the antisense strand, and the sugars in the sense strand are ribose, deoxyribose, or ribose whose hydroxyl group at the 2' position is substituted by a modifying group,
(i) 0 to 30% of the sugars binding to the bases 1 to 8 in the 5'-end to 3'-end direction of sequence a are deoxyribose, or ribose whose hydroxyl group at the 2' position is substituted by a modifying group,
(ii) 0 to 20% of the sugars binding to the bases 9 to 16 in the 5'-end to 3'-end direction of sequence a are deoxyribose, or a ribose whose hydroxyl group at the 2' position is substituted by a modifying group,
(iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
(iv) 10 to 70% of the sugars binding to the bases 1 to 17 in the 5'-end to 3'-end direction of sequence b are deoxyribose, or ribose whose hydroxyl group at the 2' position is substituted by a modifying group,
(v) 30 to 100% of the sugars binding to the bases other than sequence b of the sense strand are deoxyribose, or ribose whose hydroxyl group at the 2' position is substituted by a modifying group, and
the lipidparticle is a lipidparticle having a size that allows for intravenous administration,
wherein the constituent components of the lipid bilayer membrane are soluble in a certain polar organic solvent, and wherein the constituent components of the lipid bilayer membrane, and the complex particle are dispersible in a liquid that contains the polar organic solvent in a certain concentration, and the lipid bilayer membrane contains as constituent component, a lipid conjugate, a fatty acid conjugate, or an aliphatic hydrocarbon conjugate of a water-soluble substance.

15. The therapeutic agent for cancer or inflammatory disease according to Claim 14, wherein the polar organic solvent is an alcohol.

16. The therapeutic agent for cancer or inflammatory disease according to Claim 14, wherein the polar organic solvent is ethanol.

17. The therapeutic agent for cancer or inflammatory disease according to any one of Claim 14 to 16, wherein the lead particle is a lead particle that contains a cationic substance, and wherein the lipid bilayer membrane contains, as constituent components, a neutral lipid, and a lipid conjugate, a fatty acid conjugate or an aliphatic hydrocarbon conjugate of a water-soluble substance.

18. A therapeutic agent for cancer or inflammatory disease, the therapeutic agent comprising a lipidparticle that comprises:
a complex particle that contains, as constituent components, a cationic substance-containing lead particle and a double-stranded nucleic acid molecule that contains a sense strand and an antisense strand; and
a lipid bilayer membrane coating the complex particle,
wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5' -end to 3' -end direction of the antisense strand, and the sugars in the sense strand are ribose, deoxyribose, or ribose whose hydroxyl group at the 2' position is substituted by a modifying group,
(i) 0 to 30% of the sugars binding to the bases 1 to 8 in the 5'-end to 3'-end direction of sequence a are deoxyribose, or ribose whose hydroxyl group at the 2' position is substituted by a modifying group,
(ii) 0 to 20% of the sugars binding to the bases 9 to 16 in the 5'-end to 3'-end direction of sequence a are deoxyribose, or a ribose whose hydroxyl group at the 2' position is substituted by a modifying group,
(iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
(iv) 10 to 70% of the sugars binding to the bases 1 to 17 in the 5'-end to 3'-end direction of sequence b are deoxyribose, or ribose whose hydroxyl group at the 2' position is substituted by a modifying group,
(v) 30 to 100% of the sugars binding to the bases other than sequence b of the sense strand are deoxyribose, or ribose whose hydroxyl group at the 2' position is substituted by a modifying group, and
the lipidparticle is a lipidparticle having a size that allows for intravenous administration, and the lipid bilayer membrane contains, as constituent components, a neutral lipid, and a lipid conjugate, a fatty acid conjugate or an aliphatic hydrocarbon conjugate of a water-soluble substance.

19. The therapeutic agent for cancer or inflammatory disease according to any one of Claim 14 to 18, wherein the lipid conjugate, the fatty acid conjugate, or the aliphatic hydrocarbon conjugate of a water-soluble substance is polyethylene glycol phosphatidyl ethanolamine.

20. The therapeutic agent for cancer or inflammatory disease according to any one of Claim 14 to 19, wherein (v) 50 to 70% of the sugars binding to the bases other than sequence b of the sense strand are deoxyribose, or ribose whose hydroxyl group at the 2' position is substituted by a modifying group.

21. The therapeutic agent for cancer or inflammatory disease according to any one of Claim 14 to 20, wherein the target gene associated with tumor or inflammation is a gene associated with angiogenesis.

22. The therapeutic agent for cancer or inflammatory disease according to any one of Claim 14 to 20, wherein the target gene associated with tumor or inflammation is a gene of any one of a vascular endothelial growth factor, a vascular endothelial growth factor receptor, a fibroblast growth factor, a fibroblast growth factor receptor, a platelet-derived growth factor, a platelet-derived growth factor receptor, a hepatocyte growth factor, a hepatocyte growth factor receptor, a Krüppel-like factor, an Ets transcription factor, a nuclear factor, and a hypoxia-inducible factor.

23. The therapeutic agent for cancer or inflammatory disease according to any one of Claim 14 to 22, wherein the mRNA is either human mRNA or mouse mRNA.

24. A method for treating cancer or inflammatory disease, which comprises administering to a mammal a composition that comprises a lipidparticle that comprises:
a complex particle that contains, as constituent components, a lead particle and a double-stranded nucleic acid molecule that contains a sense strand and an antisense strand; and
a lipid bilayer membrane coating the complex particle,
wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5' -end to 3' -end direction of the antisense strand, and the sugars in the sense strand are ribose, deoxyribose, or ribose whose hydroxyl group at the 2' position is substituted by a modifying group,
(i) 0 to 30% of the sugars binding to the bases 1 to 8 in the 5'-end to 3'-end direction of sequence a are deoxyribose, or ribose whose hydroxyl group at the 2' position is substituted by a modifying group,
(ii) 0 to 20% of the sugars binding to the bases 9 to 16 in the 5'-end to 3'-end direction of sequence a are deoxyribose, or a ribose whose hydroxyl group at the 2' position is substituted by a modifying group,
(iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
(iv) 10 to 70% of the sugars binding to the bases 1 to 17 in the 5'-end to 3'-end direction of sequence b are deoxyribose, or ribose whose hydroxyl group at the 2' position is substituted by a modifying group,
(v) 30 to 100% of the sugars binding to the bases other than sequence b of the sense strand are deoxyribose, or ribose whose hydroxyl group at the 2' position is substituted by a modifying group, and
the lipidparticle is a lipidparticle having a size that allows for intravenous administration,
wherein the constituent components of the lipid bilayer membrane are soluble in a certain polar organic solvent, and wherein the constituent components of the lipid bilayer membrane, and the complex particle are dispersible in a liquid that contains the polar organic solvent in a certain concentration, and the lipid bilayer membrane contains as constituent component, a lipid conjugate, a fatty acid conjugate, or an aliphatic hydrocarbon conjugate of a water-soluble substance.

25. A method for treating cancer or inflammatory disease, which comprises administering to a mammal a composition that comprises a lipidparticle that comprises:
a complex particle that contains, as constituent components, a cationic substance-containing lead particle and a double-stranded nucleic acid molecule that contains a sense strand and an antisense strand; and
a lipid bilayer membrane coating the complex particle,
wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5'-end to 3' -end direction of the antisense strand, and the sugars in the sense strand are ribose, deoxyribose, or ribose whose hydroxyl group at the 2' position is substituted by a modifying group,
(i) 0 to 30% of the sugars binding to the bases 1 to 8 in the 5'-end to 3'-end direction of sequence a are deoxyribose, or ribose whose hydroxyl group at the 2' position is substituted by a modifying group,
(ii) 0 to 20% of the sugars binding to the bases 9 to 16 in the 5'-end to 3'-end direction of sequence a are deoxyribose, or a ribose whose hydroxyl group at the 2' position is substituted by a modifying group,
(iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
(iv) 10 to 70% of the sugars binding to the bases 1 to 17 in the 5'-end to 3'-end direction of sequence b are deoxyribose, or ribose whose hydroxyl group at the 2' position is substituted by a modifying group,
(v) 30 to 100% of the sugars binding to the bases other than sequence b of the sense strand are deoxyribose, or ribose whose hydroxyl group at the 2' position is substituted by a modifying group, and
the lipidparticle is a lipidparticle having a size that allows for intravenous administration, and the lipid bilayer membrane contains, as constituent components, a neutral lipid, and a lipid conjugate, a fatty acid conjugate or an aliphatic hydrocarbon conjugate of a water-soluble substance.

26. Use of a composition for a manufacture of a therapeutic agent for cancer or inflammatory disease, the composition comprising a lipidparticle that comprises:
a complex particle that contains, as constituent components, a lead particle and a double-stranded nucleic acid molecule that contains a sense strand and an antisense strand; and
a lipid bilayer membrane coating the complex particle,
wherein the antisense strand is a polynucleotide of 17 to 30 bases in which a sequence (sequence a) of bases 1 to 17 in the 5'-end to 3'-end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5'-end to 3' -end direction of the antisense strand, and the sugars in the sense strand are ribose, deoxyribose, or ribose whose hydroxyl group at the 2' position is substituted by a modifying group,
(i) 0 to 30% of the sugars binding to the bases 1 to 8 in the 5'-end to 3'-end direction of sequence a are deoxyribose, or ribose whose hydroxyl group at the 2' position is substituted by a modifying group,
(ii) 0 to 20% of the sugars binding to the bases 9 to 16 in the 5'-end to 3'-end direction of sequence a are deoxyribose, or a ribose whose hydroxyl group at the 2' position is substituted by a modifying group,
(iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
(iv) 10 to 70% of the sugars binding to the bases 1 to 17 in the 5'-end to 3'-end direction of sequence b are deoxyribose, or ribose whose hydroxyl group at the 2' position is substituted by a modifying group,
(v) 30 to 100% of the sugars binding to the bases other than sequence b of the sense strand are deoxyribose, or ribose whose hydroxyl group at the 2' position is substituted by a modifying group, and
the lipidparticle is a lipidparticle having a size that allows for intravenous administration,
wherein the constituent components of the lipid bilayer membrane are soluble in a certain polar organic solvent, and wherein the constituent components of the lipid bilayer membrane, and the complex particle are dispersible in a liquid that contains the polar organic solvent in a certain concentration, and the lipid bilayer membrane contains as constituent component, a lipid conjugate, a fatty acid conjugate, or an aliphatic hydrocarbon conjugate of a water-soluble substance.

27. Use of a composition for a manufacture of a therapeutic agent for cancer or inflammatory disease, the composition comprising a lipidparticle that comprises:
a complex particle that contains, as constituent components, a cationic substance-containing lead particle and a double-stranded nucleic acid molecule that contains a sense strand and an antisense strand; and
a lipid bilayer membrane coating the complex particle,
wherein the antisense strand is a polynucleotide of 17 to 30 bases in which a sequence (sequence a) of bases 1 to 17 in the 5'-end to 3' -end direction is complementary to the sequence of the 17 contiguous bases of the mRNA of a target gene associated with tumor or inflammation, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5'-end to 3'-end direction of the antisense strand, and the sugars in the sense strand are ribose, deoxyribose, or ribose whose hydroxyl group at the 2' position is substituted by a modifying group,
(i) 0 to 30% of the sugars binding to the bases 1 to 8 in the 5'-end to 3'-end direction of sequence a are deoxyribose, or ribose whose hydroxyl group at the 2' position is substituted by a modifying group,
(ii) 0 to 20% of the sugars binding to the bases 9 to 16 in the 5' -end to 3'-end direction of sequence a are deoxyribose, or a ribose whose hydroxyl group at the 2' position is substituted by a modifying group,
(iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
(iv) 10 to 70% of the sugars binding to the bases 1 to 17 in the 5'-end to 3'-end direction of sequence b are deoxyribose, or ribose whose hydroxyl group at the 2' position is substituted by a modifying group,
(v) 30 to 100% of the sugars binding to the bases other than sequence b of the sense strand are deoxyribose, or ribose whose hydroxyl group at the 2' position is substituted by a modifying group, and
the lipidparticle is a lipidparticle having a size that allows for intravenous administration, and the lipid bilayer membrane contains, as constituent components, a neutral lipid, and a lipid conjugate, a fatty acid conjugate or an aliphatic hydrocarbon conjugate of a water-soluble substance.

28. A method for suppressing the expression of a target gene, which comprises administering to mammals a composition that comprises:
a lipidparticle encapsulating a double-stranded nucleic acid molecule that contains a sense strand and an antisense strand,
wherein the antisense strand is a polynucleotide of 17 to 30 bases in which a sequence (sequence a) of bases 1 to 17 in the 5'-end to 3' -end direction is complementary to the sequence of the 17 contiguous bases of a target gene's mRNA, and the sugars in the antisense strand are ribose, deoxyribose, or ribose whose hydroxyl group at the 2' position is substituted by a modifying group, and
wherein the sense strand is a polynucleotide of 17 to 30 bases that contains a base sequence (sequence b) complementary to the base sequence of bases 1 to 17 in the 5' -end to 3' -end direction of the antisense strand, and the sugars in the sense strand are ribose, deoxyribose, or ribose whose hydroxyl group at the 2' position is substituted by a modifying group,
(i) 0 to 30% of the sugars binding to the bases 1 to 8 in the 5'-end to 3'-end direction of sequence a are deoxyribose, or ribose whose hydroxyl group at the 2' position is substituted by a modifying group,
(ii) 0 to 20% of the sugars binding to the bases 9 to 16 in the 5'-end to 3'-end direction of sequence a are deoxyribose, or a ribose whose hydroxyl group at the 2' position is substituted by a modifying group,
(iii) 30 to 100% of the sugars binding to the bases from base 17 to the 3'-end base in the 5'-end to 3'-end direction of the antisense strand are deoxyribose, or ribose whose hydroxyl group at the 2' position is substituted by a modifying group,
(iv) 10 to 70% of the sugars binding to the bases 1 to 17 in the 5'-end to 3'-end direction of sequence b are deoxyribose, or ribose whose hydroxyl group at the 2' position is substituted by a modifying group,
(v) 30 to 100% of the sugars binding to the bases other than sequence b of the sense strand are deoxyribose, or ribose whose hydroxyl group at the 2' position is substituted by a modifying group, and
the lipidparticle is a lipidparticle having a size that allows for intravenous administration, and contains a lipid bilayer membrane whose constituent component is a lipid conjugate, a fatty acid conjugate or an aliphatic hydrocarbon conjugate of a water-soluble substance.
